# FASCICULE DE BREVET EUROPEEN

(11) **EP 0 947 496 B1**
(45) Date de publication et mention de la délivrance du brevet: **09.10.2002**
(21) Numéro de dépôt: 99400597.3
(22) Date de dépôt: 11.03.1999
(51) Int. Cl.: C07C 59/64, C07C 59/72, A61K 31/19, A61K 7/06, A61K 7/48

(54) **Composés bicycliques-aromatiques et leur utilisation en médecine humaine ou vétérinaire ainsi qu'en cosmétologie**
Bicyclische aromatische Verbindungen und ihre Anwendung in der Menschen- und Tierheilkunde sowie in der Kosmetikkunde
Bicyclic aromatic compounds and their use in human and veterinary medicine as well as in cosmetology

(30) Priorité: 31.03.1998 FR 9803976
(43) Date de publication de la demande: 06.10.1999
(73) Titulaire: Galderma Research & Development, S.N.C., 06560 Valbonne (FR)
(72) Inventeur: Bernardon, Jean-Michel, 06650 Le Rouret (FR)
(74) Mandataire: Tezier Herman, Béatrice

(56) Documents cités:
- WO-A-97/33881

## Description

L'invention concerne, à titre de produits industriels nouveaux et utiles, des composés biaromatiques. Elle concerne également l'utilisation de ces nouveaux composés dans des compositions pharmaceutiques destinées à un usage en médecine humaine ou vétérinaire, ou bien encore dans des compositions cosmétiques.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

La demanderesse a déjà décrit dans la demande de brevet WO97/33881 des composés bicycliques aromatiques ayant une activité agoniste des récepteurs RXRs et/ou une activité sur la différentiation cellulaire et leur utilisation dans la préparation d'une composition pharmaceutique destinée à traiter notamment les désordres cutanés liés à une anomalie de la différenciation et de la prolifération cellulaire.

La présente invention concerne des composés qui peuvent être représentés par la formule générale (I) suivante : 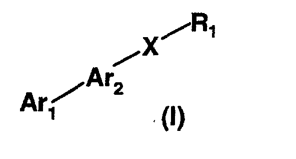 dans laquelle :
- R₁ représente
   (i) le radical -CH₃
   (ii) le radical -CH₂OR₂
   (iii) le radical -CO-R₃
   R₂ et R₃ ayant la signification donnée ci-après,
- Ar₁ représente un radical choisi parmi les radicaux de formules (a)-(c) suivantes : 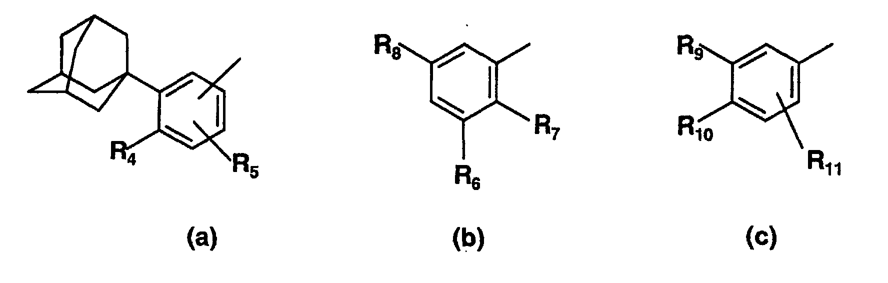 R₄ ,R₅ , R₆, R₇, R₈, R₉, R₁₀ et R₁₁ ayant les significations donnée ci-après,
- Ar₂ représente un radical choisi parmi les radicaux de formules (d)-(h) suivantes: 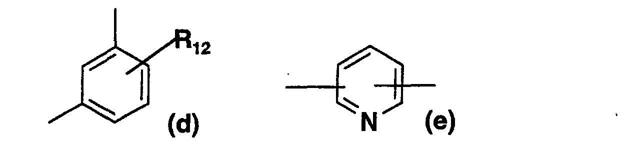 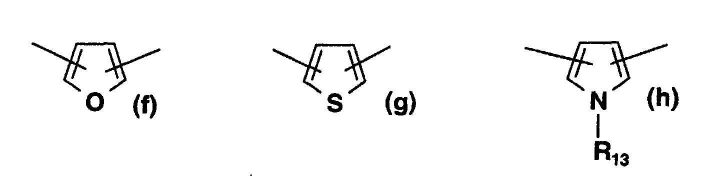 R₁₂ et R₁₃ ayant la signification donnée ci-après,
- X représente un radical choisi parmi les radicaux de formules (i)-(l), 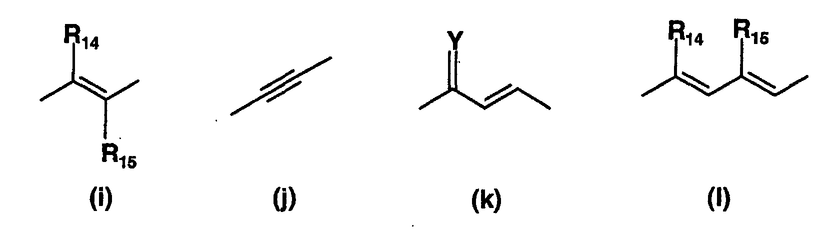 R₁₄, R₁₅ et Y ayant les significations données ci-après,
- R₂ représente un atome d'hydrogène, un radical alkyle ayant de 1 à 12 atomes de carbone ou un radical CO-R₁₆
   R₁₆ ayant la signification donnée ci-après,
- R₃ représente:
   (a) un atome d'hydrogène,
   (b) un radical alkyle ayant de 1 à 12 atomes de carbone,
   (c) un radical de formule : 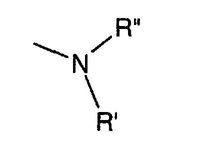
- R' et R" ayant la signification donnée ci-après,
   (d) un radical -OR₁₇
   R₁₇ ayant la signification donnée ci-après,
- R₄ représente un atome d'hydrogène, un radical polyéther, un radical alkyle ayant de 1 à 12 atomes de carbone ou un radical OR₁₈
   R₁₈ ayant les significations ci-après ,
- R₅ représente un atome d'hydrogène, un radical alkyle inférieur, un radical polyéther, un radical OR₁₉
   R₁₉ ayant les significations ci-après,
- R₆ représente un radical teriobutyle,
- R₇ représente un radical alkyle inférieur, un radical polyéther ou un radical OR₂₀
   R₂₀ ayant les significations ci-après,
- R₆ et R₇ pris ensemble peuvent former avec le cycle aromatique adjacent un cycle à 6 chaînons éventuellement substitué par des groupes méthyle et/ou éventuellement interrompu par un atome d'oxygène ou de soufre,
- R₈ représente un radical tertiobutyl, adamantyl, un radical phenyle éventuellement substitué par au moins un halogène, un alkyle ayant de 1 à 12 atomes de carbone, un hydroxyle, un alkoxy, une fonction nitro, un radical polyéther ou une fonction amino éventuellement protégée par un groupe acétyle ou éventuellement substituée par au moins un alkyle ayant de 1 à 12 atomes de carbone ou un radical benzyle ou phénethyle éventuellement substitués par au moins un halogène, un alkyle ayant de 1 à 12 atomes de carbone, un hydroxyle, un alkoxy, une fonction nitro, un radical polyéther ou une fonction amino éventuellement protégée par un groupe acétyle ou éventuellement substituée par au moins un alkyle ayant de 1 à 12 atomes de carbone,
- R₉ et R₁₀ pris ensemble forment avec le cycle aromatique adjacent un cycle à 5 ou 6 chaînons éventuellement substitué par des groupes méthyle et/ou éventuellement interrompu par un atome d'oxygène ou de soufre,
- R₁₁ représente un atome d'hydrogène, un radical alkyle ayant de 1 à 9 atomes de carbone, un radical hydroxy, un radical alkoxy, un radical polyéther ou un radical -OR₂₁
   R₂₁ ayant la signification donnée ci-après,
- R₁₂ représente, un atome d'hydrogène, un radical hydroxy, un radical alkoxy, un radical polyéther ou un radical -OR₂₂
   R₂₂ ayant la signification donnée ci-après,
- R₁₃ représente un atome d'hydrogène, un radical alkyle ayant de 1 à 12 atomes de carboneou un radical -COR₂₃
   R₂₃ ayant la signification donnée ci-après,
- R₁₄ et R₁₅ identiques ou différents, représentent un atome d'hydrogène ou un radical alkyle ayant de 1 à 12 atomes de carbone,
- Y représente un atome d'oxygène ou un radical CH_{2,}
- R₁₆ représente un radical alkyle ayant de 1 à 12 atomes de carbone,
- R₁₇ représente un atome d'hydrogène, un radical alkyle linéaire ou ramifié, ayant de 1 à 20 atomes de carbone, un radical alkényle, un radical mono ou polyhydroxyalkyle, un radical phenyle éventuellement substitué par au moins un halogène, un alkyle ayant de 1 à 12 atomes de carbone, un hydroxyle, un alkoxy, une fonction nitro, un radical polyéther ou une fonction amino éventuellement protégée par un groupe acétyle ou éventuellement substituée par au moins un alkyle ayant de 1 à 12 atomes de carbone ou un radical benzyle ou phénethyle éventuellement substitués par au moins un halogène, un alkyle ayant de 1 à 12 atomes de carbone, un hydroxyle, un alkoxy, une fonction nitro, un radical polyéther ou une fonction amino éventuellement protégée par un groupe acétyle ou éventuellement substituée par au moins un alkyle ayant de 1 à 12 atomes de carbone ou un reste de sucre,
- R' et R" identiques ou différents, représentent un atome d'hydrogène, un radical alkyle ayant de 1 à 12 atomes de carbone, un radical mono ou polyhydroxyalkyle, un radical phenyle éventuellement substitué par au moins un halogène, un alkyle ayant de 1 à 12 atomes de carbone, un hydroxyle, un alkoxy, une fonction nitro, un radical polyéther ou une fonction amino éventuellement protégée par un groupe acétyle ou éventuellement substituée par au moins un alkyle ayant de 1 à 12 atomes de carbone ou un reste d'amino acide ou encore pris ensemble forment un hétérocycle,
- R₁₈, R_{19,} R₂₀ identiques ou différents, représentent un atome d'hydrogène, un radical alkyle ayant de 1 à 12 atomes de carbone, un radical alkényle, un radical mono ou polyhydroxyalkyle, un radical phenyle éventuellement substitué par au moins un halogène, un alkyle ayant de 1 à 12 atomes de carbone, un hydroxyle, un alkoxy, une fonction nitro, un radical polyéther ou une fonction amino éventuellement protégée par un groupe acétyle ou éventuellement substituée par au moins un alkyle ayant de 1 à 12 atomes de carbone ou un radical benzyle ou phénethyle éventuellement substitués par au moins un halogène, un alkyle ayant de 1 à 12 atomes de carbone, un hydroxyle, un alkoxy, une fonction nitro, un radical polyéther ou une fonction amino éventuellement protégée par un groupe acétyle ou éventuellement substituée par au moins un alkyle ayant de 1 à 12 atomes de carbone ou un radical -(CH₂)ₙ-R₂₄,
   n et R₂₄ ayant les significations ci-après,
- R₂₁ et R₂₂ identiques ou différents, représentent un radical alkényle, un radical mono ou polyhydroxyalkyle, un radical phenyle éventuellement substitué par au moins un halogène, un alkyle ayant de 1 à 12 atomes de carbone, un hydroxyle, un alkoxy, une fonction nitro, un radical polyéther ou une fonction amino éventuellement protégée par un groupe acétyle ou éventuellement substituée par au moins un alkyle ayant de 1 à 12 atomes de carbone ou un radical benzyle ou phénethyle éventuellement substitués par au moins un halogène, un alkyle ayant de 1 à 12 atomes de carbone, un hydroxyle, un alkoxy, une fonction nitro, un radical polyéther ou une fonction amino éventuellement protégée par un groupe acétyle ou éventuellement substituée par au moins un alkyle ayant de 1 à 12 atomes de carbone ou un radical -(CH₂)ₙ-R₂₄,
   n et R₂₄ ayant les significations ci-après,
- R₂₃ représente un radical alkyle ayant de 1 à 12 atomes de carbone,
- R₂₄ représente un hétérocycle, un radical monohydroxyalkyle, un radical thiol éventuellement substitué par un alkyle ayant de 1 à 12 atomes de carbone, un radical amino éventuellement substitué par au moins un alkyl ayant de 1 à 12 atomes de carbone, un radical -COOR₂₅, ou un radical -CON(R₂₆)R₂₇,
   R₂₅, R₂₆, et R₂₇ ayant les significations ci-après,
- R₂₅ représente un atome d'hydrogène ou un radical alkyle inférieur,
- R₂₆ et R₂₇ identiques ou différents représentent un atome d'hydrogène, un radical alkyle ayant de 1 à 12 atomes de carbone, un radical phenyle éventuellement substitué par au moins un halogène, un alkyle ayant de 1 à 12 atomes de carbone, un hydroxyle, un alkoxy, une fonction nitro, un radical polyéther ou une fonction amino éventuellement protégée par un groupe acétyle ou éventuellement substituée par au moins un alkyle ayant de 1 à 12 atomes de carbone ou encore pris ensemble forment un hétérocycle,
- n est un nombre entier tel que 2 ≤ n ≤ 9,
   avec les conditions que :
   - lorsque Ar₁ représente le radical de formule (c) et X représente les radicaux de formule (i) ou (j) alors,
      . R₁₁ représente le radical -OR₂₁ ou un radical polyéther, ou
      . Ar₂ représente le radical de formule (d) avec R₁₂ représente le radical -OR₂₂ ou un radical polyéther,
   - les composés de formule (I) sont différents de :
      R₁₁ représente le radical methoxymethoxy en position ortho par rapport au substituant Ar₂ lorsque Ar₁ représente le radical de formule (c), X représente le radical de formule (i), R₁₂ représente l'hydrogène et R₁ représente le radical COR₃ avec R₃ représente le radical -OR₁₇ et R₁₇ représente l'hydrogène,
      R₁₂ représente le radical methoxymethoxy en position ortho ou para par rapport au substituant Ar₁ lorsque Ar₁ représente le radical de formule (c) , X représente le radical de formule (i), R₁₁ représente le radical méthyle en position ortho par rapport à Ar₂, R₁ représente le radical COR₃ avec R₃ représente le radical -OR₁₇ et R₁₇ représente l'hydrogène,

L'invention vise également les sels des composés de formule (I) lorsque R₁ représente une fonction acide carboxylique et les isomères géométriques et optiques desdits composés de formule (I).

Lorsque les composés selon l'invention se présentent sous forme de sels, il s'agit de préférence de sels d'un métal alcalin ou alcalino-terreux, ou encore de zinc ou d'une amine organique.

Selon la présente invention, on entend par radical alkyle ayant 1 à 12 atomes de carbone un radical ayant de 1 à 12, de préférence de 1 à 9, atomes de carbone, avantageusement les radicaux méthyle, éthyle, propyle, isopropyle, butyle, tertiobutyle, pentyle, hexyle, heptyle, nonyle, décyle et dodécyle.

Par radical alkyle linéaire ou ramifié ayant de 1 à 20 atomes de carbone, on entend notamment les radicaux méthyle, éthyle, propyle, isopropyle, tertiobutyle, octyle, 2-éthylhexyle.

Par radical alkoxy, on entend un radical ayant de préférence 1 à 9 atomes de carbone, notamment les radicaux méthoxy, propyloxy, pentyloxy, heptyloxy.

Par radical monohydroxyalkyle ou polyhydroxyalkyle, on doit entendre un radical contenant de 1 à 6 atomes de carbone et de 1 à 5 groupes hydroxyles.

Parmi les radicaux monohydroxyalkyle, on préfère un radical présentant 2 ou 3 atomes de carbone, notamment un radical 2-hydroxyéthyle, 2-hydroxypropyle ou 3-hydroxypropyle.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Par radical aryle on entend de préférence un radical phenyle événtuellement substitués par au moins un halogène, un alkyle ayant 1 à 12 atomes de carbone, un hydroxyle, un alkoxy, une fonction nitro, un radical polyéther ou une fonction amino éventuellement protégé par un groupe acétyle ou éventuellement substitué par au moins un alkyle ayant 1 à 12 atomes de carbone.

Par radical aralkyle on entend de préférence le radical benzyle ou phénethyle événtuellement substitués par au moins un halogène, un alkyle ayant 1 à 12 atomes de carbone, un hydroxyle, un alkoxy, une fonction nitro, un radical polyéther ou une fonction amino éventuellement protégé par un groupe acétyle ou éventuellement substitué par au moins un alkyle ayant 1 à 12 atomes de carbone.

Parmi les radicaux alkényle, on préfère un radical contenant de 2 à 5 atomes de carbone et présentant une ou plusieurs insaturations éthyléniques, tel que plus particulièrement le radical allyle.

Par radical polyéther, on entend un radical ayant de 1 à 6 atomes de carbone et de 1 à 3 atomes d'oxygène ou de soufre, tel que les radicaux méthoxyméthyl éther, méthoxyéthoxyméthyl éther ou méthylthiométhyl éther.

Parmi les restes de sucre, on préfère un reste dérivant par exemple du glucose, du galactose, du mannose ou de l'acide glucuronique.

Parmi les restes d'aminoacide, on préfère un reste dérivant par exemple d'au moins un des 20 aminoacides de configuration L ou D constitutifs de protéines de mammifères. Ils sont plus particulièrement choisis parmi les restes dérivant de la lysine, de la glycine ou de l'acide aspartique.

Parmi les hétérocycles, on préfère un radical pipéridino, morpholino, pyrrolidino ou pipérazino, éventuellement substitué en position 4 par un radical alkyle en C₁-C₆ ou par un mono- ou polyhydroxyalkyle tels que définis ci-dessus.

Parmi les composés de formule (I) ci-dessus rentrant dans le cadre de la présente invention, on peut citer les suivants :
- acide 3-(2'-benzyloxy-3',5'-di-tert-butyl-6-hydroxybiphenyl-3-yl)acrylique,
- acide 3-(3',5'-di-*tert*-butyl-6-hydroxy-2'-pentyloxybiphenyl-3-yl)acrylique,
- acide 3-[3-(3-benzyloxy-5,5,8,8-tetramethyl-5,6,7,8-tetrahydro-naphthalen-2-yl)-4-methoxymethoxyphenyl]acrylique,
- acide 3-[3-(3-benzyloxy-5,5,8,8-tetramethyl-5,6,7,8-tetrahydronaphthalen-2-yl)-4-hydroxyphenyl]acrylique,
- acide 3-[3-(3-propyloxy-5,5,8,8-tetramethyl-5,6,7,8-tetrahydronaphthalen-2-yl)-4-methoxymethoxyphenyl]acrylique,
- acide 3-[3-(3-pentyloxy-5,5,8,8-tetramethyl-5,6,7,8-tetrahydronaphthalen-2-yl)-4-methoxymethoxyphenyl]acrylique,
- acide 3-(5'-adamantan-1-yl-4'-methoxy-2'-methyl-biphenyl-3-yl)acrylique,
- acide 3-(5'-adamantan-1-yl-6-hydroxy-4'-methoxy-2'-methyl-biphenyl-3-yl)-acrylique,
- acide 3-(5'-adamantan-1-yl-4'-methoxy-6-methoxymethoxy-2'-methyl biphenyl-3-yl)acrylique,
- acide 3-{4-methoxy-3-[3-(3-methoxybenzyloxy)-5,5,8,8-tetramethyl-5,6,7,8-tetrahydronaphthalen-2-yl]phenyl}acrylique,
- acide 3-{4-methoxy-3-[3-(4-methoxybenzyloxy)-5,5,8,8-tetramethyl-5,6,7,8-tetrahydronaphthalen-2-yl]phenyl}acrylique,
- acide 3-{3-[3-(6-hydroxyhexyloxy)-5,5,8,8-tetramethyl-5,6,7,8-tetrahydronaphthalen-2-yl]-4-methoxyphenyl}acrylique,
- acide 3-{3-[3-(7-hydroxyheptyloxy)-5,5,8,8-tetramethyl-5,6,7,8-tetrahydro naphthalen-2-yl]-4-methoxyphenyl}acrylique,
- acide 3-{3-[3-(5-hydroxypentyloxy)-5,5,8,8-tetramethyl-5,6,7,8-tetrahydronaphthalen-2-yl]-4-methoxyphenyl}acrylique,
- acide 3-{3-[3-(3-hydroxypropyloxy)-5,5,8,8-tetramethyl-5,6,7,8-tetrahydronaphthalen-2-yl]-4-methoxyphenyl}acrylique,
- acide 3-[3-(1-methoxymethoxy-5,5,8,8-tetramethyl-5,6,7,8-tetrahydronaphthalen-2-yl-phenyl]acrylique,
- acide 3-(3'-adamantan-1-yl-4'-hydroxybiphenyl-3-yl)acrylique,
- acide 5-[4-methoxy-3-(5,5,8,8-tetramethyl-5,6,7,8-tetrahydronaphthalen-2-yl)-phenyl]-3-methylpenta-2,4-dienoique,
- acide 5-[4-methoxymethoxy-3-(5,5,8,8-tetramethyl-5,6,7,8-tetrahydronaphthalen-2-yl)-phenyl]-3-methylpenta-2,4-dienoique,
- acide 5-[4-hydroxy-3-(5,5,8,8-tetramethyl-5,6,7,8-tetrahydro-naphthalen-2-yl)phenyl]-3-methylpenta-2,4-dienoique,
- acide 3-{3-[3-(5-tert-butoxycarbonylpentyloxy)-5,5,8,8-tetramethyl-5,6,7,8-tetrahydronaphthalen-2-yl]-4-methoxyphenyl}acrylique,
- acide 3-{3-[3-(7-tert-butoxycarbonylheptyloxy)-5,5,8,8-tetramethyl-5,6,7,8-tetrahydronaphthalen-2-yl]-4-methoxyphenyl}acrylique,
- acide 3-{3-[3-(7-carboxyheptyloxy)-5,5,8,8-tetramethyl-5,6,7,8-tetrahydronaphthalen-2-yl]-4-methoxyphenyl}acrylique,
- acide 3-{3-[3-(5-carboxypentyloxy)-5,5,8,8-tetramethyl-5,6,7,8-tetrahydronaphthalen-2-yl]-4-methoxyphenyl}acrylique,
- acide 3-{3-[3-(5-carbamoylpentyloxy)-5,5,8,8-tetramethyl-5,6,7,8-tetrahydro-naphthalen-2-yl]-4-methoxyphenyl}acrylique,
- acide 3-{3-[3-(7-carbamoylheptyloxy)-5,5,8,8-tetramethyl-5,6,7,8-tetrahydro-naphthalen-2-yl]-4-methoxyphenyl}acrylique,
- acide 3-{4-methoxy-3-[5,5,8,8-tetramethyl-3-(2-morpholin-4-yl-ethoxy)-5,6,7,8-tetrahydronaphthalen-2-yl]phenyl}acrylique,
- acide 3-{4-methoxy-3-[5,5,8,8-tetramethyl-3-(2-piperidin-1-yl-ethoxy)-5,6,7,8-tetrahydronaphthalen-2-yl]-phenyl}acrylique,
- acide 3-{4-methoxy-3-[3-(2-methoxymethoxybenzyloxy)-5,5,8,8-tetramethyl-5,6,7,8-tetrahydronaphthalen-2-yl]-phenyl}acrylique,
- acide 3-{4-methoxy-3-[3-(3-methoxymethoxybenzyloxy)-5,5,8,8-tetramethyl-5,6,7,8-tetrahydronaphthalen-2-yl]phenyl}acrylique,
- acide 3-{4-methoxy-3-[3-(4-methoxymethoxybenzyloxy)-5,5,8,8-tetramethyl-5,6,7,8-tetrahydronaphthalen-2-yl]phenyl}acrylique,
- acide 3-{4-methoxy-3-[3-(3-hydroxybenzyloxy)-5,5,8,8-tetramethyl-5,6,7,8-tetrahydronaphthalen-2-yl]phenyl}acrylique,
- acide 3-[4-fluoro-3-(3,5,5,8,8-pentamethyl-5,6,7,8-tetrahydronaphthalen-2-yl)phenyl]acrylique,
- acide 3-{4-hydroxy-3-[3-(3-methoxybenzyloxy)-5,5,8,8-tetramethyl-5,6,7,8-tetrahydronaphthalen-2-yl]phenyl}acrylique,
- acide 3-{4-hydroxy-3-[3-(4-methoxybenzyloxy)-5,5,8,8-tetramethyl-5,6,7,8-tetrahydronaphthalen-2-yl]-phenyl-acrylique,
- acide 3-{4-hydroxy-3-[3-(4-fluorobenzyloxy)-5,5,8,8-tetramethyl-5,6,7,8-tetrahydro-naphthalen-2-yl]phenyl}acrylique,
- acide 3-{4-hydroxy-3-[3-(4-chlorobenzyloxy)-5,5,8,8-tetramethyl-5,6,7,8-tetrahydro-naphthalen-2-yl]phenyl}acrylique,
- acide 3-[4-hydroxy-3-(3-methoxymethoxy-5,5,8,8-tetramethyl-5,6,7,8-tetrahydro-naphthalen-2-yl)-phenyl]acrylique,
- acide 3-(3',5'-di-*tert*-butyl-6-hydroxy-2'-propyloxybiphenyl-3-yl)acrylique,
- acide 3-(3',5'-di-*tert*-butyl-6-hydroxy-2'-butyloxybiphenyl-3-yl)acrylique,
- acide 3-(2'-butoxy-3',5'-di-tert-butyl-6-methoxy-biphenyl-3-yl)acrylique,
- acide 3-(3',5'-di-*tert*-butyl-6-methoxy-2'-propoxy-biphenyl-3-yl)acrylique,
- acide 3-[4-hydroxy-3-(5,5,8,8-tetramethyl-4-propoxy-5,6,7,8-tetrahydronaphthalen-2-yl)phenyl]acrylique,
- acide 3-[3-(3-benzyloxy-5,5,8,8-tetramethyl-5,6,7,8-tetrahydronaphthalen-2-yl)-4-methoxyphenyl]acrylique,
- 3-[3-(3-benzyloxy-5,5,8,8-tetramethyl-5,6,7,8-tetrahydronaphthalen-2-yl)-4-methoxyphenyl]acrylate d'ethyle,
- acide 3-methyl-5-[3-(3,5,5,8,8-pentamethyl-5,6,7,8-tetrahydro-naphthalen-2-yl)-phenyl]-penta-2,4-dienoique,
- 3-[3-(3-benzyloxy-5,5,8,8-tetramethyl-5,6,7,8-tetrahydro-naphthalen-2-yl)-4-methoxy-phenyl]prop-2-en-1-ol,
- 3-[3-(3-benzyloxy-5,5,8,8-tetramethyl-5,6,7,8-tetrahydro-naphthalen-2-yl)-4-methoxyphenyl]propenal,
- N-ethyl 3-[3-(3-benzyloxy-5,5,8,8-tetramethyl-5,6,7,8-tetrahydronaphthalen-2-yl)-4-methoxy-phenyl]acrylamide,
- 3-[3-(3-benzyloxy-5,5,8,8-tetramethyl-5,6,7,8-tetrahydro-naphthalen-2-yl)-4-methoxy-phenyl]-1-morpholin-4-yl-propenone,
- N-(4-hydroxyphenyl)-3-[3-(3-benzyloxy-5,5,8,8-tetramethyl-5,6,7,8-tetrahydro-naphthalen-2-yl)-4-methoxy-phenyl]acrylamide,
- acide 5-(5'-adamantan-1-yl-4'-methoxy-2'-methyl-biphenyl-3-yl)-3-methyl-penta-2,4-dienoïque,
- acide 5-[4-methoxymethoxy-3-(3,5,5,8,8-pentamethyl-5,6,7,8-tetrahydronaphthalen-2-yl)-phenyl]-3-methyl-penta-2,4-dienoïque,
- acide 5-[4-hydroxy-3-(3,5,5,8,8-pentamethyl-5,6,7,8-tetrahydronaphthalen-2-yl)-phenyl]-3-methyl-penta-2,4-dienoïque,
- acide 4-[3-(3,5,5,8,8-pentamethyl-5,6,7,8-tetrahydro-naphthalen-2-yl)-phenyl]-penta-2,4-dienoïque,
- acide 5-(3',5'-di*-tert*-butyl-2'-methoxy-biphenyl-3-yl)-3-methyl-penta-2,4-dienoïque,
- acide 5-(3',5'-di-*tert*-butyl-2'-propoxy-biphenyl-3-yl)-3-methyl-penta-2,4-dienoïque,
- acide 5-(2'-butoxy-3',5'-di-*tert*-butyl-biphenyl-3-yl)-3-methyl-penta-2,4-dienoïque,
- acide 5-(2'-butoxy-3',5'-di*-tert*-butyl-6-hydroxy-biphenyl-3-yl)-3-methyl-penta-2,4-dienoïque,
- acide 5-(3',5'-di-*tert*-butyl-6-hydroxy-2'-propoxy-biphenyl-3-yl)-3-methyl-penta-2,4-dienoïque,
- acide 5-(3',5'-di*-tert*-butyl-6-hydroxy-2'-methoxy-biphenyl-3-yl)-3-methyl-penta-2,4-dienoïque.

Selon la présente invention les composés de formule (I) plus particulièrement préférés sont ceux pour lesquels au moins l'une des, et de préférence toutes les, conditions suivantes est remplie :
- R₁ représente le radical -CO-R₃
- Ar₂ représente les radicaux de formule (d) ou (e)
- R₁₁ représente le radical -O-R₂₁,
- R₇ représente le radical -O-R₂₀

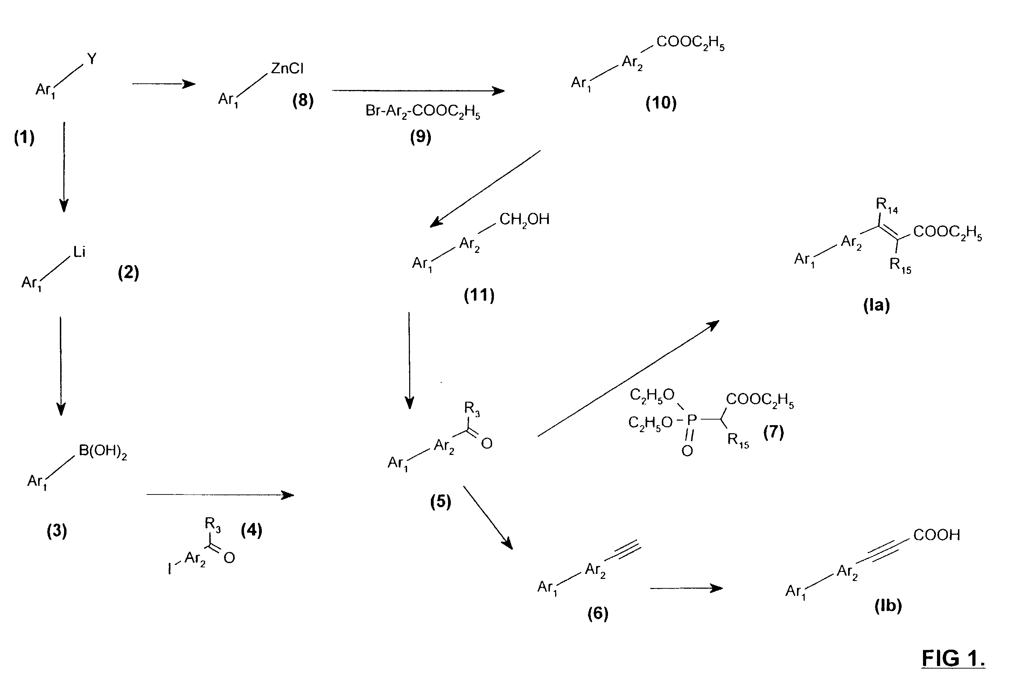

La présente invention a également pour objet les procédés de préparations des composés de formule (I), en particulier selon le schéma réactionnel donné à la figure 1.

Les dérivés de formule (la) peuvent être obtenus (FIG. 1) à partir des dérivés aldéhydiques ou cétoniques (5), selon une réaction de type Horner avec un lithio ou sodio dérivé d'un phosphonate (7). Les composés carbonylés (5) pouvant être obtenus :
- soit par une réaction de couplage entre un acide boronique (3) et un dérivé halogéné (4). Cette réaction est effectuée en présence d'un catalyseur au palladium, par exemple le tétrakis(triphénylphosphine)palladium selon les conditions décrites par N. Miyaura et al. Synthetic Communications (1981) 11(7), 513-519.

Le dérivé acide boronique (3) peut être obtenu par exemple, à partir du dérivé halogéné (1) par transformation en lithien (2) puis réaction avec le triméthyl borate et hydrolyse.
- soit par une réaction de couplage entre un dérivé zincique (8) et un dérivé ester-halogéné (9) en présence d'un catalyseur par exemple un dérivé du palladium ou de nickel (NiCl₂ dppe) puis transformation de la fonction ester (10) en alcool (11) et oxydation en aldéhyde (5).

Les composés de formule (Ib) peuvent être obtenus (FIG. 1) à partir du dérivé acétylénique (6) par réaction avec le n-butyllithium puis carboxylation en présence de CO₂.

Les composés acétyléniques (6) pouvant être obtenus soit :
- à partir des dérivés aldéhydiques (5) (lorsque R₃ est un atome d'hydrogène), par réaction avec le tétrabromure de carbone et la triphenylphosphine pour donner un dérivé 2',2'-dibromostyrene qui est transformé en dérivé acétylénique par une base non nucléophile tel le n-butyllithium dans un solvant aprotique tel le tetrahydrofuranne,
- à partir des dérivés cétoniques (5) (lorsque R₃ est un alkyle ayant de 1 à 12 atomes de carbone) par une suite de réactions comprenant le traitement avec une base tel le diisopropylamidure de lithium puis avec un chlorure de dialkylphosphate et de nouveau avec le diisopropylamidure de lithium.

Lorsque R₁ représente le radical -COOH, les composés sont préparés:
- soit en protégeant R₁ par un groupe protecteur de type alkyle, allylique ou tertiobutylique.

Le passage à la forme libre peut être effectué:
- dans le cas d'un groupe protecteur alkyle, au moyen de soude ou d'hydroxyde de lithium dans un solvant alcoolique tel le méthanol ou dans le THF.
- dans le cas d'un groupe protecteur allylique, au moyen d'un catalyseur tel certains complexes de métaux de transition en présence d'une amine secondaire telle la morpholine.
- dans le cas d'un groupement protecteur de type tertiobutylique au moyen d'iodure de triméthylsilane.

Lorsque R₁ représente une fonction alcool les composés peuvent être obtenus:
- à partir des dérivés aldéhydiques correspondants par action d'un hydrure alcalin, tel le borohydrure de sodium, dans un solvant alcoolique (par exemple le méthanol).
- à partir des dérivés acides par réduction avec l'hydrure double de lithium et d'aluminium.

Lorsque R₁ représente une fonction aldéhyde les composés peuvent être obtenus à partir des dérivés alcools par oxydation en présence d'oxyde de manganèse, de pyridinium dichromate ou du réactif de Swern.

Lorsque R₁ représente une fonction amide les composés peuvent être obtenus à partir des dérivés carboxyliques correspondants par réaction avec des amines aliphatiques, aromatiques, hétérocycliques soit par l'intermédiaire d'un chlorure d'acide ou en présence de dicyclohexylcarbodiimide ou de carbonyldiimidazole.

Les composés de l'invention se lient aux récepteurs RXRs et possèdent une activité antagoniste.

Les propriétés de binding et de transactivation comme agoniste aux récepteurs RXRs peuvent être déterminées par des méthodes connues dans l'art, comme par exemple : LEVIN et al, Nature 1992, **355,** 359-61 ; ALLENBY et al, Proc. Natl. Acad. Sci., 1993, **90,** 30-4.

L'activité antagoniste RXRα peut être évaluée dans le test de transactivation par détermination de la dose (IC₅₀) qui inhibe de 50% l'activité transactivatrice d'un agoniste sélectif RXRα : l'acide 6-[1-(3,5,5,8,8-pentaméthyl-5,6,7,8-tétrahydronaphtalen-2-yl)cyclopropyl]nicotinique (CD 3127) selon le protocole suivant :

Les cellules Hela sont co-transfectées avec un vecteur d'expression codant pour RXRα (p565-RXRα) et un plasmide rapporteur contenant l'élément de réponse 1/2 CRBP II cloné en amont du promoteur hétérologue de la thymidine kinase et du gène rapporteur de la chloramphénicolm-acétyl-transfèrase (CAT). Dix-huit heures après co-transfection les cellules sont traitées avec une concentration fixe du CD 3127 et des concentrations croissantes de la molécule à évaluer. Après vingt-quatre heures de traitement le dosage de l'activité CAT est effectué par ELISA. La concentration fixe de CD3127 utilisée est 10⁻⁸M et correspond à son EC₅₀.

La présente invention a ainsi pour objet à titre de médicament les composés de formule (I) telle que définie ci-dessus.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique, ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation, pour prévenir ou pour réparer les vergetures, ou encore pour favoriser la cicatrisation,
10) pour lutter contre les troubles de la fonction sébacée tels que l'hyperséborrhée de l'acné ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose, l'hypertension, le diabète non-insulino dépendant ainsi que l'obésité,
17) dans le traitement de désordres cutanés dus à une exposition aux rayonnements U.V..

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutase, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,5-diphényl-imidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### A. EXEMPLES DE COMPOSES

### EXEMPLE 1

### Acide 3-(3',5'-di-tert-butyl-2'-methoxybiphenyl-3-yl)acrylique.

### (a) 2-bromo-46-di-tert-butylanisole.

Dans un ballon et sous courant d'azote, on introduit 6 g (21 mmoles) de 2-bromo-4,6-di-*tert*-butylphenol et 60 ml de DMF. On ajoute par petites quantités 694 mg (29 mmoles) d'hydrure de sodium (80% dans l'huile) et agite jusqu'à cessation du dégagement gazeux. On ajoute ensuite 1,45 ml (23,2 mmoles) de iodométhane et agite à température ambiante pendant trois heures. On verse le milieu réactionnel dans l'eau glacée, extrait avec de l'acétate d'éthyle, décante la phase organique, sèche sur sulfate de magnésium, évapore. Le résidu obtenu est purifié par chromatographie sur colonne de silice, élué avec de l'heptane. Après évaporation des solvants, on recueille 6,1 g (98%) de produit attendu sous forme d'une huile incolore.
RMN ¹H (d, CDCl₃) : 1,3 (s, 9H); 1,4 (s, 9H); 3,9 (s, 3H); 7,3 (d, *J* = 2,3 Hz, 1H); 7,4 (d, *J* = 2,3 Hz, 1H).

### (b) acide 2-méthoxy-3,5-di-tert-butylphenylboronique.

Dans un tricol et sous courant d'azote, on introduit 6 g (20,1 mmoles) de 2-bromo-4,6-di*-tert*-butylanisole et 50 ml de THF. A -78°C, ajoute goutte à goutte 9,7 ml (24,1 mmoles) de n-butyllithium (2,5M dans l'hexane) et agite 15'. A cette même température, on ajoute 7 ml (30,1 mmoles) de triisopropylborate et agite pendant 2 heures. A -50°C on ajoute 20 ml d'acide chlorhydrique (1N) et laisse remonter à température ambiante. On extrait le milieu réactionnel avec de l'éther éthylique, décante la phase organique, sèche sur sulfate de magnésium, évapore. On triture le solide obtenu dans l'heptane, filtre et sèche. On recueille 4 g (75%) d'acide boronique attendu qui est utilisé tel quel pour la suite de la synthèse.
RMN ¹H (d, CDCl₃) : 1,3 (s, 9H); 1,4 (s, 9H); 3,8 (s, 3H); 6,0 (s, 2H); 7,5 (d, *J* = 2,5 Hz, 1H); 7,7 (d, *J* = 2,5 Hz, 1H).

### ( c) acide 3-(3',5'-di-tert-butyl-2'-methoxybiphenyl-3-yl)acrylique.

Dans un tricol et sous courant d'azote, on introduit 2,2 g (8,3 mmoles) d' acide 2-méthoxy-3,5-di*-tert*-butylphenylboronique 942 mg (4,15 mmoles) d'acide 3-bromocinnamique et 70 ml de DME. On ajoute goutte à goutte 11,4 ml d'une solution aqueuse de carbonate de potassium (2M) et dégaze le milieu réactionnel. On ajoute ensuite 144 mg (0,12 mmole) de tétrakistriphénylphosphinepalladium(0) et chauffe à reflux pendant 20 heures. On ajoute au milieu réactionnel de l'eau et de l'acétate d'éthyle, acidifie à pH 1 avec de l'acide chlorhydrique. On décante la phase organique, lave à l'eau, sèche sur sulfate de magnésium, évapore. Le résidu obtenu est purifié par chromatographie sur colonne de silice élué avec un mélange d'heptane et d'acétate d'éthyle (75-25). On recueille après évaporation des solvants, 990 mg (66%) d'acide 3-(3',5'-di-*tert*-butyl-2'-methoxybiphenyl-3-yl)acrylique de point de fusion 182-3°C.
RMN ¹H (d, DMSO): 1,1 (s, 9H); 1,2 (s, 9H); 3,0 (s, 3H); 6,4 (d, *J* = 16 Hz, 1H); 7,0 (d, *J* = 2,3 Hz, 1H); 7,1 (d, *J* = 2,3 Hz, 1H); 7,3-7,6 (m, 5H).

### EXEMPLE 2

### Acide 3-(2'-benzyloxy-3',5'-di-tert-butyl-6-hydroxybiphenyl-3-yl)acrylique

### (a) 3-bromo-4-hydroxybenzaldéhyde.

Dans un tricol de 500 ml, sous atmosphère d'azote, on introduit 15 g (123 mmoles) de 4-hydroxybenzaldéhyde, 687 mg (12,3 mmoles) de fer en poudre, 200 ml de dichlorométhane et 10 ml de THF. On refroidit à 0°C, ajoute goutte à goutte 19,6 g (123 mmoles) de brome et agite à la température ambiante pendant une heure. On ajoute une solution saturée de thiosulfate de sodium, évapore le dichlorométhane, extrait par de l'acétate d'éthyle, décante la phase organique, lave à l'eau jusqu'à pH neutre, sèche sur sulfate de magnésium et évapore les solvants. Le résidu obtenu est purifié par chromatographie sur colonne de silice élué par un système éluant composé de 80% d'heptane et 20% de dichlorométhane. Après évaporation des solvants, on recueille 9,7g (39%) d'une poudre cotonneuse blanche de point de fusion 121-124°C.

### (b) 3-bromo-4-méthoxyméthoxybenzaldéhyde.

Dans un ballon de 250 ml, sous atmosphère d'azote, on introduit 9 g (44,7 mmoles) de 3-bromo-4-hydroxybenzaldéhyde, 30 ml de THF et 30 ml de DMF. On ajoute 1,48 g (49,2 mmoles) d'hydrure de sodium à 80%, par petites fractions, agite trente minutes à température ambiante et coule goutte à goutte 4,32 g (52,7 mmoles) de chlorure de méthoxyméthyle. Le milieu réactionnel est agité pendant une heure à température ambiante, acidifié par de l'acide chlorhydrique 1N et extrait par de l'acétate d'éthyle. On décante la phase organique, lave à l'eau jusqu'à pH neutre, sèche sur sulfate de magnésium et évapore les solvants. On recueille 11,5g (100%) d'une huile orange.

### (c) 3-(3-bromo-4-méthoxyméthoxyphényl)acrylate d'éthyle.

Dans un tricol et sous courant d'azote, on introduit 12 g (53 mmoles) de triéthylphosphonoacétate et 50 ml de THF. On ajoute par petites quantités 2,45 g (81,6 mmoles) d'hydrure de sodium (80% dans l'huile) et agite jusqu'à cessation du dégagement gazeux. On ajoute ensuite une solution de 10 g (40,8 mmoles) de 3-bromo-4-méthoxyméthoxybenzaldéhyde dans 50 ml de THF et agite à température ambiante pendant une heure. On verse le milieu réactionnel dans l'eau, acidifie avec de l'acide chlorhydrique, extrait avec de l'acétate d'éthyle, décante la phase organique, sèche sur sulfate de magnésium, évapore. Le résidu obtenu est purifié par chromatographie sur colonne de silice élué avec un mélange d'heptane et d'acétate d'éthyle (90-10) On recueille après évaporation des solvants 9,6 g (74%) de 3-(3-bromo-4-méthoxyméthoxyphényl)acrylate d'éthyle de point de fusion 63-65°C.

### (d) 2-benzyloxy-3,5-di-tert-butylbromobenzene.

De manière analogue à l'exemple 1(a) par réaction de 6 g (21 mmoles) de 2-bromo-4,6-di*-tert*-butylphenol avec 2,8 ml (23,2 mmoles) de bromure de benzyle, on obtient 7,4 g (94%) de produit attendu sous forme d'une huile incolore.
RMN ¹H (d, CDCl₃) : 1,3 (s, 9H); 1,4 (s, 9H); 5,1 (s, 2H); 7,25-7,45 (m, 5H); 7,55-7,6 (m, 2H).

### (e) acide 2-benzyloxy-3,5-di-tert-butylphenylboronique.

De manière analogue à l'exemple 1(b) par réaction de 7,2 g (19,3 mmoles) de 2-benzyloxy-3,5-di*-tert*-butylbromobenzene avec 6,7 ml (29 mmoles) de triisopropylborate, on obtient 4,29 g(65%) d'acide boronique attendu.
RMN ¹H (d, CDCl₃) : 1,35 (s, 9H); 1,45 (s, 9H); 5,1 (s, 2H); 5,9 (s, 2H); 7,3-7,55 (m, 6H); 7,7 (d, *J* = 2,5 Hz, 1H).

### (f) 3-(2'-benzyloxy-3',5'-di-tert-butyl-6-méthoxymethoxybiphenyl-3-yl)acrylate d'éthyle.

De manière analogue à l'exemple 1(c) par réaction de 3,24 g (9,53 mmoles) d' acide 2-benzyloxy-3,5-di-*tert*-butylphenylboronique avec 2 g (6,35 mmoles) de 3-(3-bromo-4-méthoxyméthoxyphényl)acrylate d'éthyle, on obtient 2,4 g (71%) d'ester éthylique attendu .
RMN ¹H (d, CDCl₃) : 1,1 (t, *J* = 7 Hz, 3H); 1,35 (s, 9H); 1,45 (s, 9H); 3,5 (s, 3H); 4, 25 (q, *J* = 7 Hz, 2H); 4,5 (s, 2H); 5,1 (s, 2H); 6,3 (d, *J* = 16 Hz, 1H); 6,9-7,0 (m, 2H); 7,15-7,25 (m, 5H); 7,4-7,5 (m, 2H); 7,6-7,7 (m, 2H).

### (g) 3-(2'-benzyloxy-3',5'-di-tert-butyl-6-hydroxybiphenyl-3-yl)acrylate d'éthyle.

Dans un ballon, on introduit 2,14 g (4 mmoles) de l'ester éthylique précédent 10 ml de THF et 10 ml d'éthanol. On ajoute 1 ml d'acide sulfurique concentré et agite à température ambiante pendant 24 heures. On verse le milieu réactionnel dans l'eau, extrait avec de l'acétate d'éthyle, décante la phase organique, lave à l'eau, sèche sur sulfate de magnésium, évapore. Le résidu obtenu est trituré dans l'heptane, filtré, séché. On recueille 1,93 g (100%) du produit attendu.
RMN ¹H (d, CDCl₃) : 1,3 (t, *J* = 7 Hz, 3H); 1,35 (s, 9H); 1,45 (s, 9H); 4,25 (q, *J* = 7 Hz, 2H); 4,5-4,7 (m, 2H); 6,4 (d, *J* = 16 Hz, 1H); 7,0 (d, *J* = 8 Hz, 1H); 7,0-7,1 (m, 2H); 7,25 (s, 1H); 7,3-7,4 (m, 3H); 7,45-7,50 (m, 2H); 7,6 (s, 1H); 7,7 (d, *J* = 6Hz, 1H).

### (h) acide 3-(2'-benzyloxy-3',5'-di-tert-butyl-6-hydroxybiphenyl-3-yl)acrylique.

Dans un ballon, on introduit 1,93 g (4 mmoles) de 3-(2'-benzyloxy-3',5'-di-*tert*-butyl-6-hydroxybiphenyl-3-yl)acrylate d'éthyle 20 ml de THF 2 ml de méthanol et 4 ml d'une solution de soude (10N). On chauffe à reflux pendant 12 heures, verse le milieu réactionnel dans l'eau, acidifie à pH 1 avec de l'acide chlorhydrique, extrait avec de l'acétate d'éthyle, décante la phase organique, sèche sur sulfate de magnésium, évapore. Le résidu obtenu est trituré dans l'heptane, filtré, séché. On recueille 1,6 g (87%) d'acide 3-(2'-benzyloxy-3',5'-di-tert-butyl-6-hydroxybiphenyl-3-yl)acrylique de point de fusion 230-1°C.
RMN ¹H (d, CDCl₃) : 1,3 (s, 9H); 1,5 (s, 9H); 4,6 (s, 2H); 6,3 (d, *J* = 16 Hz, 1H); 7,0-7,1 ( m, 3H); 7,2-7,3 (m, 4H); 7,4-7,5 (m, 2H); 7,6 (s, 1H); 7,7 (d, *J* = 16 Hz, 1H); 8,1 (m, 1H).

### EXEMPLE 3

### Acide 3-(3',5'-di-tert-butyl-6-hydroxy-2'-pentyloxybiphenyl-3-yl)acrylique

### (a) 3,5-di-tert-butyl-2-pentyloxybromobenzene.

De manière analogue à l'exemple 1(a) par réaction de 6 g (21 mmoles) de 2-bromo-4,6-di-*tert*-butylphenol avec 3 ml (23,2 mmoles) de iodopentane, on obtient 7,3 g (98%) de produit attendu sous forme d'une huile incolore.
RMN ¹H (d, CDCl₃) : 0,9 (t, *J* = 7 Hz, 3H); 1,35 (s, 9H); 1,45 (s, 9H); 1,3-1,5 (m, 4H); 1,8-2,0 (m, 2H); 4,0 (q, *J* = 7 Hz, 2H); 7,3 (d, *J* = 2,5 Hz, 1H); 7,4 (d, *J* = 2,5 Hz, 1H).

### (b) acide 35-di-tert-butyl-2-pentyloxyphenylboronique.

De manière analogue à l'exemple 1(b) par réaction de 7,26 g (20,5 mmoles) de 35-di*-tert*-butyl-2-pentyloxybromobenzene avec 7,1 ml (30,75 mmoles) de triisopropylborate, on obtient 6,4 g (98%) d'acide boronique attendu.
RMN ¹H (d, CDCl₃) : 0,9 (t, *J* = 7 Hz, 3H); 1,2-1,5 (m, 4H); 1,35 (s, 9H); 1,45 (s, 9H); 1,85-1,95 (m, 2H); 3,8-4,0 (m, 2H); 6,1 (s, 1H); 6,8-7,6 (m, 3H).

### (c) 3-(3',5'-di-tert-butyl-6-méthoxymethoxy-2'-pentyloxybiphenyl-3-yl)acrylate d'éthyle.

De manière analogue à l'exemple 1(c) par réaction de 3 g (9,5 mmoles) d' acide 3,5-di-*tert*-butyl-2-pentyloxyphenylboronique avec 2 g (6,35 mmoles) de 3-(3-bromo-4-méthoxyméthoxyphényl)acrylate d'éthyle, on obtient 2,1 g (65%) d'ester éthylique attendu .
RMN ¹H (d, CDCl₃) : 0,7 (t, *J* = 7 Hz, 3H); 1,0 (m, 4H); 1,2 (m, 5H); 1,25 (s, 9H); 1,35 (s, 9H); 3,2-3,3 (m, 2H); 3,35 (s, 3H); 4,2 (q, *J* = 7 Hz, 2H); 5,1 (s, 2H); 6,25 (d, *J* = 16 Hz, 1H); 7,05 (d, *J* = 2,5 Hz, 1H); 7,15 (d, *J* = 8,5 Hz, 1H); 7,3 (d, J = 2,5 Hz, 1H); 7,4 (dd, *J* = 8,5 Hz et *J* = 2,5 Hz, 1H); 7,5 (d, *J* = 2,5 Hz, 1H); 7,6 (d, *J* = 16 Hz, 1H).

### (d) 3-(3',5'-di-tert-butyl-6-hydroxy-2'-pentyloxybiphenyl-3-yl)acrylate d'éthyle.

De manière analogue à l'exemple 2(g) à partir de 1,84 g (3,6 mmoles) de l'ester éthylique précédent, on obtient 1,6 g (88%) de produit attendu.
RMN ¹H (d, CDCl₃) : 0,85 (t, *J* = 7 Hz, 3H); 0,9-1,3 (m, 7H); 1,35 (s, 9H); 1,45 (s, 9H); 1,45-1,6 (m, 2H); 3,5-3,65 (m, 2H); 4,3 (q, *J* = 7 Hz, 2H); 6,35 (d, *J* = 16 Hz, 1H); 7,05 (d, *J =* 8 Hz, 1H); 7,15 (d, *J* = 2,4 Hz, 1H); 7,4 (d, *J* = 2,4 Hz, 1H); 7,5-7,6 (m, 2H); 7,7 (d, *J* = 16 Hz, 1H).

### (e) acide 3-(3',5'-di-tert-butyl-6-hydroxy-2'-pentyloxybiphenyl-3-yl)acrylique.

De manière analogue à l'exemple 2(h) à partir de 1,62 g (3,2 mmoles) de 3-(3',5'-di-*tert*-butyl-6-hydroxy-2'-pentyloxybiphenyl-3-yl)acrylate d'éthyle, on obtient 1,2 g (86%) d'acide 3-(3',5'-di-*tert*-butyl-6-hydroxy-2'-pentyloxybiphenyl-3-yl)acrylique de point de fusion 166-7°C.
RMN ¹H (d, CDCl₃) : 0,8 (t, *J* = 7 Hz, 3H); 1,1-1,25 (m, 4H); 1,35 (s, 9H); 1,45 (s, 9H); 1,6 (t, *J* = 7 Hz, 2H); 3,5-3,6 (m, 2H); 6,4 (d, *J* = 16 Hz, 1H); 7,1 (d, *J* = 8 Hz, 1H); 7,2 (s,1H); 7,4 (m, 2H); 7,5-7,6 (m, 2H); 7,8 (d, *J* = 16 Hz, 1H).

### EXEMPLE 4

### Acide 3-(3',5'-Di-tert-butyl-6-hydroxy-2'-methoxybiphenyl-3-yl)acrylique

### (a) 3-(-3',5'-di-tert-butyl-2'-méthoxy-6-méthoxymethoxybiphenyl-3-yl)acrylate d'éthyle.

De manière analogue à l'exemple 1(c) par réaction de 2,18 g (8,26 mmoles) d' acide 3,5-di-*tert*-butyl-2-pentyloxyphenylboronique avec 2 g (6,35 mmoles) de 3-(3-bromo-4-méthoxyméthoxyphényl)acrylate d'éthyle, on obtient 2 g (70%) d'ester éthylique attendu .
RMN ¹H (d, CDCl₃) : 1,2-1,3 (m, 3H); 1,35 (s, 9H); 1,45 (s, 9H); 3,3 (s, 3H); 3,4 (s, 3H); 3,8 (q, *J* = 7 Hz, 2H); 5,15 (s, 2H); 6,35 (d, *J* = 16 Hz, 1H); 7,1 (d, *J* = 2 Hz, 1H); 7,2 (d, *J* = 8 Hz, 1H); 7,35 (d, *J* = 2 Hz, 1H); 7,5 (dd, *J* = 8 Hz et *J* = 2 Hz, 1H); 7,6 (d, *J* = 2 Hz, 1H); 7,7 (d, *J* = 16 Hz, 1H).

### (b) 3-(3',5'-di-tert-butyl-6-hydroxy-2'-méthoxybiphenyl-3-yl)acrylate d'éthyle.

De manière analogue à l'exemple 2(g) à partir de 1,45 g (3,17 mmoles) de l'ester éthylique précédent, on obtient 1,25 g (96%) de produit attendu.
RMN ¹H (d, CDCl₃) : 1,1-1,2 (m, 4H); 1,35 (s, 9H); 1,45 (s, 9H); 3,5 (s, 3H); 4,25 (q, *J* = 7 Hz, 2H); 6,35 (d, *J* = 16 Hz, 1H); 7,05 (d, *J* = Hz, 1H); 7,15 (d, *J* = 2,3 Hz, 1H); 7,4 (d, *J* = 2,3 Hz, 1H); 7,5-7,6 (m, 2H); 7,7 (d, *J* = 16 Hz, 1H).

### (c) acide 3-(3',5'-di-tert-butyl-6-hydroxy-2'-méthoxybiphenyl-3-yl)acrylique.

De manière analogue à l'exemple 2(h) à partir de 1,23 g (3 mmoles) de 3-(3',5'-di-tert-butyl-6-hydroxy-2'-méthoxybiphenyl-3-yl)acrylate d'éthyle, on obtient 800 mg (69%) d'acide 3-(3',5'-di-*tert*-butyl-6-hydroxy-2'-méthoxybiphenyl-3-yl)acrylique de point de fusion 166-7°C.
RMN ¹H (d, CDCl₃) : 1,35 (s, 9H); 1,45 (s, 9H); 3,5 (s, 3H); 6,4 (d, *J* = 16 Hz, 1H); 7,1 (d, *J* = 8 Hz, 1H); 7,2 (d, *J* = 2,5 Hz, 1H); 7,45 (d, *J* = 2,5 Hz, 1H); 7,5-7,6 (m, 2H); 7,8 (d, *J* = 16 Hz, 1H).

### EXEMPLE 5

### Acide 3-[3-(3-benzyloxy-5,5,8,8-tetramethyl-5,6,7,8-tetrahydronaphthalen-2-yl)-4-methoxymethoxyphenyl]acrylique.

### (a) 3-benzyloxy-2-bromo-5,5,8,8-tetramethyl-5,6,7,8-tetrahydronaphthalene.

De manière analogue à l'exemple 1(a) par réaction de 5 g (25 mmoles) de 3-hydroxy-2-bromo-5,5,8,8-tetramethyl-5,6,7,8-tetrahydronaphthalene avec 3,5 ml (29,5 mmoles) de bromure de benzyle on obtient 6,15 g (66%) de produit attendu.
RMN ¹H (CDCl₃) : 1.20 (s, 6H), 1.24 (s, 6H), 1.64 (s, 4H), 5.12 (s, 2H), 6.82 (s, 1H Ar), 7.21 à 7.50 (m, 6H Ar).

### (b) acide 3-benzyloxy -5,5,8,8-tetramethyl-5,6,7,8-tetrahydro-2-naphthylboronique.

De manière analogue à l'exemple 1(b) par réaction de 6,15 g (16,5 mmoles) de 3-benzyloxy-2-bromo-5,5,8,8-tetramethyl-5,6,7,8-tetrahydronaphthalene avec 5,7 ml (24,7 mmoles) de triisopropylborate, on obtient 3,5 g (62%) d'acide boronique attendu de point de fusion 125-6°C.
RMN ¹H (CDCl₃) : 1.26 (s, 6H), 1.29 (s, 6H), 1.68 (s, 4H), 5.12 (s, 2H), 5.83 (s, 2H), 6.88 (s, 1H Ar), 7.35 à 7.43 (m, 5H Ar), 7.80 (s, 1H Ar).

### (c) 3-[3-(3-benzyloxy-5,5,8,8-tetramethyl-5,6,7,8-tetrahydro-naphthalen-2-yl)-4-methoxymethoxy-phenyl]acrylate d'éthyle.

De manière analogue à l'exemple 1(c) par réaction de 3,4 g (10 mmoles) d' acide 3-benzyloxy -5,5,8,8-tetramethyl-5,6,7,8-tetrahydro-2-naphthylboronique avec 3,17 g (10 mmoles) de 3-(3-bromo-4-méthoxyméthoxy-phényl)acrylate d'éthyle, on obtient 3 g (56%) d'ester éthylique attendu.
RMN ¹H (CDCl₃) : 1.27 (s,12 H), 1.33 (t, *J* = 7.1Hz, 3H) 1.69 (s,4 H), 3.31 (s, 3H), 4.25 (q, *J* = 7.1Hz, 2H), 5.01 (s, 2H), 5.06 (s, 2H), 6.32 (d, *J*=15.9Hz,1 H), 6.89 (s, 1H Ar), 7.19 (s, 1H Ar), 7.21 à 7.31 (m, 6H Ar), 7.46 (dd, *J* = 8.6Hz, *J* = 2.3Hz, 1H Ar), 7.52 (d, *J* =2.3Hz, 1H Ar), 7.567 (d, *J* =15.9Hz, 1H).

### (d) acide 3-[3-(3-benzyloxy-5,5,8,8-tetramethyl-5,6,7,8-tetrahydro-naphthalen-2-yl)-4-methoxymethoxyphenyl]acrylique.

De manière analogue à l'exemple 2(h) à partir de 1,3 g (2,46 mmoles) de l'ester éthylique précédent, on obtient 1 g (90%) d' acide 3-[3-(3-benzyloxy-5,5,8,8-tetramethyl-5,6,7,8-tetrahydro-naphthalen-2-yl)-4-methoxymethoxyphenyl]acrylique de point de fusion 161-3°C.
RMN ¹H (CDCl₃) : 1.27 à 1.28 (m,12 H), 1.69 (s,4 H), 3.32 (s, 3H), 5.01 (s, 2H), 5.07 (s, 2H), 6.33 (d, *J*=16Hz,1 H), 6.70 (s, 1H Ar), 7.20 (s, 1H Ar), 7.21 à 7.31 (m, 6H Ar), 7.48 (dd, *J* = 8.6Hz, *J* = 2.1Hz, 1H Ar), 7.55 (d, *J*=2.Hz, 1H Ar), 7.55 (d, *J* =2Hz, 1H).
RMN ¹³C (CDCl₃) :.1099, 32.11, 33.91, 34.69, 35.33, 56.20; 70.76, 95.02, 111.13, 115.23, 115.36, 125.11, 125.76, 12708, 127.61, 127.68, 128.49, 129.25, 129.76, 129.83, 132.29, 137.37, 137.72, 145.74, 147.05, 153.85, 157.49, 172.54

### EXEMPLE 6

### Acide 3-[3-(3-benzyloxy-5,5,8,8-tetramethyl-5,6,7,8-tetrahydronaphthalen-2-yl)-4-hydroxyphenyl]acrylique

### (a) 3-[3-(3-benzyloxy-5,5,8,8-tetramethyl-5,6,7,8-tetrahydronaphthalen-2-yl)-4-hydroxyphenyl]acrylate d'éthyle.

De manière analogue à l'exemple 2(g) à partir de 1,8 g (3,4 mmoles) de 3-[3-(3-benzyloxy-5,5,8,8-tetramethyl-5,6,7,8-tetrahydronaphthalen-2-yl)-4-methoxy methoxyphenyl]acrylate d'éthyle, on obtient 1,2 g (72%) de 3-[3-(3-benzyloxy-5,5,8,8-tetramethyl-5,6,7,8-tetrahydronaphthalen-2-yl)-4-hydroxyphenyl]acrylate d'éthyle.
RMN ¹H (CDCl₃) : 1.25 à 1.29 (m, 12 H), 1.31(t, *J* = 7.1Hz, 3H), 1.70 (s, 4H), 4.26 (q, *J* = 7.1Hz, 2H), 5.07 (s, 2H), 6.33 (d, *J*=16Hz, 1H), 6.81 (s, 1H, OH), 6.99 (s, 1H Ar), 7.01 (d, *J* = 9.8Hz, 1H Ar), 7.23 (s, 1H Ar), 7.26 à 7.32 (m, 5H Ar), 7.42 (d, *J* = 2.1Hz, 1H Ar) 7.49 (dd, *J* = 8.3Hz, *J* = 2.1Hz, 1H Ar), 7.69 (d, *J* = 16Hz, 1H).

### (b) acide 3-[3-(3-benzyloxy-5,5,8,8-tetramethyl-5,6,7,8-tetrahydronaphthalen-2-yl)-4-hydroxyphenyl]-acrylique.

De manière analogue à l'exemple 2(h) à partir de 1,2 g (2,47 mmoles) de l'ester éthylique précédent, on obtient 980 mg (98%) d' acide 3-[3-(3-benzyloxy-5,5,8,8-tetramethyl-5,6,7,8-tetrahydronaphthalen-2-yl)-4-hydroxyphenyl]acrylique de point de fusion 123-4°C.
RMN ¹H (CDCl₃): 1.28 à 1.30 (m,12H),1.70 (s,4H), 5.08 (s, 2H), 6.35 (d, *J*=15.7Hz,1H), 6.38 (s,1H), 7.00 (s, 1H Ar), 7.04 (d, *J* = 7.5Hz, 1H Ar), 7.24 (s, 1H Ar), 7.25 à 7.37 (m, 5H Ar), 7.45 (d, *J* = 2Hz, 1 H Ar) 7.52 (dd, *J* = 8.4Hz, *J* = 2Hz,1 H Ar), 7.80 (d, *J* = 15.9Hz, 1H).
RMN ¹³C (CDCl₃) : 31.88, 32.02, 34.00, 34.65, 34.99, 35.08, 72.30, 112.66, 114.70, 118.16, 124.49, 127.07, 127.23, 127.64, 128.30, 128.68, 129.22, 130.55, 132.19, 136.06, 139.95, 147.01, 147.08, 152.48, 156.45, 172.75 .

### EXEMPLE 7

### Acide 3-[3-(3-propyloxy-5,5,8,8-tetramethyl-5,6,7,8-tetrahydronaphthalen-2-yl)-4-methoxymethoxyphenyl]acrylique.

### (a) 3-[3-(3-propyloxy-5,5,8,8-tetramethyl-5,6,7,8-tetrahydronaphthalen-2-yl)-4-methoxymethoxyphenyl]acrylate d'éthyle.

De manière analogue à l'exemple 1(c) par réaction de 3 g (10,4 mmoles) d'acide (3-propyloxy-5,5,8,8-tetramethyl-5,6,7,8-tetrahydronaphthalen-2-yl)boronique (préparation décrite dans WO 97/33881) avec 3,2 g (10,1 mmoles) de 3-(3-bromo-4-méthoxyméthoxyphényl)acrylate d'éthyle (préparé à l'exemple 2(c) ), on obtient après chromatographie sur colonne de silice et élution avec un mélange d'heptane et d'acétate d'éthyle (98-2) 2,1g (43%) de l'ester éthylique attendu.

### (b) acide 3-[3-(3-propyloxy-5,5,8,8-tetramethyl-5,6,7,8-tetrahydronaphthalen-2-yl)-4-methoxymethoxyphenyl]acrylique.

De manière analogue à l'exemple 2(h) à partir de 724 mg (1,5 mmoles) de 3-[3-(3-propyloxy-5,5,8,8-tetramethyl-5,6,7,8-tetrahydronaphthalen-2-yl)-4-methoxy methoxyphenyl]acrylate d'ethyle, on obtient 620 mg (91%) d'acide 3-[3-(3-propyloxy-5,5,8,8-tetramethyl-5,6,7,8-tetrahydronaphthalen-2-yl)-4-methoxymethoxyphenyl] acrylique de point de fusion 170-1°C.

### EXEMPLE 8

### Acide 3-[3-(3-pentyloxy-5,5,8,8-tetramethyl-5,6,7,8-tetrahydronaphthalen-2-yl)-4-methoxymethoxyphenyl]acrylique.

### (a) 2-bromo-3-pentyloxy-5,5,8,8-tetramethyl-5,6,7,8-tetrahydronaphthalene.

De maniére analogue à l'exemple 1(a) par réaction de 5 g (25 mmoles) de 3-bromo-5,5,8,8-tetramethyl-5,6,7,8-tetrahydro-2-naphthol avec 3,9 ml (29,5 mmoles) de 5-iodopentane, on obtient 7 g (79%) du produit attendu sous forme d'une huile incolore.

### (b) acide (3-pentyloxy-5,5,8,8-tetramethyl-5,6,7,8-tetrahydronaphthalen-2-yl)boronique.

De manière analogue à l'exemple 1(b) à partir de 6,9 g (19,5 mmoles) de 2-bromo-3-pentyloxy-5,5,8,8-tetramethyl-5,6,7,8-tetrahydronaphthalene, on obtient 4,68 g (75%) du dérivé boronique attendu .

### (c) 3-[3-(3-pentyloxy-5,5,8,8-tetramethyl-5,6,7,8-tetrahydronaphthalen-2-yl)-4-methoxymethoxyphenyl]acrylate d'éthyle.

De manière analogue à l'exemple 1(c) par réaction de 4 g (12,6 mmoles) d'acide (3-pentyloxy-5,5,8,8-tetramethyl-5,6,7,8-tetrahydronaphthalen-2-yl)boronique avec 2,64 g (8,4 mmoles) de 3-(3-bromo-4-méthoxyméthoxyphényl)acrylate d'éthyle (préparé à l'exemple 2(c) ), on obtient après chromatographie sur colonne de silice élué avec un mélange d'heptane et d'acétate d'éthyle (98-2) 3,2 g (75%) de l'ester éthylique attendu.

### (d) acide 3-[3-(3-pentyloxy-5,5,8,8-tetramethyl-5,6,7,8-tetrahydronaphthalen-2-yl)-4-methoxymethoxyphenyl]acrylique.

De manière analogue à l'exemple 2(h) à partir de 1,7 g (3,26 mmoles) de 3-[3-(3-pentyloxy-5,5,8,8-tetramethyl-5,6,7,8-tetrahydronaphthalen-2-yl)-4-methoxy methoxyphenyl]acrylate d'éthyle, on obtient 1,35 g (86%) d' acide 3-[3-(3-pentyloxy-5,5,8,8-tetramethyl-5,6,7,8-tetrahydronaphthalen-2-yl)-4-methoxymethoxyphenyl] acrylique de point de fusion 178-80°C.

### EXEMPLE 9

### Acide 3-(5'-adamantan-1-yl-4'-methoxy-2'-methyl-biphenyl-3-yl)acrylique

### (a) 4-bromo-3-methylphénol.

Dans un tricol de 2 litres, sous atmosphère d'azote, on introduit 80,0 g (740 mmoles) de 3-méthylphénol, 400 ml d'acide acétique glacial. On refroidit à 15°C, ajoute goutte à goutte 38 ml (742 mmoles) de brome et agite à 15°C pendant trois heures. On verse le milieu réactionnel sur 1litre d'eau, extrait par de l'éther éthylique, décante la phase organique, lave à l'eau jusqu'à pH neutre, sèche sur sulfate de magnésium et évapore les solvants. Le résidu obtenu est trituré dans l'heptane, filtré, séché. On recueille 70 g (50%) d'une poudre blanche de point de fusion 55-56°C.

### (b) 2-(1-adamantyl)-4-bromo-3-methylphénol.

Dans un ballon de 2000 ml, sous atmosphère d'azote, on introduit 70,0 g (376 mmoles) de 4-bromo-3-methylphénol, 63 g (414 mmoles) de 1-adamantanol, 500 ml d'heptane, 50ml de dichlorométhane. On ajoute goutte à goutte 20 ml (376 mmoles) d'acide sulfurique à 98% et agite à température ambiante pendant trois heures. On verse le milieu réactionnel dans l'eau, neutralise par une solution d'hydrogéno carbonate de sodium jusqu'à pH=8-9, extrait par de l'acétate d'éthyle, décante la phase organique, lave à l'eau jusqu'à pH neutre, sèche sur sulfate de magnésium et évapore les solvants. Le résidu obtenu est trituré dans l'heptane, filtré, séché. On recueille 59 g (49%) d'une poudre blanche rosâtre de point de fusion 139-141°C

### (c) 2-(1-adamantyl)-4-bromo-5-methylanisole.

Dans un tricol de 250 ml et sous courant d'azote, on introduit 8 g (24.9 mmoles) de 2-(1-adamantyl)-4-bromo-3-methylphénol, 75 ml de DMF, 25 ml de THF. On ajoute par petites quantités 880 mg (27.4 mmoles) d'hydrure de sodium (75% dans l'huile) et agite jusqu'à cessation du dégagement gazeux. On ajoute ensuite 1,9 ml (30 mmoles) de iodométhane et agite à température ambiante pendant deux heures. On verse le milieu réactionnel dans l'eau glacée, extrait avec de l'éther éthylique, décante la phase organique, sèche sur sulfate de magnésium, évapore. Le résidu obtenu est trituré dans l'heptane, filtré et séché. On recueille 6.44 g (77%) de poudre beige clair de point de fusion 129-131°C.

### (d) acide 5-(1-adamantyl)-4-methoxy-2-méthylphenylboronique.

Dans un tricol de 250 ml et sous courant d'azote, on introduit 6 g (17.9 mmoles) de. 2-(1-adamantyl)-4-bromo-5-methylanisole et 40 ml de THF. A -78°C, on ajoute goutte à goutte 7.9 ml (19.7 mmoles) de n-butyllithium (2,5 M dans l'hexane) et agite 15', à cette même température on ajoute 5 ml (21.5 mmoles) de triisopropylborate et agite pendant 30 minutes. A -50°C on ajoute 20 ml d'acide chlorhydrique (1N) et laisse remonter à température ambiante. On extrait le milieu réactionnel avec de l'éther éthylique, décante la phase organique, sèche sur sulfate de magnésium, évapore. On triture le solide obtenu dans l'heptane, filtre et sèche. On recueille 4.75 g (88%) d'acide boronique attendu de point de fusion 276-278°C qui sera utilisé tel quel pour la suite de la synthèse.

### (e) acide 3-(5'-adamantan-1-yl-4'-methoxy-2'-methylbiphenyl-3-yl)acrylique.

Dans un tricol de 100 ml et sous courant d'azote, on introduit 1.0 g (3.33 mmoles) d'acide 5-(1-adamantyl)-4-methoxy-2-méthylphenylboronique obtenu précédemment, 580 mg (2.56 mmoles) d'acide 3-bromocinnamique et 30 ml de DME. On ajoute goutte à goutte 4.5 ml d'une solution aqueuse de carbonate de potassium (2M) et dégaze le milieu réactionnel. On ajoute ensuite 150 mg (0,13 mmole) de tétrakis triphénylphosphinepalladium(0) et chauffe à reflux pendant 2 heures. On ajoute au milieu réactionnel de l'eau et de l'acétate d'éthyle, acidifie à pH 1 avec de l'acide chlorhydrique (1N). On décante la phase organique, lave à l'eau, sèche sur sulfate de magnésium, évapore. Le résidu obtenu est purifié par chromatographie sur colonne de silice élué avec un mélange d'heptane et d'acétate d'éthyle (85-15). On recueille après évaporation des solvants 750 mg d'un gel épais que l'on laisse cristalliser sous courant d'azote. On recristallise les cristaux jaunes ainsi obtenus dans l'éthanol, filtre, sèche pour obtenir 452 mg (44%) d'acide 3-(5'-adamantan-1-yl-4'-methoxy-2'-methylbiphenyl-3-yl)acrylique de point de fusion 194-6°C.
RMN ¹H (CDCl₃): 1.76 (br,6 H), 2.05 (br,3 H), 2.10 (br, 6H), 2.26 (s, 3H), 3.88 (s, 3H), 6.48 (d, *J*=15.8Hz,1 H), 6.78 (s, 1H Ar), 7.07 (s, 1H Ar), 7.35 à 7.51 (m, 4H Ar), 7.84 (d, *J* = 15.8Hz, 1H Ar).

### EXEMPLE 10

### Acide 3-(5'-adamantan-1-yl-6-hydroxy-4'-methoxy-2'-methyl-biphenyl-3-yl)-acrylique

### (a) 3-(3-bromo-4-hyroxyphényl)acrylate d'éthyle.

De manière analogue à l'exemple 2(g) par réaction de 10.1 g (20.3 mmoles) de 3-(3-bromo-4-méthoxyméthoxyphényl)acrylate d'éthyle obtenu à l'exemple 2(c) avec 5.4 ml (102 mmoles) d'acide sulfurique 98%, on obtient 3.9 g de 3-(3-bromo-4-hydroxyphényl)acrylate d'éthyle (49%) sous forme d'une poudre blanche de point de fusion 112-3°C.

### (b) 3-(5'-adamantan-1-yl-6-hydroxy-4'-methoxy-2'-methyl-biphenyl-3-yl)acrylate d'éthyle.

De manière analogue à l'exemple 1(c) par réaction de 3.0 g (10 mmoles) d'acide 5-(1-adamantyl)-4-methoxy-2-méthylphenylboronique obtenu à l'exemple 10(d) avec 2.25 g (8.33 mmoles) de 3-(3-bromo-4-hydroxyphényl)acrylate d'éthyle obtenu précédemment, on obtient 570 mg (15%) d'une poudre blanche de point de fusion 164-6°C.

### (c) acide 3-(5'-adamantan-1-yl-6-hydroxy-4'-methoxy-2'-methylbiphenyl-3-yl) acrylique.

De manière analogue à l'exemple 2(h) à partir de 270 mg (0.6 mmol) 3-(5'-adamantan-1-yl-6-hydroxy-4'-methoxy-2'-methylbiphenyl-3-yl)acrylate d'éthyle obtenu précédemment, on obtient 190 mg (76%). d'une poudre blanche se décomposant à partir de 120°C.
RMN ¹H (CDCl₃):1.76 (br,6H), 2.07 (br,9H), 2.13 (s,3H), 3.89 (s,3H), 5.20 (br,1H), 6.31 (d,*J*=15.75Hz,1H), 6.83 (s,1H), 7.00 (d,*J*=9.25Hz,1H), 7.48 (dd,*J1*=9.25Hz,*J2*=2.0Hz,1H), 7.74 (d,*J*=15.75Hz,1H).

### EXEMPLE 11

### Acide 3-(5'-adamantan-1-yl-4'-methoxy-6-methoxymethoxy-2'-methylbiphenyl-3-yl)acrylique

### (a) 3-(5'-adamantan-1-yl-4'-methoxy-6-methoxymethoxy-2'-methylbiphenyl-3-yl) acrylate d'éthyle.

De manière analogue à l'exemple 2(b) par réaction de 300 mg (0.67 mmole) de 3-(5'-adamantan-1-yl-6-hydroxy-4'-methoxy-2'-methylbiphenyl-3-yl)acrylate d'éthyle obtenu à l'exemple 11(b), avec 64 mg (0.8 mmole) de chlorure de méthoxyméthyle, on obtient 320 mg (97%) d'un gel blanc.
RMN ¹H (CDCl₃): 1.32 (t,*J*=7.00Hz,3H), 1.75(br,6H), 2.04+2.09 (br+br,6H+3H), 2.14 (s,3H), 3.37 (s,3H), 3.87 (s,3H), 4.25 (q,*J*=7.0Hz,2H), 5.11 (s,2H), 6.33 (d,*J*=15.8Hz,1H), 6.76 (s,1H), 7.00 (s,1H), 7.20 (d,*J*=8.5Hz,1H), 7.36 (d,*J*=2.25Hz,1H), 7.46 (dd,*J1*=8.5Hz,*J2*=2.25Hz,1H), 7.66 (d,*J*=15.8Hz,1H).

### (b) acide 3-(5'-adamantan-1-yl-4'-methoxy-6-methoxymethoxy-2'-methyl-biphenyl-yl) acrylique.

De manière analogue à l'exemple 2(h) à partir de 300 mg (0.61 mmole) de 3-(5'-adamantan-1-yl-4'-methoxy-6-methoxymethoxy-2'-methyl-biphenyl-3-yl)acrylate d'éthyle obtenu précédemment, on obtient 200 mg (71%). d'une poudre blanche se décomposant a partir de 113°C.
RMN ¹H (CDCl₃): 1.76 (br,6H), 2.04 (br,3H), 2.09 (br,6H), 2.14 (s,3H), 3.38 (s,3H), 3.87 (s,3H), 5.13 (s,2H), 6.34 (d,*J*=15.75Hz,1H), 6.76 (s,1H), 7.00 (s,1H), 7.22 (d,*J*=8.5Hz,1H), 7.39 (d,*J*=2.25Hz,1H), 7.49 (dd,*J1*=8.5Hz,*J2*=2.25Hz,1H), 7.76 (d,*J*=15.75Hz,1H).

### EXEMPLE 12

### Acide 3-{4-methoxy-3-[3-(3-methoxybenzyloxy)-5,5,8,8-tetramethyl-5,6,7,8-tetrahydro-naphthalen-2-yl]phenyl}acrylique.

### (a) 2-bromo-3-méthoxyméthoxy-5,5,8,8-tetramethyl-5,6,7,8-tetrahydronaphthalene.

De manière analogue à l'exemple 1(a) par réaction de 100 g (353 mmoles) de 3-bromo-5,5,8,8-tetramethyl-5,6,7,8-tetrahydro-2-naphthol avec 32 ml (406 mmoles) de chlorure de méthoxyméthyle on obtient 100 g (86%) de produit attendu sous forme d'une poudre beige de point de fusion 69-71°C.

### b) acide 3-méthoxyméthoxy -5,5,8,8-tetramethyl-5,6,7,8-tetrahydro-2-naphthyl boronique.

De manière analogue à l'exemple 1(b) par réaction de 90 g (275 mmoles) de 2-bromo-3-méthoxyméthoxy-5,5,8,8-tetramethyl-5,6,7,8-tetrahydronaphthalene obtenu précédemment, on obtient 70 g (87%) de produit attendu sous forme d'une poudre blanche de point de fusion 130-132°C.

### c) 3-bromo-4-methoxybenzaldehyde.

De manière analogue à l'exemple 1(a) par réaction de 50 g (270 mmoles) de 3-bromo-4-hydroxybenzaldehyde (obtenu suivant l'exemple 2(a)), et de 20.2 ml (338 mmoles) de iodométhane, on obtient 48g (83%) de produit attendu sous forme d'une poudre blanche de point de fusion 48-50°C.

### d) 3-(3-bromo-4-methoxyphenyl)acrylate d'éthyle.

De manière analogue à l'exemple 2(c) par réaction de 46 g (214 mmoles) de 3-bromo-4-methoxybenzaldehyde obtenu précédemment, et de 55.2 ml (278 mmoles) de triéthylphosphonoacétate, on obtient 47 g (77%) de produit attendu sous forme de paillettes blanches de point de fusion 76-78°C.

### e) 3-[4-methoxy-3-(3-methoxymethoxy-5,5,8,8-tetramethyl-5,6,7,8-tetrahydronaphthalen-2-yl)phenyl]acrylate d'éthyle.

De manière analogue à l'exemple 1(c) par réaction de 53.3 g (182 mmoles) d'acide 3-méthoxyméthoxy-5,5,8,8-tetramethyl-5,6,7,8-tetrahydro-2-naphthyl boronique obtenu à l'exemple 13(b) et de 40 g (140 mmoles) de 3-(3-bromo-4-methoxyphenyl)acrylate d'éthyle obtenu précédemment, on obtient 63 g (99%) de produit attendu sous forme d'une huile jaune et épaisse .
RMN ¹H (CDCl₃): 1.23 à 1,35 (m,15H), 1.70 (s,4H), 3.34 (s3H), 3.81 (s,3H), 4.25 (q,*J*=7.25Hz,2H), 5.03 (s,2H), 6.32 (d,*J*=15.75Hz,1H), 9.95 (d,*J*=9.0Hz,1H), 7.13 (s,1H), 7.42 à 7.66 (m,2H), 7.67 (d,*J*=15.75Hz,1H).

### f) 3-[3-(3-hydroxy-5,5,8,8-tetramethyl-5,6,7,8-tetrahydro-naphthalen-2-yl)-4-methoxyphenyl]acrylate d'éthyle.

De manière analogue à l'exemple 2(g) par réaction de 63 g (140 mmoles) de 3-[4-methoxy-3-(3-methoxymethoxy-5,5,8,8-tetramethyl-5,6,7,8-tetrahydronaphthalen-2-yl)phenyl]acrylate d'éthyle obtenu précédemment, et de 22 ml (420 mmoles) d'acide sulfurique on obtient 42.5 g (75%) de produit attendu sous forme d'une poudre jaune clair de point de fusion 147-149°C.

### (g) 3-{4-methoxy-3-[3-(3-methoxybenzyloxy)-5,5,8,8-tetramethyl-5,6,7,8-tetrahydronaphthalen-2-yl]phenyl}acrylate d'éthyle.

De manière analogue à l'exemple 1(a) par réaction de 1 g (2.45 mmoles) de 3-[3-(3-hydroxy-5,5,8,8-tetramethyl-5,6,7,8-tetrahydronaphthalen-2-yl)-4-methoxy phenyl]acrylate d'éthyle obtenu précédemment avec 4 ml (3 mmoles) de chlorure de 3-methoxybenzyle, on obtient 1.30 g (100%) de produit attendu sous forme d'une huile jaune.
RMN ¹H (CDCl₃): 1.24 à 1.35 (m,15H), 1.69 (s,4H), 3.69 (s,3H), 3.76 (s,3H), 4.25 (q,*J*=7.0Hz,2H), 5.0 (s,2H), 6.30 (d,*J*=16.0Hz,1H), 6.75 à 6.96 (m,5H), 7.16 à 7.22 (m,2H), 7.45 à 7.51 (m,2H), 7.67 (d,*J*=16.0Hz,1H).

### (h) acide 3-{4-methoxy-3-[3-(3-methoxybenzyloxy)-5,5,8,8-tetramethyl-5,6,7,8-tetrahydro-naphthalen-2-yl]phenyl}acrylique.

De manière analogue à l'exemple 2(h) à partir de 1.3 g (2.45 mmoles) de 3-{4-methoxy-3-[3-(3-methoxybenzyloxy)-5,5,8,8-tetramethyl-5,6,7,8-tetrahydronaphthalen-2-yl]phenyl}acrylate d'éthyle obtenu précédemment, on obtient 830 mg (67%) de produit attendu sous forme d'une poudre blanche de point de fusion 168-170°C.

### EXEMPLE 13

### Acide 3-{4-methoxy-3-[3-(4-methoxybenzyloxy)-5,5,8,8-tetramethyl-5,6,7,8-tetrahydronaphthalen-2-yl]phenyl}acrylique.

### (a) 3-{4-methoxy-3-[3-(4-methoxybenzyloxy)-5,5,8,8-tetramethyl-5,6,7,8-tetrahydronaphthalen-2-yl]phenyl}acrylate d'éthyle.

De manière analogue à l'exemple 1(a) par réaction de 1.23 g (3 mmoles) de 3-[3-(3-hydroxy-5,5,8,8-tetramethyl-5,6,7,8-tetrahydro-naphthalen-2-yl)-4-methoxyphenyl]acrylate d'éthyle obtenu à l'exemple 13(f) avec 490 µl (3.6 mmoles) de chlorure de 4-methoxybenzyle, on obtient 1.58 g (100%) de produit attendu sous forme d'une huile jaune.
RMN ¹H (CDCl₃): 1.21 à 1.35 (m,15H), 1.69 (s,4H), 3.75 (s,3H), 3.77 (s,3H), 4.25 (q,*J*=7.0Hz,2H), 4.93 (s,2H), 6.29 (d,*J*=16.0Hz,1H), 6.80 à 6.94 (m,4H), 7.15 à 7.18 (m,2H), 7.31 (d,*J*=8.5Hz,1H), 7.44 à 7.50 (m,2H), 7.66 (d,*J*=16.25Hz,1H).

### (b) acide 3-{4-methoxy-3-[3-(4-methoxybenzyloxy)-5,5,8,8-tetramethyl-5,6,7,8-tetrahydro-naphthalen-2-yl]phenyl}acrylique

De manière analogue à l'exemple 2(h) à partir de 1.58 g (3 mmoles) de 3-{4-methoxy-3-[3-(4-methoxybenzyloxy)-5,5,8,8-tetramethyl-5,6,7,8-tetrahydronaphthalen-2-yl]phenyl}acrylate d'éthyle obtenu précédemment, on obtient 1.1 g (71%) de produit attendu sous forme d'une poudre blanche de point de fusion 201-203°C.

### EXEMPLE 14

### Acide 3-{3-[3-(6-hydroxyhexyloxy)-5,5,8,8-tetramethyl-5,6,7,8-tetrahydronaphthalen-2-yl]-4-methoxyphenyl}acrylique.

### (a) 3-{3-[3-(6-hydroxyhexyloxy)-5,5,8,8-tetramethyl-5,6,7,8-tetrahydronaphthalen-2-yl]-4-methoxyphenyl}acrylate d'éthyle.

De manière analogue à l'exemple 1(a) par réaction de 1.4 g (3.4 mmoles) de 3-[3-(3-hydroxy-5,5,8,8-tetramethyl-5,6,7,8-tetrahydronaphthalen-2-yl)-4-methoxy phenyl]acrylate d'éthyle obtenu à l'exemple 13(f) avec 540 µl (4.1 mmoles) de 1-bromohexanol, on obtient 850 mg (50%) de produit attendu sous forme d'une huile jaune.
^{RMN1}H (CDCl₃): 1.23 à 1.35 (m,19H), 1.43 à 1.70 (m,8H), 3.56 (q,*J*=5.5Hz,2H), 3.81 (s,3H), 3.90 (t,*J*=5.0Hz,2H), 4.25 (q,*J*=7.25Hz,2H), 6.31 (d,*J*=16.0Hz,1H), 6.85 (s,1H), 6.96 (d,*J*=8.5Hz,1H), 7.20 (s,1H), 7.47 (dd,*J1*=8.25Hz,*J2*=2.25Hz,1H), 7.52 (d,*J*=2.25Hz,1H), 7.67 (d,*J*=16.0Hz,1H).

### (b) acide 3-{3-[3-(6-hydroxyhexyloxy)-5,5,8,8-tetramethyl-5,6,7,8-tetrahydro naphthalen-2-yl]-4-methoxyphenyl}acrylique.

De manière analogue à l'exemple 2(h) à partir de 850 mg (1.67 mmoles) de 3-{3-[3-(6-hydroxyhexyloxy)-5,5,8,8-tetramethyl-5,6,7,8-tetrahydronaphthalen-2-yl]-4-methoxyphenyl}acrylate d'éthyle obtenu précédemment, on obtient 510 mg (63%) de produit attendu sous forme d'une poudre blanche de point de fusion 118-120°C.

### EXEMPLE 15

### Acide 3-{3-[3-(7-hydroxy-heptyloxy)-5,5,8,8-tetramethyl-5,6,7,8-tetrahydro naphthalen-2-yl]-4-methoxyphenyl}acrylique

### (a) 3-{3-[3-(7-hydroxyheptyloxy)-5,5,8,8-tetramethyl-5,6,7,8-tetrahydro naphthalen-2-yl]-4-methoxyphenyl}acrylate d'éthyle.

Dans un ballon on introduit 1.23 g (3 mmoles) de 3-[3-(3-hydroxy-5,5,8,8-tetramethyl-5,6,7,8-tetrahydronaphthalen-2-yl)-4-methoxyphenyl]acrylate d'éthyle obtenu à l'exemple 13(f), 440 mg (3.2 mmoles) de carbonate de potassium, 540 µl (3.5 mmoles) de 1-bromoheptanol et 30 ml de butanone. On chauffe au reflux pendant 8 heures, verse le milieu réactionnel dans l'eau, acidifie à pH=1 avec de l'acide chlorhydrique, extrait par de l'éther éthylique, décante la phase organique, sèche sur sulfate de magnésium, évapore. On recueille 1.57 g (100%) de produit attendu sous forme d'une huile jaune.
RMN ¹H (CDCl₃): 1.26 (s,6H), 1.32 (s,6H), 1.08 à 1.7 (m,17H), 3.5 à 3.7 (m,2H), 3.81 (s,3H), 3.89 (t,*J*=6.25Hz,2H), 4.24 (q,*J*=7.0Hz,2H), 6.3 (d,*J*=16.0Hz,1H), 6.84 (s,1H), 6.95 (d,*J*=8.25Hz,1H), 7.19 (s,1H), 7.47 (dd,*J1*=8.25Hz,*J2*=2.25Hz,1H), 7.50 (d,*J*=2.25Hz,1H), 7.67 (d,*J*=16.0Hz,1H).

### (b) acide 3-{3-[3-(7-hydroxyheptyloxy)-5,5,8,8-tetramethyl-5,6,7,8-tetrahydronaphthalen-2-yl]-4-methoxyphenyl}acrylique.

De manière analogue à l'exemple 2(h) à partir de 1.57 g (3 mmoles) de 3-{3-[3-(7-hydroxyheptyloxy)-5,5,8,8-tetramethyl-5,6,7,8-tetrahydronaphthalen-2-yl]-4-methoxyphenyl}acrylate d'éthyle obtenu précédemment, on obtient 700 mg (47%) de produit attendu sous forme d'une poudre blanche de point de fusion 105-107°C.

### EXEMPLE 16

### Acide 3-{3-[3-(5-hydroxypentyloxy)-5,5,8,8-tetramethyl-5,6,7,8-tetrahydronaphthalen-2-yl]-4-methoxyphenyl}acrylique.

### (a) 3-{3-[3-(5-acetoxypentyloxy)-5,5,8,8-tetramethyl-5,6,7,8-tetrahydro-naphthalen-2-yl]-4-methoxyphenyl}acrylate d'éthyle.

De manière analogue à l'exemple 1(a) par réaction de 1.20 g (2.90 mmoles) de 3-[3-(3-hydroxy-5,5,8,8-tetramethyl-5,6,7,8-tetrahydronaphthalen-2-yl)-4-methoxy phenyl]acrylate d'éthyle obtenu à l'exemple 13(f), avec 727 mg (3.48 mmoles) d'acétate de 5-bromopentyle on obtient 1.42 g (92%) de produit attendu sous forme d'une huile jaune.
RMN ¹H (CDCl₃): 1.26 à 1.37 (m,17H), 1.50 à 1.67 (m,4H), 1.70 (s,4H), 2.01 (s,3H), 3.80 (s,3H), 3.80 à 4.00 (m,4H), 4.25 (q,*J*=7.25Hz,2H), 6.30 (d,*J*=15.75Hz,1H), 6.84 (s,1H), 6.94 (d,*J*=8.25Hz,1H), 7.17 (s,1H), 7.44 à 7.48 (m,2H), 7.66 (d,*J*=16.0Hz,1H).

### (b) acide 3-{3-[3-(5-Hydroxypentyloxy)-5,5,8,8-tetramethyl-5,6,7,8-tetrahydronaphthalen-2-yl]-4-methoxyphenyl}acrylique.

De manière analogue à l'exemple 2(h) à partir de 1.42 g (2.65 mmoles) de 3-{3-[3-(5-acetoxypentyloxy)-5,5,8,8-tetramethyl-5,6,7,8-tetrahydronaphthalen-2-yl]-4-methoxyphenyl}acrylate d'éthyle obtenu précédemment, on obtient 730 mg (60%) de produit attendu sous forme d'une poudre blanche de point de fusion 115-117°C.

### EXEMPLE 17

### Acide 3-{3-[3-(3-hydroxypropyloxy)-5,5,8,8-tetramethyl-5,6,7,8-tetrahydronaphthalen-2-yl]-4-methoxyphenyl}acrylique.

### (a) 3-{3-[3-(3-hydroxy-propyloxy)-5,5,8,8-tetramethyl-5,6,7,8-tetrahydronaphthalen-2-yl]-4-methoxyphenyl}acrylate d'éthyle.

De manière analogue à l'exemple 16(a) par réaction de 1.2 g (2.9 mmoles) de 3-[3-(3-hydroxy-5,5,8,8-tetramethyl-5,6,7,8-tetrahydro-naphthalen-2-yl)-4-methoxy phenyl]acrylate d'éthyle obtenu à l'exemple 13(f), avec 490 mg (3.5mmoles) de 1-bromopropanol on obtient 632 mg (47%) de produit attendu sous forme d'une huile jaune.
RMN ¹H (CDCl₃): 1.27 à 1.35 (m,9H), 1.70 (s,4H), 1,85 à 1.92 (m,2H), 1.96 (br,1H), 3.64 (m,2H), 3.82 (s,3H), 4.09 (t,*J*= 5.5Hz,2H), 4.25 (q,*J*=7.25Hz,2H), 6.31 (d,*J*=16.0Hz,1H), 6.87 (s,1H), 6.96 (d,*J*=8.5Hz,1H), 7.15 (s,1H), 7.44 (d,*J*=2.25Hz,1H), 7.49 (dd,*J1*=8.5Hz,*J2*=2.25Hz,1H), 7.66 (d,*J*=16.0Hz,1H).

### (b) acide 3-{3-[3-(3-hydroxypropyloxy)-5,5,8,8-tetramethyl-5,6,7,8-tetrahydronaphthalen-2-yl]-4-methoxyphenyl}acrylique.

De manière analogue à l'exemple 2(h) à partir de 632 mg (1.35 mmoles) de 3-{3-[3-(3-hydroxypropyloxy)-5,5,8,8-tetramethyl-5,6,7,8-tetrahydronaphthalen-2-yl]-4-methoxyphenyl}acrylate d'éthyle obtenu précédemment, on obtient 449 mg (75%) de produit attendu sous forme d'une poudre blanche de point de fusion 198-200°C.

### EXEMPLE 18

### Acide 3-[3-(1-benzyloxy-5,5,8,8-tetramethyl-5,6,7,8-tetrahydro-naphthalen-2-yl)phenyl]acrylque.

### (a) 1-methoxymethoxy-5,5,8,8-tetramethyl-5,6,7,8-tetrahydronaphthalene.

De manière analogue à l'exemple 1(a) par réaction de 8,65 g (42,4 mmoles) de 5,5,8,8-tetramethyl-5,6,7,8-tetrahydro-2-naphthol avec 3,55 ml (46,6 mmoles) de chlorure de méthoxyméthyle, on obtient 10,5 g (100%) du produit attendu.

### (b) acide 1-methoxymethoxy-5,5,8,8-tetramethyl-5,6,7,8-tetrahydro-2-naphthalene boronique.

Dans un ballon, on introduit 1 g (4 mmoles) de 1-methoxymethoxy-5,5,8,8-tetramethyl-5,6,7,8-tetrahydronaphthalene et 12 ml de THF. On refroidit à -78°C et ajoute goutte à goutte 630 µl (4,2 mmoles) de TMEDA puis 3,4 ml (4,4 mmoles) de sec-BuLi (1,3 M). On agite à -78°C pendant 1 heure puis ajoute 1,4 ml (6 mmoles) de triisopropylborate et laisse remonter à température ambiante. A -40°C, on additionne 3 ml d'acide chlorhydrique (5%) et agite à température ambiante 1 heure. On verse le milieu réactionnel dans l'eau, extrait avec de l'acétate d'éthyle, décante la phase organique, sèche sur sulfate de magnésium, évapore. Le résidu obtenu est purifié par chromatographie sur colonne de silice élué avec un mélange de dichlorométhane et de méthanol (98-2). On recueille 900 mg (77%) du dérivé boronique attendu sous forme d'une huile légèrement jaune.

### (c) acide 3-[3-(1-methoxymethoxy-5,5,8,8-tetramethyl-5,6,7,8-tetrahydro-naphthalen-2-yl)phenyl]acrylique.

De manière analogue à l'exemple 1 (c) par réaction de 3,4 g (11,6 mmoles) d'acide 3-(1-methoxymethoxy-5,5,8,8-tetramethyl-5,6,7,8-tetrahydro-2-naphthalene boronique avec 1,32 g (5,8 mmoles) d'acide 3-bromocinnamique, on obtient après trituration dans l'heptane 1,4 g (62%) d' acide 3-[3-(1-methoxymethoxy-5,5,8,8-tetramethyl-5,6,7,8-tetrahydro-naphthalen-2-yl)
phenyl]acrylique sous forme d'une poudre blanche et de point de fusion 188-9°C.

### (d) 3-[3-(1-hydroxy-5,5,8,8-tetramethyl-5,6,7,8-tetrahydro-naphthalen-2-yl)phenyl] acrylate de méthyle.

Dans un ballon, on introduit 1,24 g (3,15 mmoles) d'acide 3-[3-(1-méthoxyméthoxy-5,5,8,8-tetramethyl-5,6,7,8-tetrahydro-naphthalen-2-yl)phenyl] acrylique obtenu au ( c) , 15 ml de méthanol et 15 ml de THF. On ajoute 620 µL d'acide sulfurique concentré, agite à température ambiante pendant huit heures et chauffe à reflux pendant deux heures. On verse le milieu réactionnel dans l'eau, extrait avec de l'éther éthylique, décante la phase organique, sèche sur sulfate de magnésium, évapore. On recueille 1,1 g (100%) de 3-[3-(1-hydroxy-5,5,8,8-tetramethyl-5,6,7,8-tetrahydro-naphthalen-2-yl)phenyl] acrylate de méthyle de point de fusion 197-8°C.

### (e) 3-[3-(1-benzyloxy-5,5,8,8-tetramethyl-5,6,7,8-tetrahydro-naphthalen-2-yl)phenyl] acrylate de méthyle.

De manière analogue à l'exemple 1(a) par réaction de 500 mg (1,37 mmoles) de 3-[3-(1-hydroxy-5,5,8,8-tetramethyl-5,6,7,8-tetrahydronaphthalen-2-yl)phenyl] acrylate de méthyle avec 180 µl (1,5 mmoles) de bromure de benzyle, on obtient 550 mg (88%) de produit attendu sous forme d'une huile jaune.

### (f) acide 3-[3-(1-benzyloxy-5,5,8,8-tetramethyl-5,6,7,8-tetrahydronaphthalen-2-yl)phenyl]acrylique.

De manière analogue à l'exemple 2(h) à partir de 498 mg (1,01 mmoles) de 3-[3-(1-benzyloxy-5,5,8,8-tetramethyl-5,6,7,8-tetrahydronaphthalen-2-yl)phenyl] acrylate d'éthyle, on obtient 360 mg (82%) d' acide 3-[3-(1-Benzyloxy-5,5,8,8-tetramethyl-5,6,7,8-tetrahydronaphthalen-2-yl)phenyl]acrylique de point de fusion 153-4°C.

### EXEMPLE 19

### Acide 3-{3-[3-(5-tert-butoxycarbonylpentyloxy)-5,5,8,8-tetramethyl-5,6,7,8-tetrahydronaphthalen-2-yl]-4-methoxyphenyl}acrylique

### (a) 6-bromohexanoate de tert-butyle.

Dans un ballon et sous courant azote, on introduit 1.95 g ( 10 mmoles) d'acide 6-bromo hexanoïque dissous dans 10 ml de dichlorométhane. On ajoute goutte à goutte une solution de 4.37 g ( 20 mmoles) de 2,2,2-trichloroacétimidate de *tert*-butyle dans 20 ml de cyclohexane suivi immédiatement par 200 µl d'éthérate de trifluoroborane. On agite à température ambiante 5 minutes, ajoute 5 g d'Hydrogénocarbonate de sodium et agite 1 minute. On dépose la suspension obtenue sur une colone de silice préalablement mouillée par du cyclohexane et élue cette dernière par un mélange cyclohexane/acétate d'éthyle 8/2. Aprés évaporation des sovants on obtient 1.63 g (65%) d'une huile incolore.
RMN ¹H (CDCl₃):1.45 (s,9H), 1.44à1.70 (m,4H), 1.87 (m,2H), 2.23 (t,*J*=7.5Hz,2H), 3.41 (t,*J*=6.75Hz,2H).

### (c) 3-{3-[3-(5-tert-butoxycarbonylpentyloxy)-5,5,8,8-tetramethyl-5,6,7,8-tetrahydronaphthalen-2-yl]-4-methoxyphenyl}acrylate d'éthyle.

De manière analogue à l'exemple 1(a) par réaction de 2.36 g (5.8 mmoles) de 3-[3-(3-hydroxy-5,5,8,8-tetramethyl-5,6,7,8-tetrahydro-naphthalen-2-yl)-4-methoxy phenyl]acrylate d'éthyle obtenu à l'exemple 13(f) avec 1.6 g (6.3 mmoles) de 6-Bromohexanoate de *tert*-butyle obtenu précédemment, on obtient 3.3 g (100%) de produit attendu sous forme d'une huile jaune.
RMN ¹H (CDCl₃):1.21 à 1.75 (m,32H), 2.13 (t,*J*=7.25Hz,2H), 3.80 (s,3H), 3.88 (t,*J*=6.25Hz,2H), 4.25 (q,*J*=7.0Hz,2H), 6.30 (d,*J*=16.0Hz,1H), 6.83 (s,1H), 6.94 (d,*J*=8.75Hz,1H), 7.16 (s,1H), 7.44 à 7.48 (m,2H), 7.66 (d,*J*=16.0Hz,1H).

### (b) acide 3-{3-[3-(5-tert-butoxycarbonylpentyloxy)-5,5,8,8-tetramethyl-5,6,7,8-tetrahydronaphthalen-2-yl]-4-methoxyphenyl}acrylique.

Dans un ballon on place une solution de 1.0 g (1.7 mmoles) de 3-{3-[3-(5-*tert*-butoxycarbonylpentyloxy)-5,5,8,8-tetramethyl-5,6,7,8-tetrahydronaphthalen-2-yl]-4-methoxyphenyl}acrylate d'éthyle obtenu précédemment dissous dans 25 ml de THF et ajoute 730 mg (17 mmoles) d'hydroxyde de lithium et 2 ml d'eau. On chauffe au reflux du THF pendant 8 heures, agite à température ambiante pendant 48 heures, verse le milieu réactionnel dans l'eau, acidifie à pH 1 avec de l'acide chlohydrique, extrait avec de l'éther éthylique, décante la phase organique, sèche sur sulfate de magnésium, filtre, évapore. Le résidu obtenu est chromatographié sur une colone de silice élué par un mélange heptane/Acétate d'éthyle 8/2 . Aprés évaporation des solvants on recueille 126 mg (14%) d'une poudre blanche de point de fusion 130-132°C.

### EXEMPLE 20

### Acide 3-{3-[3-(7-tert-butoxycarbonyl-heptyloxy)-5,5,8,8-tetramethyl-5,6,7,8-tetrahydro naphthalen-2-yl]-4-methoxyphenyl}acrylique.

### (a) 8-bromooctanoate de tert-butyle.

De manière analogue à l'exemple 21(a), à partir de 3 g (13.4 mmoles) d'acide 8-bromooctanoïque, on obtient 3 g (80%) d'une huile jaune clair.
RMN ¹H (CDCl₃): 1.29 à 1.37 (m,6H), 1.44 (s,9H), 1.50 à 1.75 (m,2H), 1.75 à 1.95 (m,2H), 2.20 (t,J=7.5Hz,2H), 3.40 (t,J=6.75Hz,2H).

### (b) 3-{3-[3-(7-tert-butoxycarbonylheptyloxy)-5,5,8,8-tetramethyl-5,6,7,8-tetrahydronaphthalen-2-yl]-4-methoxyphenyl}acrylate d'éthyle.

De manière analogue à l'exemple 1(a) par réaction de 3.82 g (9.34 mmoles) de 3-[3-(3-hydroxy-5,5,8,8-tetramethyl-5,6,7,8-tetrahydro-naphthalen-2-yl)-4-methoxyphenyl]acrylate d'éthyle obtenu à l'exemple 13(f) avec 3 g (10.7 mmoles) de 8-bromooctanoate de *tert*-butyle obtenu précédemment, on obtient 4.36 g (77%) de produit attendu sous forme d'une huile jaune.
RMN ¹H (CDCl₃): 1.26 à 1.13 (m,21H), 1.44 (s,9H), 1.43 à 1.65 (m,4H), 1.70 (s,4H), 2.16 (t,J=7.25Hz,2H), 3.80 (s,3H), 3.88 (t,J=6.5Hz,2H), 4.24 (q,J=7.0Hz,2H), 6.30 (d,J=15.75Hz,1H), 6.84 (s,1H), 6.93 (d,J=8.25Hz,1H), 7.17 (s,1H), 7.43 à 7.49 (m,2H), 7.66 (d,J=15.75Hz,1H).

### (c) acide 3-{3-[3-(7-tert-butoxycarbonylheptyloxy)-5,5,8,8-tetramethyl-5,6,7,8-tetrahydronaphthalen-2-yl]-4-methoxyphenyl}acrylique.

De manière analogue à l'exemple 21(c) à partir de 1.15 g (1.9 mmoles) de 3-{3-[3-(7-*tert*-butoxycarbonylheptyloxy)-5,5,8,8-tetramethyl-5,6,7,8-tetrahydro naphthalen-2-yl]-4-methoxyphenyl}acrylate d'éthyle obtenu précédemment, on obtient 363 mg (33%) du produit attendu sous forme d'une poudre blanche de point de fusion 126-128°C

### EXEMPLE 21

### Acide 3-{3-[3-(7-carboxyheptyloxy)-5,5,8,8-tetramethyl-5,6,7,8-tetrahydronaphthalen-2-yl]-4-methoxyphenyl}acrylique.

### (a) 3-{3-[3-(7-carboxyheptyloxy)-5,5,8,8-tetramethyl-5,6,7,8-tetrahydronaphthalen-2-yl]-4-methoxyphenyl}acrylate d'éthyle.

On place dans un ballon, une solution de 3.75 g (6.18 mmoles) de 3-{3-[3-(7-*tert-*butoxycarbonylheptyloxy)-5,5,8,8-tetramethyl-5,6,7,8-tetrahydronaphthalen-2-yl]-4-methoxyphenyl}acrylate d'éthyle obtenu à l'exemple 22(b) dissous dans 60 ml de dichlorométhane, ajoute 4.76 ml (61.8 mmoles) d'acide trifluoroacétique et agite à température ambiante pendant 4 heures. On verse le milieu réactionnel dans l'eau, extrait avec de l'éther éthylique, décante la phase organique, séche sur sulfate de magnésium, filtre et évapore.
On obtient 3.4 g (100%) de produit attendu sous forme d'une mousse blanche.
RMN ¹H (CDCl₃): 1.26 à 1.35 (m,21H), 1.50 à 1.67 (m,4H), 1.70 (s,4H), 2.30 (t,J=7.5Hz,2H), 3.80 (s,3H), 3.88 (t,J=6.25Hz,2H), 4.25 (q,J=7.25Hz,2H), 6.31 (d,J=15.75Hz,1H), 6.84 (s,1H), 6.94 (d,J=8.25Hz,1H), 7.17 (s,1H), 7.44 à 7.49 (m,2H), 7.67 (d,J=16.0Hz,1H).

### (b) acide 3-{3-[3-(7-carboxyheptyloxy)-5,5,8,8-tetramethyl-5,6,7,8-tetrahydronaphthalen-2-yl]-4-methoxyphenyl}acrylique.

De manière analogue à l'exemple 2(h) à partir de 1 g (1.8 mmoles) de 3-{3-[3-(7-carboxyheptyloxy)-5,5,8,8-tetramethyl-5,6,7,8-tetrahydronaphthalen-2-yl]-4-methoxyphenyl}acrylate d'éthyle obtenu précédemment, on obtient 830 mg (88%) du produit attendu sous forme d'une poudre blanche de point de fusion 212-214°C.

### EXEMPLE 22

### Acide3-{3-[3-(5-carboxypentyloxy)-5,5,8,8-tetramethyl-5,6,7,8-tetrahydronaphthalen-2-yl]-4-methoxyphenyl}acrylique.

### (a) 3-{3-[3-(5-carboxypentyloxy)-5,5,8,8-tetramethyl-5,6,7,8-tetrahydronaphthalen-2-yl]-4-methoxyphenyl}acrylate d'éthyle.

De manière analogue à l'exemple 23(a) à partir de 2.3 g (3.97 mmoles) de 3-{3-[3-(5-*tert*-butoxycarbonylpentyloxy)-5,5,8,8-tetramethyl-5,6,7,8-tetrahydronaphthalen-2-yl]-4-methoxyphenyl}acrylate d'éthyle obtenu à l'exemple 21(b), on obtient 2.1 g (100%) de produit attendu sous forme d'une mousse blanche.
RMN ¹H (CDCl₃) :1.24 à 1.42 (m,17H), 1.55 à 1.70 (m,8H), 2.28 (t,J=7.0Hz,2H), 3.80 (s,3H), 3.91 (t,J=5.75Hz,2H), 4.25 (q,J=7.25Hz,2H), 6.32 (d,J=15.75Hz,1H), 6.84 (s,1H), 6.95 (d,J=9.0Hz,1H), 7.19 (s,1H), 7.46 à 7.50 (m,2H), 7.69 (d,J=16.0Hz,1H).

### (b) acide 3-{3-[3-(5-carboxypentyloxy)-5,5,8,8-tetramethyl-5,6,7,8-tetrahydronaphthalen-2-yl]-4-methoxyphenyl}acrylique.

De manière analogue à l'exemple 2(h) à partir de 900 mg (1.72 mmoles) de 3-{3-[3-(5-carboxypentyloxy)-5,5,8,8-tetramethyl-5,6,7,8-tetrahydronaphthalen-2-yl]-4-methoxyphenyl}acrylate d'éthyle obtenu précédemment, on obtient 647 mg (76%) du produit attendu sous forme d'une poudre blanche de point de fusion 166-168°C.

### EXEMPLE 23

### Acide 3-{3-[3-(5-carbamoylpentyloxy)-5,5,8,8-tetramethyl-5,6,7,8-tetrahydronaphthalen-2-yl]-4-methoxyphenyl}acrylique.

### (a) 3-{3-[3-(5-carbamoylpentyloxy)-5,5,8,8-tetramethyl-5,6,7,8-tetrahydronaphthalen-2-yl]-4-methoxyphenyl)acrylate d'éthyle.

Dans un ballon, on introduit une solution de 1.27 g (2.43 mmoles) de 3-{3-[3-(5-carboxypentyloxy)-5,5,8,8-tetramethyl-5,6,7,8-tetrahydronaphthalen-2-yl]-4-methoxyphenyl}acrylate d'éthyle obtenu à l'exemple 24(a) dissous dans 35 ml de dichlorométhane, ajoute 505 µl (2.55 mmoles) de dicyclohexylamine et agite 10 minutes. On ajoute ensuite goutte à goutte 186 µl (2.55 mmoles) de chlorure de thionyle et agite 10 minutes à température ambiante. On evapore le milieu réactionnel à sec, reprend le résidu obtenu dans de l'éther éthylique, filtre la suspension et évapore le filtrat. Le résidu obtenu est dissous dans 15 ml de THF et introduit goutte à goutte sous azote dans un tricol contenant 20 ml de THF, 405 µl (2.92 mmoles) de triéthylamine et 160 µl d'une solution d'ammoniaque à 34% (2.67 mmoles). On agite à température ambiante pendant 30 minutes, verse le milieu réactionnel dans l'eau, acidifie à pH=1 avec de l'acide chlorhydrique 1N, extrait avec de l'éther éthylique, décante la phase organique, séche sur sulfate de magnésium, filtre, évapore. Le résidu obtenu est purifié par chromatographie sur colonne de silice élué par un mélange heptane et d'acétate d'éthyle (80-20). Après évaporation des solvants, on obtient 750 mg (60%) de produit attendu sous forme d'une poudre blanche de point de fusion 133-135°C.

### (b) acide 3-{3-[3-(5-carbamoylpentyloxy)-5,5,8,8-tetramethyl-5,6,7,8-tetrahydronaphthalen-2-yl]-4-methoxyphenyl}acrylique.

De manière analogue à l'exemple 2(h) à partir de 900 mg (1.4 mmoles) de 3-{3-[3-(5-carbamoylpentyloxy)-5,5,8,8-tetramethyl-5,6,7,8-tetrahydronaphthalen-2-yl]-4-methoxyphenyl}acrylate d'éthyle obtenu précédemment, on obtient 190 mg (27%) du produit attendu sous forme d'une poudre blanche de point de fusion 179-181°C.

### EXEMPLE 24

### Acide 3-{3-[3-(7-carbamoylheptyloxy)-5,5,8,8-tetramethyl-5,6,7,8-tetrahydronaphthalen-2-yl]-4-methoxy-phenyl}acrylique.

### (a) 3-{3-[3-(7-carbamoylpentyloxy)-5,5,8,8-tetramethyl-5,6,7,8-tetrahydronaphthalen-2-yl]-4-methoxyphenyl}acrylate d'éthyle.

De manière analogue à l'exemple 25(a) à partir de 1.5g (2.70 mmoles) de 3-{3-[3-(7-carboxyheptyloxy)-5,5,8,8-tetramethyl-5,6,7,8-tetrahydronaphthalen-2-yl]-4-methoxyphenyl}acrylate d'éthyle. obtenu à l'exemple 23(a), on obtient 1 g (68%) du produit attendu sous forme d'une poudre beige de point de fusion 132-134°C.

### (b) acide 3-{3-[3-(7-carbamoylheptyloxy)-5,5,8,8-tetramethyl-5,6,7,8-tetrahydronaphthalen-2-yl]-4-methoxyphenyl}acrylique.

De manière analogue à l'exemple 2(h) à partir de 1 g (1.8 mmoles) de 3-{3-[3-(7-carbamoylheptyloxy)-5,5,8,8-tetramethyl-5,6,7,8-tetrahydronaphthalen-2-yl]-4-methoxyphenyl}-acrylate d'éthyle obtenu précédemment, on obtient 700 mg (74%) du produit attendu sous forme d'une poudre blanche de point de fusion 199-201°C.

### EXEMPLE 25

### Acide 3-{4-methoxy-3-[5,5,8,8-tetramethyl-3-(2-morpholin-4-yl-ethoxy)-5,6,7,8-tetrahydro-naphthalen-2-yl]-phenyl}-acrylique.

### (a) 3-{4-methoxy-3-[5,5,8,8-tetramethyl-3-(2-morpholin-4-yl-ethoxy)-5,6,7,8-tetrahydronaphthalen-2-yl]-phenyl}acrylate d'éthyle

De manière analogue à l'exemple 16(a) par réaction de 1.4 g (3.4 mmoles) de 3-[3-(3-hydroxy-5,5,8,8-tetramethyl-5,6,7,8-tetrahydro-naphthalen-2-yl)-4-methoxy phenyl]acrylate d'éthyle obtenu à l'exemple 13(f) avec 760 mg (4.08 mmoles) de chlorhydrate de 4-(2-chloroethyl)morpholine, on obtient 1.5 g (85%) du produit attendu sous forme d'une huile jaune.
RMN ¹H (CDCl₃) :1.27 (s,6H), 1.29 à 1.35 (m,9H), 1.70 (s,4H), 2.37 (m,4H), 2.60 (t,*J*=5.75Hz,2H), 3.59 (m,4H), 3.81 (s,3H), 4.05 (t,*J*=5.50Hz,2H), 4.25 (q,*J*=7.0Hz,2H), 6.30 (d,*J*=15.75Hz,1H), 6.85 (s,1H), 6.91 à 6.95 (m,1H), 7.16 (s,1H), 7.44 à 7.49 (m,2H), 7.65 (d,*J*=16.0Hz,1H).

### (b) acide 3-{4-methoxy-3-[5,5,8,8-tetramethyl-3-(2-morpholin-4-yl-ethoxy)-5,6,7,8-tetrahydronaphthalen-2-yl]phenyl}acrylique.

De manière analogue à l'exemple 2(h) à partir de 1.5 g (2.9 mmoles) de 3-{4-methoxy-3-[5,5,8,8-tetramethyl-3-(2-morpholin-4-yl-ethoxy)-5,6,7,8-tetrahydronaphthalen-2-yl]phenyl}acrylate d'éthyle obtenu précédemment, on obtient 1.17 g (83%) du produit attendu sous forme d'une poudre blanche se décomposant à partir de 140°C.
RMN ¹H (CD₃OD(Réf:q à δ=2.85ppm)):0.79 (s,6H), 0.87 (s,6H), 1.26 (s,4H), 1.90 (m,4H), 2.18 (t,*J*=5.0Hz,2H), 3.07 (m,4H), 3.31 (s,3H), 3.61 (t,J=5.0Hz,2H), 5.91 (d,*J*=15.75Hz,1H), 6.45 (s,1H), 6.56 (d,*J*=8.5Hz,1H), 6.62 (s,1H), 6.87 (d,*J*=1.75Hz,1H), 7.01 à 7.09 (m,2H).

### EXEMPLE 26

### Acide 3-{4-methoxy-3-[5,5,8,8-tetramethyl-3-(2-piperidin-1-yl-ethoxy)-5,6,7,8-tetrahydronaphthalen-2-yl]-phenyl}acrylique

### (a) 3-{4-methoxy-3-[5,5,8,8-tetramethyl-3-(2-piperidin-1-yl-ethoxy)-5,6,7,8-tetrahydronaphthalen-2-yl]-phenyl}acrylate d'éthyle.

De manière analogue à l'exemple 16(a) par réaction de 1.4 g (3.4 mmoles) de 3-[3-(3-hydroxy-5,5,8,8-tetramethyl-5,6,7,8-tetrahydronaphthalen-2-yl)-4-methoxy phenyl]acrylate d'éthyle obtenu à l'exemple 13(f) et de 760 mg (4.08 mmoles) de chlorhydrate de 1-(2-chloroethyl)piperidine, on obtient 1.57 g (89%) du produit attendu sous forme d'une huile incolore.
RMN ¹H (CDCl₃): 1.22 à 1.38 (m,16H), 1.46 à 1.55 (m,4H), 1.69 (m,5H), 2.36 (m,4H), 2.60 (t,*J*=6.25Hz,2H), 3.81 (s,3H), 4.05 (t,*J*=6.0Hz,2H), 4.24 (q,*J*=7.25Hz,2H), 6.30 (d,*J*=16.0Hz,1H), 6.85 (s,1H), 6.93 (d,*J*=9.25Hz,1H), 7.16 (s,1H), 7.44 à 7.48 (m,2H), 7.66 (d,*J*=16.0Hz,1H).

### (b) acide 3-{4-methoxy-3-[5,5,8,8-tetramethyl-3-(2-piperidin-1-yl-ethoxy)-5,6,7,8-tetrahydronaphthalen-2-yl]phenyl}acrylique

De manière analogue à l'exemple 2(h) à partir de 1.57 g (3 mmoles) de 3-{4-methoxy-3-[5,5,8,8-tetramethyl-3-(2-piperidin-1-yl-ethoxy)-5,6,7,8-tetrahydronaphthalen-2-yl]-phenyl}-acrylate d'éthyle obtenu précédemment, on obtient 900 mg (61%) du produit attendu sous forme d'une poudre blanche de point de fusion 251-253°C.

### EXEMPLE 27

### Acide 3-{4-methoxy-3-[3-(2-methoxymethoxybenzyloxy)-5,5,8,8-tetramethyl-5,6,7,8-tetrahydronaphthalen-2-yl]-phenyl}acrylique

### (a) 2-methoxymethoxybenzaldehyde.

De manière analogue à l'exemple 1(a) par réaction de 30 g (245 mmoles) de 2-Hydroxybenzaldehyde avec 21 ml (270 mmoles) de chlorure de méthoxyméthyle on obtient 40.7 g (100%) de produit attendu sous forme d'une huile blanchâtre.
RMN ¹H (CDCl₃): 3.53 (s,3H), 5.31 (s,2H), 7.09 (t,*J*=7.5Hz,1H),7.22 (d,*J*=8.25Hz,1H), 7.50 à 7.57 (m,1H), 7.85 (dd,*J*_{*1*}=7.75Hz,*J*_{*2*}=1.75Hz,1H), 10.51 (s,1H).

### (b) (2-methoxymethoxyphenyl)methanol.

Dans un ballon de 2 litres sous courant d'azote, on introduit une solution de 40 g (241 mmoles) de 2-methoxymethoxybenzaldehyde dissous dans 250 ml de THF et ajoute par petites portions 3.65 g (96 mmoles) d'hydrure de lithium aluminium. Le milieu réactionnel est agité à température ambiante pendant 15 minutes avant d'être hydrolisé lentement par une solution saturée de chlorure d'ammonium, puis acidifié à pH=1 par une solution d'acide chlohydrique 1N. On extrait le milieu réactionnel avec de l'acétate d'éthyle, décante la phase organique, sèche sur sulfate de magnésium, filtre, évapore le fitrat. On recueille 39.2 g (96%) d'une huile incolore.
RMN ¹H (CDCl₃) :2.29 (m,1H), 3.49 (s,3H), 4.80 (d,J=6.0Hz,2H), 5.25 (s,2H), 6.98 à 7.12 (m,2H), 7.23 à 7.34 (m,2H).

### (c) chlorure de 2-methoxymethoxybenzyle.

Dans un tricol de 250 ml sous courant d'azote, on introduit successivement 5.0 g (29.7 mmoles) de (2-methoxymethoxyphenyl)methanol obtenu précédemment, 90 ml de dichlorométhane et 8.27 ml (59.5 mmoles) de triéthylamine. On ajoute ensuite goutte à goutte 2.76 g (35.7 mmoles) de chlorure de méthane sulfonyle et agite à température ambiante pendant 1 heure. On hydrolyse le milieu réactionnel par de l'acide chlorhydrique, extrait par du dichlorométhane, séche la phase organique sur du sulfate de magnesium, filtre et évapore le fitrat. L'huile orange obtenue est purifiée par chromatographie sur colonne de silice, éluée par un mélange d'acétate d'éthyle et d'heptane (1-9). On recueille après évaporation des solvants, 3.0 g (55%) du produit attendu sous forme d'une huile jaune.
RMN ¹H (CDCI3) :3.50 (s,3H), 4.67 (s,2H), 3.25 (s,2H), 6.99 (td,J1=7.5Hz,J2=1.0Hz,1H), 7.11(d,J=7.75Hz,1H), 7.24 à 7.38 (m,2H).

### (d) 3-{4-methoxy-3-[3-(2-methoxymethoxybenzyloxy)-5,5,8,8-tetramethyl-5,6,7,8-tetrahydronaphthalen-2-yl]phenyl}acrylate d'éthyle.

De manière analogue à l'exemple 1(a) par réaction de 2.5 g (6.12 mmoles) de 3-[3-(3-hydroxy-5,5,8,8-tetramethyl-5,6,7,8-tetrahydronaphthalen-2-yl)-4-methoxy phenyl]acrylate d'éthyle obtenu à l'exemple 13(f) avec 1.26 g (6.73 mmoles) de chlorure de 2-methoxymethoxybenzyle obtenu précédemment, on obtient 3.2 g (94%) de produit attendu sous forme d'une huile jaune.
RMN ¹H (CDCl₃) :1.27 à 1.35 (m,15H), 1.69 (s,4H), 3.43 (s,3H), 3.74 (s,3H), 4.25 (q,J=7.0Hz,2H), 5.07 (s,2H), 5.18 (s,2H), 6.29 (d,J=16.0Hz,1H), 6.90 à 6.95 (m,3H), 7.06 (d,J=7.5Hz,1H), 7.15 à 7.21 (m,3H), 7.47 (dd,J1=8.5Hz,J2=2.25Hz,1H), 7.52 (d,J=2.25Hz,1H), 7.66 (d,J=16.0Hz,1H).

### (e) acide 3-{4-methoxy-3-[3-(2-methoxymethoxybenzyloxy)-5,5,8,8-tetramethyl-5,6,7,8-tetrahydronaphthalen-2-yl]phenyl}acrylique

De manière analogue à l'exemple 2(h) à partir de 1.0 g (1.8 mmoles) de 3-{4-methoxy-3-[3-(2-methoxymethoxybenzyloxy)-5,5,8,8-tetramethyl-5,6,7,8-tetrahydro naphthalen-2-yl]phenyl}acrylate d'éthyle obtenu précédemment, on obtient 760 mg (80%) du produit attendu sous forme d'une poudre blanche de point de fusion 159-161°C.

### EXEMPLE 28

### Acide 3-{4-methoxy-3-[3-(3-methoxymethoxybenzyloxy)-5,5,8,8-tetramethyl-5,6,7,8-tetrahydronaphthalen-2-yl]phenyl}acrylique

### (a) 3-methoxymethoxybenzaldehyde.

De manière analogue à l'exemple 1(a) par réaction de 30 g (245 mmoles) de 3-hydroxybenzaldehyde avec 21 ml (270 mmoles) de chlorure de méthoxyméthyle on obtient 40.7 g (100%) de produit attendu sous forme d'une huile marron.
RMN ¹H (CDCl₃) : 3.49 (s,3H), 5.23 (s,2H), 7.27 à 7.32 (m,1H), 7.42 à7.55 (m,3H), 9.97 (s,1H).

### (b) (3-methoxymethoxyphenyl)methanol.

De manière analogue à l'exemple 29(b) par réaction de 40 g (240 mmoles) de 3-methoxymethoxybenzaldehyde avec 3.65 g (96 mmoles) d'hydrure de lithium aluminium, on obtient 40.7 g (100%) de produit attendu sous forme d'une huile incolore.
RMN ¹H (CDCl₃) :1.83 (br,1H), 3.48 (s,3H), 4.67 (s,2H), 5.18 (s,2H), 6.94 à 7.06 (m,3H), 7.24 à 7.31 (m,1H).

### (c) chlorure de 3-methoxymethoxybenzyle.

De manière analogue à l'exemple 29(c) par réaction de 5.0 g (29.7 mmoles) de (3-methoxymethoxyphenyl)methanol avec 8.27 ml (59.5 mmoles) de triéthylamine et 2.76 ml (35.7 mmoles) de chlorure de méthane sulfonyle on obtient 2.66 g (48%) de produit attendu sous forme d'une huile jaune.
RMN ¹H (CDCl₃) :3.48 (s,3H), 4.55 (s,2H), 5.22 (s,2H), 6.97 à 7.07 (m,3H), 7.24 à 7.30 (m,1 H).

### (d) 3-{4-methoxy-3-[3-(3-methoxymethoxybenzyloxy)-5,5,8,8-tetramethyl-5,6,7,8-tetrahydronaphthalen-2-yl]phenyl}acrylate d'éthyle.

De manière analogue à l'exemple 1(a) par réaction de 2.5 g (6.12 mmoles) de 3-[3-(3-hydroxy-5,5,8,8-tetramethyl-5,6,7,8-tetrahydronaphthalen-2-yl)-4-methoxyphenyl]acrylate d'éthyle obtenu à l'exemple 13(f) avec 1.26 g (6.73 mmoles) de chlorure de 3-methoxymethoxybenzyle obtenu précédemment, on obtient 2.90 g (85%) de produit attendu sous forme d'une huile jaune.
RMN ¹H (CDCl₃) :1.27 à 1.35 (m,15H), 1.69 (s,4H), 3.43 (s,3H), 3.77 (s,3H), 4.24 (q,J=7.25Hz,2H), 4.99 (s,2H), 5.09 (s,2H), 6.31 (d,J=15.75Hz,1H), 6.88 à 6.96 (m,5H), 7.16 à 7.23 (m,2H), 7.45 à 7.51 (m,2H), 7.67 (d,J=15.75Hz,1H).

### (e) acide 3-{4-methoxy-3-[3-(3-methoxymethoxybenzyloxy)-5,5,8,8-tetramethyl-5,6,7,8-tetrahydronaphthalen-2-yl]phenyl}acrylique.

De manière analogue à l'exemple 2(h) à partir de 1.0 g (1.79 mmoles) de 3-{4-methoxy-3-[3-(3-methoxymethoxybenzyloxy)-5,5,8,8-tetramethyl-5,6,7,8-tetrahydro-naphthalen-2-yl]phenyl}acrylate d'éthyle obtenu précédemment, on obtient 813 mg (85%) du produit attendu sous forme d'une poudre blanche de point de fusion 144-146°C.

### EXEMPLE 29

### Acide 3-{4-methoxy-3-[3-(4-methoxymethoxybenzyloxy)-5,5,8,8-tetramethyl-5,6,7,8-tetrahydronaphthalen-2-yl]phenyl}acrylique

### (a) 4-methoxymethoxybenzaldehyde.

De manière analogue à l'exemple 1(a) par réaction de 30 g (245 mmoles) de 4-hydroxybenzaldehyde avec 21 ml (270 mmoles) de chlorure de méthoxyméthyle on obtient 40.7 g (100%) de produit attendu sous forme d'une huile orange.
RMN ¹H (CDCl₃) : 3.49 (s,3H), 5.28 (s,2H), 7.15 (d,J=8.75Hz,2H), 7.84 (d,J=8.75Hz,2H), 9.9 (s,1H).

### (b) (4-methoxymethoxyphenyl)methanol.

De manière analogue à l'exemple 29(b) par réaction de 40 g (240 mmoles) de 4-methoxymethoxybenzaldehyde avec 3.65 g (96 mmoles) d'hydrure de lithium aluminium, on obtient 15 g (37%) de produit attendu sous forme d'une huile incolore.
RMN ¹H (CDCl₃) :1.71 (t,J=5.75Hz,1H), 3.47 (s,3H), 4.61 (d,J=5.75Hz,2H), 5.17 (s,2H), 7.02 (d,J=8.5Hz,2H), 7.29 (d,J=8.5Hz,2H).

### (c) chlorure de 4-methoxymethoxybenzyle.

De manière analogue à l'exemple 29(c) par réaction de 5.0 g (29.7 mmoles) de (4-methoxymethoxyphenyl)methanol avec 8.27 ml (59.5 mmoles) de triéthylamine et 2.76 ml (35.7 mmoles) de chlorure de méthane sulfonyle, on obtient 2.66 g (48%) de produit attendu sous forme d'une huile jaune.
RMN ¹H (CDCl₃) :3.47 (s,3H), 4.56 (s,2H), 5.17 (s,2H), 7.02 (d,J=8.75Hz,2H), 7.31 (d,J=8.75Hz,2H).

### (d) 3-{4-methoxy-3-[3-(4-methoxymethoxybenzyloxy)-5,5,8,8-tetramethyl-5,6,7,8-tetrahdronaphthalen-2-yl]phenyl}acyylate d'éthyle.

De manière analogue à l'exemple 1(a) par réaction de 2.5 g (6.12 mmoles) de 3-[3-(3-hydroxy-5,5,8,8-tetramethyl-5,6,7,8-tetrahydronaphthalen-2-yl)-4-methoxyphenyl]acrylate d'éthyle obtenu à l'exemple 13(f) avec 1.26 g (6.73 mmoles) de chlorure de 4-methoxymethoxybenzyle obtenu précédemment, on obtient 3.0 g (87%) de produit attendu sous forme d'une poudre blanche de point de fusion 106-108°C

### (e) acide 3-{4-methoxy-3-[3-(4-methoxymethoxybenzyloxy)-5,5,8,8-tetramethyl-5,6,7,8-tetrahydronaphthalen-2-yl]phenyl}acrylique

De manière analogue à l'exemple 2(h) à partir de 1.0 g (1.79 mmoles) de 3-{4-methoxy-3-[3-(4-methoxymethoxybenzyloxy)-5,5,8,8-tetramethyl-5,6,7,8-tetrahydro-naphthalen-2-yl]phenyl}acrylate d'éthyle obtenu précédemment, on obtient 877 mg (91%) du produit attendu sous forme d'une poudre blanche de point de fusion 184-186°C.

### EXEMPLE 30

### Acide 3-{4-méthoxy-3-[3-(3-hydroxybenzyloxy)-5,5,8,8-tetramethyl-5,6,7,8-tetrahydronaphthalen-2-yl]-phenyl}acrylique.

### (a) 3-{4-methoxy-3-[3-(3-hydroxybenzyloxy)-5,5,8,8-tetramethyl-5,6,7,8-tetrahydronaphthalen-2-yl]phenyl}acrylate d'éthyle.

De manière analogue à l'exemple 2(g) mais dans l'éthanol comme solvant, à partir de 1.9 g (3.4 mmoles) de 3-{4-methoxy-3-[3-(3-methoxymethoxybenzyloxy)-5,5,8,8-tetramethyl-5,6,7,8-tetrahydronaphthalen-2-yl]phenyl}acrylate d'éthyle obtenu à l'exemple 30(d), on obtient 1.38 g (79%) du produit attendu sous forme d'une poudre blanche de point de fusion 131-133°C.

### (b) acide 3-{4-methoxy-3-[3-(3-hydroxybenzyloxy)-5,5,8,8-tetramethyl-5,6,7,8-tetrahydronaphthalen-2-yl]phenyl}acrylique.

De manière analogue à l'exemple 2(h) à partir de 1.11 g (2.16 mmoles) de 3-{4-methoxy-3-[3-(3-hydroxy-benzyloxy)-5,5,8,8-tetramethyl-5,6,7,8-tetrahydronaphthalen-2-yl]phenyl}acrylate d'éthyle obtenu précédemment, on obtient 1.0 g (95%) du produit attendu sous forme d'une poudre blanche de point de fusion 229-231 °C.

### EXEMPLE 31

### Acide3-[4-fluoro-3-(3,5,5,8,8-pentamethyl-5,6,7,8-tetrahydronaphthalen-2-yl)phenyl] acrylique.

De manière analogue à l'exemple 1(c) par réaction de 2.0 g (8.12 mmoles) d'acide 3,5,5,8,8-pentaméthyl-5,6,7,8-tétrahydro-2-naphtylboronique (préparé à l'exemple 5(a) du brevet WO 97/33881) et de 1.54 g (6.25 mmoles) d'acide 3-(3-bromo-4-fluorophenyl)acrylique, on obtient 1.45 g (63%) du produit attendu sous forme d'une poudre beige de point de fusion 181-183°C.

### EXEMPLE 32

### Acide 3-{4-hydroxy-3-[3-(3-methoxybenzyloxy)-5,5,8,8-tetramethyl-5,6,7,8-tetrahydronaphthalen-2-yl]phenyl}acrylique

### (a) 2-bromo-3-(3-methoxybenzyloxy)-5,5,8,8-tetramethyl-5,6,7,8-tetrahydro naphthalene.

De manière analogue à l'exemple 1(a) par réaction de 5.0 g (17.7 mmoles) de 2-bromo-3-hydroxy-5,5,8,8-tetramethyl-5,6,7,8-tetrahydronaphthalene avec 3.0 g (19.4 mmoles) de chlorure de 3-methoxybenzyle, on obtient 7.14 g (100%) de produit attendu sous forme d'une huile marron.
RMN ¹H (CDCl₃) :1.21 (s,6h), 1.24 (s,6H), 1.64 (s,4H), 3.82 (s,3H), 5.09 (s,2H), 6.83 à 6.87 (m,2H), 7.03 à 7.08 (m,2H), 7.29 (m,1H), 7.43 (s,1H).

### (b) acide 3-(3-methoxybenzyloxy)-5,5,8,8-tetramethyl-5,6,7,8-tetrahydro-2-naphthyl boronique.

De manière analogue à l'exemple 1(b) à partir de 7.10 g (17.6 mmoles) de 2-bromo-3-(3-methoxybenzyloxy)-5,5,8,8-tetramethyl-5,6,7,8-tetrahydro-naphthalene obtenu précédemment, on obtient 4.85 g (74%) de produit attendu sous forme d'une poudre beige un peu collante.
RMN ¹H (CDCl₃) :1.26 (s,6H), 1.29 (s,6H), 1.67 (s,4H), 3.81 (s,3H), 5.06 (s,2H), 6.85 à 7.0 (m,3H), 7.31 (t,J=7.75Hz,1H), 7.78 (s,1H).

### (c) 3-(3-bromo-4-methoxymethoxyphenyl)acrylate d'éthyle.

De manière analogue à l'exemple 2(c) par réaction de 30 g (122 mmoles) de 3-(3-bromo-4-methoxymethoxyphenyl)acrylate d'éthyle obtenu précédemment avec 30.27g (135 mmoles) de triéthylphosphonoacétate, on obtient 34.6 g (90%) de produit attendu sous forme de cristaux blancs de point de fusion 64-66°C.

### (d) 3-(3-bromo-4-hydroxyphenyl)acrylate d'éthyle.

De manière analogue à l'exemple 2(g) à partir de 30 g (95 mmoles) de 3-(3-bromo-4-methoxymethoxyphenyl)acrylate d'éthyle obtenu précédemment, on obtient 25.8 g (100%) de produit attendu sous forme d'une poudre blanche de point de fusion 113-115°C.

### (e) 3-{4-hydroxy-3-[3-(3-methoxybenzyloxy)-5,5,8,8-tetramethyl-5,6,7,8-tetrahydronaphthalen-2-yl]phenyl}acrylate d'éthyle.

De manière analogue à l'exemple 1(c) par réaction de 1.0 g (3.7 mmoles) de 3-(3-bromo-4-hydroxyphenyl)acrylate d'éthyle obtenu précédemment, avec 1.77 g (4.8 mmoles) d' acide 3-(3-methoxybenzyloxy)-5,5,8,8-tetramethyl-5,6,7,8-tetrahydro-2-naphthylboronique obtenu à l'exemple 34(c), on obtient 630 mg (33%) de produit attendu sous forme d'une huile incolore.
RMN ¹H (CDCl₃):1.25 à 1.36 (m,15H), 1.70 (s,4H), 3.78 (s,3H), 4.26 (q,J=7.25Hz,2H), 4.99 (s,2H), 6.32 (d,J=15.75Hz,1H), 6.84 (d,J=8.5Hz,2H), 6.93 (s,1H), 6.98 à 7.02 (m,2H), 7.19 (d,J=8.5Hz,1H), 7.25 (s,1H), 7.40 (d,J=2.25Hz,1H), 7.69 (d,J=15.75Hz,1H).

### (f) acide 3-{4-hydroxy-3-[3-(3-methoxybenzyloxy)-5,5,8,8-tetramethyl-5,6,7,8-tetrahydronaphthalen-2-yl]phenyl}acrylique.

De manière analogue à l'exemple 2(h), à partir de 600 mg (1.16 mmoles) de 3-{4-hydroxy-3-[3-(3-methoxybenzyloxy)-5,5,8,8-tetramethyl-5,6,7,8-tetrahydronaphthalen-2-yl]phenyl}acrylate d'éthyle obtenu précédemment, on obtient 539 mg (95%) de produit attendu sous forme d'une poudre blanche de point de fusion 197-199°C.

### EXEMPLE 33

### Acide 3-{4-hydroxy-3-[3-(4-methoxybenzyloxy)-5,5,8,8-tetramethyl-5,6,7,8-tetrahydronaphthalen-2-yl]-phenyl}acrylique.

### (a) 2-bromo-3-(4-methoxybenzyloxy)-5,5,8,8-tetramethyl-5,6,7,8-tetrahydronaphthalene.

De manière analogue à l'exemple 1(a) par réaction de 5.0 g (17.7 mmoles) de 3-hydroxy-2-bromo-5,5,8,8-tetramethyl-5,6,7,8-tetrahydronaphthalene avec 3.0 g (19.4 mmoles) de chlorure de 4-methoxybenzyle, on obtient 7.14 g (100%) de produit attendu sous forme d'une huile marron.
RMN ¹H (CDCl₃) :1.21 (s,6h), 1.24 (s,6H), 1.64 (s,4H), 3.81 (s,3H), 5.04 (s,2H), 6.83 (s,1H), 6.92 (d,J=7.5Hz,2H), 7.39 (d,J=7.5Hz,2H), 7.43 (s,1H).

### (b) acide 3-(4-methoxybenzyloxy)-5,5,8,8-tetramethyl-5,6,7,8-tetrahydro-2-naphthylboronique.

De manière analogue à l'exemple 1(b) à partir de 7.14 g (17.7 mmoles) de 2-bromo-3-(4-methoxybenzyloxy)-5,5,8,8-tetramethyl-5,6,7,8-tetrahydro-naphthalene obtenu précédemment, on obtient 3.55 g (54%) de produit attendu sous forme d'une huile orange.
¹H (CDCl₃):1.28 (S,12H), 1.57 (s,4H), 3.82 (s,3H), 5.03 (s,2H), 5.78 (s,2H), 6.89 (s,1H), 6.93 (d,J=8.75Hz,1H), 7.35 (d,J=8.5Hz,1H), 7.78 (s,1H).

### (c) 3-{4-hydroxy-3-[3-(4-methoxybenzyloxy)-5,5,8,8-tetramethyl-5,6,7,8-tetrahydronaphthalen-2-yl]phenyl}acrylate d'éthyle.

De manière analogue à l'exemple 1(c) par réaction de 1.0 g (3.7 mmoles) de 3-(3-bromo-4-hydroxyphenyl)acrylate d'éthyle obtenu à l'exemple 34(d) avec 2.0 g (5.5 mmoles) d' acide 3-(4-methoxybenzyloxy)-5,5,8,8-tetramethyl-5,6,7,8-tetrahydro-2-naphthylboronique obtenu précédemment, on obtient 930 mg (49%) de produit attendu sous forme d'une huile incolore.
RMN ¹H (CDCl₃):1.29 (s,12H), 1.34 (t,J=7.0Hz,3H), 1.70 (s,4H), 3.72 (s,3H), 4.26 (q,J=7.25Hz,2H), 5.02 (s,2H), 6.33 (d,J=15.75Hz,1H), 6.70 (s,1H), 6.80 à 6.87 (m,3H), 7.02 (d,J=7.25Hz,2H), 7.18 à 7.25 (m,2H), 7.42 (d,J=2.25Hz,1H), 7.48 (d,J=8.5Hz,1H), 7.69 (d,J=16.0Hz,1H).

### (d) acide 3-{4-hydroxy-3-[3-(3-methoxybenzyloxy)-5,5,8,8-tetramethyl-5,6,7,8-tetrahydronaphthalen-2-yl]phenyl}acrylique.

De manière analogue à l'exemple 2(h), à partir de 930 mg (1.8 mmoles) de 3-{4-hydroxy-3-[3-(4-methoxybenzyloxy)-5,5,8,8-tetramethyl-5,6,7,8-tetrahydronaphthalen-2-yl]phenyl}acrylate d'éthyle obtenu précédemment, on obtient 800 mg (91%) de produit attendu sous forme d'une poudre blanche de point de fusion 170-172°C.

### EXEMPLE 34

### Acide 3-{4-hydroxy-3-[3-(4-fluorobenzyloxy)-5,5,8,8-tetramethyl-5,6,7,8-tetrahydronaphthalen-2-yl]phenyl}acrylique.

### (a) 2-bromo-3-(4-fluorobenzyloxy)-5,5,8,8-tetramethyl-5,6,7,8-tetrahydro naphthalene.

De manière analogue à l'exemple 1(a) par réaction de 5.0 g (17.7 mmoles) de 3-hydroxy-2-bromo-5,5,8,8-tetramethyl-5,6,7,8-tetrahydronaphthalene avec 3.67 g (19.4 mmoles) de bromure de 4-fluorobenzyle, on obtient 4.3 g (62%) de produit attendu sous forme de cristaux marron clair de point de fusion : 74-76°C.

### (b) acide 3-(4-fluorobenzyloxy)-5,5,8,8-tetramethyl-5,6,7,8-tetrahydro-2-naphthyl boronique.

De manière analogue à l'exemple 1(b) à partir de 4.3 g (11.0 mmoles) de 2-bromo-3-(4-fluorobenzyloxy)-5,5,8,8-tetramethyl-5,6,7,8-tetrahydro-naphthalene obtenu précédemment, on obtient 3.24 g (83%) de produit attendu sous forme d'une huile orange.
RMN ¹H (CDCl₃) :1.27 (s,6H), 1.29 (s,6H), 1.67 (s,4H), 5.08 (s,2H), 5.81 (s,2H), 7.06 à 7.13 (m,2H), 7.38 à 7.43 (m,2H), 7.80 (s,1H),.

### (c) 3-{4-hydroxy-3-[3-(4-fluorobenzyloxy)-5,5,8,8-tetramethyl-5,6,7,8-tetrahydronaphthalen-2-yl]phenyl}acrylate d'éthyle.

De manière analogue à l'exemple 1(c) par réaction de 1.0 g (3.7 mmoles) de 3-(3-bromo-4-hydroxyphenyl)acrylate d'éthyle obtenu à l'exemple 34(d) avec 1.97 g (5.5 mmoles) d' acide 3-(4-fluorobenzyloxy)-5,5,8,8-tetramethyl-5,6,7,8-tetrahydro-2-naphthylboronique obtenu précédemment, on obtient 825 mg (44%) de produit attendu sous forme d'un solide blanc de point de fusion 123-125°C.

### (d) acide 3-(4-hydroxy-3-[3-(3-fluorobenzyloxy)-5,5,8,8-tetramethyl-5,6,7,8-tetrahydronaphthalen-2-yl]phenyl}acrylique.

De manière analogue à l'exemple 2(h), à partir de 800 mg (1.6 mmoles) de 3-{4-hydroxy-3-[3-(4-fluorobenzyloxy)-5,5,8,8-tetramethyl-5,6,7,8-tetrahydronaphthalen-2-yl]phenyl}acrylate d'éthyle obtenu précédemment, on obtient 425 mg (56%) de produit attendu sous forme d'une poudre blanche de point de fusion 157-159°C.

### EXEMPLE 35

### Acide 3-{4-hydroxy-3-[3-(4-chlorobenzyloxy)-5,5,8,8-tetramethyl-5,6,7,8-tetrahydronaphthalen-2-yl]phenyl}acrylique.

### (a) 2-bromo-3-(4-chlorobenzyloxy)-5,5,8,8-tetramethyl-5,6,7,8-tetrahydronaphthalene.

De manière analogue à l'exemple 1(a) par réaction de 5.0 g (17.7 mmoles) de 3-hydroxy-2-bromo-5,5,8,8-tetramethyl-5,6,7,8-tetrahydronaphthalene avec 3.12 g (19.4 mmoles) de chlorure de 4-chlorobenzyle, on obtient 7.22 g (100%) de produit attendu sous forme de cristaux marron clair de point de fusion : 108-110°C.

### (b) acide 3-(4-chlorobenzyloxy)-5,5,8,8-tetramethyl-5,6,7,8-tetrahydro-2-naphthyl boronique.

De manière analogue à l'exemple 1(b) à partir de 7.14 g (17.5 mmoles) de 2-bromo-3-(4-chlorobenzyloxy)-5,5,8,8-tetramethyl-5,6,7,8-tetrahydro-naphthalene obtenu précédemment, on obtient 3.50 g (54%) de produit attendu sous forme d'une huile orange.
RMN ¹H (CDCl₃) :1.26 (s,6H), 1.29 (s,6H), 1.67 (s,4H), 5.09 (s,2H), 5.72 (s,2H), 6.84 (s,1H), 7.37 (m,4H), 7.80 (s,1H).

### (c) 3-{4-hydroxy-3-[3-(4-chlorobenzyloxy)-5,5,8,8-tetramethyl-5,6,7,8-tetrahydronaphthalen-2-yl]phenyl}acrylate d'éthyle.

De manière analogue à l'exemple 1(c) par réaction de 1.0 g (3.7 mmoles) de 3-(3-bromo-4-hydroxyphenyl)acrylate d'éthyle obtenu à l'exemple 34(d) avec 2.05 g (5.5 mmoles) d' acide 3-(4-chlorobenzyloxy)-5,5,8,8-tetramethyl-5,6,7,8-tetrahydro-2-naphthylboronique obtenu précédemment, on obtient 590 mg (30%) de produit attendu sous forme d'un solide blanc de point de fusion 152-154°C.

### (d) acide 3-{4-hydroxy-3-[3-(4-chlorobenzyloxy)-5,5,8,8-tetramethyl-5,6,7,8-tetrahydronaphthalen-2-yl]phenyl}acrylique.

De manière analogue à l'exemple 2(h), à partir de 580 mg (1.12 mmoles) de 3-{4-hydroxy-3-[3-(4-chlorobenzyloxy)-5,5,8,8-tetramethyl-5,6,7,8-tetrahydronaphthalen-2-yl]phenyl}acrylate d'éthyle obtenu précédemment, on obtient 382 mg (70%) de produit attendu sous forme d'une poudre blanche de point de fusion 147-149°C.

### EXEMPLE 36

### Acide 3-[4-Hydroxy-3-(3-methoxymethoxy-5,5,8,8-tetramethyl-5,6,7,8-tetrahydronaphthalen-2-yl)-phenyl]acrylique.

### (a) 3-{4-hydroxy-3-[3-methoxymethoxy-5,5,8,8-tetramethyl-5,6,7,8-tetrahydronaphthalen-2-yl]phenyl}acrylate d'éthyle.

De manière analogue à l'exemple 1(c) par réaction de 1.0 g (3.7 mmoles) de 3-(3-bromo-4-hydroxyphenyl)acrylate d'éthyle obtenu à l'exemple 34(d) avec 1.6 g (5.5 mmoles) d' acide 3-methoxymethoxy-5,5,8,8-tetramethyl-5,6,7,8-tetrahydro-2-naphthylboronique obtenu à l'exemple 13(b), on obtient 150 mg (9%) de produit attendu sous forme d'un solide blanc de point de fusion 101-103°C.

### (d) acide 3-{4-hydroxy-3-[3-methoxymethoxy-5,5,8,8-tetramethyl-5,6,7,8-tetrahydronaphthalen-2-yl]phenyl}acrylique.

De manière analogue à l'exemple 2(h), à partir de 150 mg (0.34 mmole) de 3-{4-hydroxy-3-[3-methoxymethoxy-5,5,8,8-tetramethyl-5,6,7,8-tetrahydro-naphthalen-2-yl]phenyl}acrylate d'éthyle obtenu précédemment, on obtient 100 mg (72%) de produit attendu sous forme d'une poudre blanche de point de fusion 191-193°C.

### EXEMPLE 37

### Acide 3-(3',5'-dii-tert-butyl-6-hydroxy-2'-propyloxybiphenyl-3-yl)-acrylique.

### (a) 1-bromo-3,5-di-tert-butyl-2-propyloxy-benzene.

De manière analogue à l'exemple 1(a) par réaction de 15 g (52.6 mmoles) de 6-bromo-2,4-di-tert-butyl-phenol avec 7.4 g (57.9 mmoles) de 1-bromopropane, on obtient 17.2 g (100%) de produit attendu sous forme de cristaux orange de point de fusion : 41-43°C.

### (b) acide 2-propyloxy-3,5-di-tert-butylphenylboronique.

De manière analogue à l'exemple 1(b) à partir de 16.0 g (48.9 mmoles) de 1-bromo-3,5-di-tert-butyl-2-propyloxybenzene, obtenu précédemment, on obtient 9.1 g (63%) de produit attendu sous forme d'une poudre blanche de point de fusion 104-106°C.

### (c) 3-(3',5'-Di-tert-butyl-6-hydroxy-2'-propyloxybiphenyl-3-yl)acrylate d'ethyle.

De manière analogue à l'exemple 1(c) par réaction de 1.0 g (3.7 mmoles) de 3-(3-bromo-4-hydroxyphenyl)acrylate d'éthyle obtenu à l'exemple 34(d) avec 2.14 g (7.33 mmoles) d' acide 2-propyloxy-3,5-di-tert-butylphenylboronique obtenu précédemment on obtient 70 mg (4%) de produit attendu sous forme d'une huile incolore.
RMN ¹H (CDCl₃) :0.85 (t,J=7.5Hz,3H), 1.34 (s,9H), 1.40 (t,J=7.75Hz,3H), 1.45 (s,9H), 1.55 à 1.59 (m,2H), 3.43 à 3.65 (m,2H), 4.27 (q,J=8.25Hz,2H), 6.37 (d,J=15.75Hz,1H), 7.05 (d,J=8.25Hz,1H), 7.16 (d,J=2.5Hz,1H), 7.30 (s,1H), 7.43 (d,J=2.5Hz,1H), 7.50 à 7.55 (m,2H), 7.72 (d,J=16.0Hz,1H).

### (d) acide 3-(3',5'-di-tert-butyl-6-hydroxy-2'-propyloxybiphenyl-3-yl)acrylique.

De manière analogue à l'exemple 2(h), à partir de 70 mg (0.16 mmole) de 3-(3',5'-di-*tert*-butyl-6-hydroxy-2'-propyloxybiphenyl-3-yl)acrylate d'ethyle obtenu précédemment, on obtient 50 mg (75%) de produit attendu sous forme d'une poudre blanche de point de fusion 187-189°C

### EXEMPLE 38

### Acide 3-(3',5'-di-tert-butyl-6-hydroxy-2'-butyloxybiphenyl-3-yl)acrylique.

### (a) 1-bromo-3,5-di-tert-butyl-2-butyloxybenzene.

De manière analogue à l'exemple 1(a) par réaction de 15 g (52.6 mmoles) de 2-bromo-4,6-di-*tert*-butylphenol avec 7.93 g (57.9 mmoles) de 1-bromobutane, on obtient 17.7 g (100%) de produit attendu sous forme d'une huile orange .
RMN ¹H (CDCl₃) :0.99 (t,J=7.5Hz,3H), 1.28 (s,9H), 1.39 (s,9H), 1.45 à 1.60 (m,2H), 1.80 à 1.92 (m,2H), 4.03 (t,J=7.0Hz,2H), 7.27 (d,J=2.5Hz,1H), 7.39 (d,J=2.5Hz,1h).

### (b) acide 2-butyloxy-3,5-di-tert-butylphenylboronique.

De manière analogue à l'exemple 1(b) à partir de 16.0 g (46.8 mmoles) de 1-bromo-3,5-di-*tert*-butyl-2-butyloxybenzene, obtenu précédemment, on obtient 14g (97%) de produit attendu sous forme d'une poudre blanche de point de fusion 92-94°C.

### (c) 3-(3',5'-di-tert-butyl-6-hydroxy-2'-butyloxybiphenyl-3-yl)acrylate d'ethyle.

De manière analogue à l'exemple 1(c) par réaction de 6.2 g (22.8 mmoles) de 3-(3-bromo-4-hydroxyphenyl)acrylate d'éthyle obtenu à l'exemple 34(d) avec 14 g (45.7 mmoles) d' acide 2-butyloxy-3,5-di-*tert*-butylphenylboronique obtenu précédemment on obtient 700 mg (7%) de produit attendu sous forme d'une huile jaune.
RMN ¹H (CDCl₃) :0.78 (t,J=7.5Hz,3H), 1.31 à1.56 (m,25H), 3.20 à 3.66 (m,2H), 4.27 (q,J=7.25Hz,2H), 6.37 (d,J=16.0Hz,1H), 7.06 (d,J=8.25Hz,1H), 7.16 (d,J=2.5Hz,1H), 7.30 (s,1H), 7.43 (d,J=2.25Hz,1H), 7.49 à 7.55 (m,2H), 7.72 (d,J=16.0Hz,1H).

### (d) acide 3-(3',5'-di-tert-butyl-6-hydroxy-2'-butyloxybiphenyl-3-yl)acrylique.

De manière analogue à l'exemple 2(h), à partir de 430 mg (0.95 mmole) de 3-(3',5'-di-*tert*-butyl-6-hydroxy-2'-butyloxybiphenyl-3-yl)acrylate d'ethyle obtenu précédemment, on obtient 300 mg (74%) de produit attendu sous forme d'une poudre blanche de point de fusion 194-196°C.

### EXEMPLE 39

### Acide 3-(2'-butoxy-3',5'-di-tert-butyl-6-methoxybiphenyl-3-yl)acrylique.

### (a) 3-(2'-butoxy-3',5'-di-tert-butyl-6-methoxybiphenyl-3-yl)acrylate d'ethyle.

De manière analogue à l'exemple 1(a) par réaction de 230 mg ( 0.5 mmole) de 3-(3',5'-di-*tert*-butyl-6-hydroxy-2'-butyloxybiphenyl-3-yl)acrylate d'ethyle obtenu à l'exemple 40(c) avec 82 mg (0.58 mmole) de iodométhane, on obtient 230 mg (100%) de produit attendu sous forme d'une huile jaune.
RMN ¹H (CDCl₃) :0.67 (t,J=7.25Hz,3H), 1.07 (m,2H), 1.25 à 1.36 (m,14H), 1.43 (s,9H), 3.40 (br,2H), 3.84 (s,3H), 4.25 (q,J=7.0Hz,2H), 6.33 (d,J=15.5Hz,1H), 6.98 (d,J=8.5Hz,1H), 7.11 (d,J=2.25Hz,1H), 7.35 (d,J=2.5Hz,1H), 7.48 (dd,J1=8.5Hz,J2=2.25Hz,1H), 7.56 (d,J=2.25Hz,1H), 7.68 (d,J=15.75Hz,1H).

### (b) acide 3-(2'-butoxy-3',5'-di-tert-butyl-6-methoxybiphenyl-3-yl)acrylique.

De manière analogue à l'exemple 2(h), à partir de 230 mg (0.51 mmole) de 3-(2'-butoxy-3',5'-di-*tert*-butyl-6-methoxybiphenyl-3-yl)acrylate d'ethyle obtenu précédemment, on obtient 120 mg (55%) de produit attendu sous forme d'une poudre blanche de point de fusion 204-206°C.

### EXEMPLE 40

### Acide 3-(3',5'-di-tert-butyl-methoxy-2'-propoxybiphenyl-3-yl)acrylique.

### (a) 3-(3',5'-di-tert-butyl-6-methoxy-2'-propoxybiphenyl-3-yl)acrylate d'éthyle.

De manière analogue à l'exemple 1(c) par réaction de 1.0 g (3.5 mmoles) de 3-(3-bromo-4-methoxyphenyl)acrylate d'éthyle.obtenu à l'exemple 13(d) avec 1.54 g (5.26 mmoles) d' acide .2-propyloxy-3,5-di-*tert*-butylphenylboronique obtenu à l'exemple 39(b) on obtient 1.20 g (76%) de produit attendu sous forme d'une poudre blanche de point de fusion :107-109°C.

### (b) acide 3-(3',5'-di-tert-butyl-6-methoxy-2'-propoxybiphenyl-3-yl)acrylique.

De manière analogue à l'exemple 2(h), à partir de 1.0 g (2.2 mmoles) de 3-(3',5'-di-*tert*-butyl-6-methoxy-2'-propoxy-biphenyl-3-yl)-acrylate d'éthyle obtenu précédemment, on obtient 800 mg (85%) de produit attendu sous forme d'une poudre blanche de point de fusion 207-209°C.

### EXEMPLE 41

### Acide 3-[4-hydroxy-3-(5,5,8,8-tetramethyl-4-propoxy-5,6,7,8-tetrahydro-naphthalen-2-yl)-phenyl]-acrylique.

### (a) 3-bromo-5,5,8,8-tetramethyl-5,6,7,8-tetrahydro-naphthalen-1-ol.

Dans un tricol de 2 litres et sous courant d'azote on introduit 9.73 g (73 mmoles) de chlorure d'aluminium avec 80 ml de dichlorométhane et ajoute goutte à goutte une solution de 25 g (145 mmoles) de 3-bromophénol dans 100 ml de dichlorométhane et agite à température ambiante 15 minutes. On coule ensuite une solution de 26.2 g (143 mmoles) de 2,5-dichloro-2,5-dimethyl-hexane dans 100 ml de dichlorométhane et agite à température ambiante 1 heure. On rajoute ensuite en quatre fois et avec une heure d'agitation entre chaques coulées, une solution de 40g (218 mmoles) de 2,5-dichloro-2,5-dimethyl-hexane dans 200 ml de dichlorométhane et agite pendant 15 heures. On évapore le dichlorométhane du milieu réactionnel, reprend le résidu dans l'acétate d'éthyle et l'eau, décante la phase organique, sèche sur sulfate de magnésium, filtre et évapore. Le résidu obtenu est purifié par chromatographie sur silice en éluant tout d'abord avec de l'heptane puis avec un mélange heptane/acétate d'éthyle (95 /5). Après évaporation des solvants on obtient 30 g (73%) du produit attendu sous forme de cristaux huileux noir.
RMN ¹H (CDCl₃) :1.25 (s,6H), 1.38 (s,6H), 1.58 à 1.67 (m,4H), 4.99 (s,1H), 6.25 (d,J=2.0Hz,1H), 7.04 (d,J=2.0Hz,1H).

### (b) 2-bromo-4-propyloxy-5,5,8,8-tetraméthyl-5,6,7,8-térahydronaphtalene.

De manière analogue à l'exemple 1(a) par réaction de 18 g ( 63.6 mmoles) de 3-bromo-5,5,8,8-tetramethyl-5,6,7,8-tetrahydronaphthalen-1-ol obtenu à l'exemple précédant avec 8.61 g (70 mmoles) de 1-bromopropane, on obtient 16.2 g (78%) de produit attendu sous forme de cristaux jaunes de point de fusion : 86-88°C.

### (c) acide 4-propyloxy-5,5,8,8-tetramethyl-5,6,7,8-tetrahydro-2-naphthylboronique.

De manière analogue à l'exemple 1(b) à partir de 16.0 g (49.2 mmoles) de 2-bromo-4-propyloxy-5,5,8,8-tetraméthyl-5,6,7,8-tétrahydronaphtalene obtenu précédemment, on obtient 6.2 g (46%) de produit attendu sous forme d'une poudre blanche de point de fusion 230-232°C.

### (d) 3-[4-hydroxy-3-(5,5,8,8-tetramethyl-4-propoxy-5,6,7,8-tetrahydro-naphthalen-2-yl)phenyl]acrylate d'éthyle.

De manière analogue à l'exemple 1(c) par réaction de 1.0 g (3.7 mmoles) de 3-(3-bromo-4-hydroxyphenyl)acrylate d'éthyle obtenu à l'exemple 34(d) avec 1.61 g (5.5 mmoles) d'acide 4-propyloxy-5,5,8,8-tetramethyl-5,6,7,8-tetrahydro-2-naphthylboronique obtenu à l'exemple 43(c), on obtient 640 mg (40%) de produit attendu sous forme d'une poudre blanche de point de fusion :114-116°C.

### (e) acide 3-[4-hydroxy-3-(5,5,8,8-tetramethyl-4-propoxy-5,6,7,8-tetrahydronaphthalen -2-yl)phenyl]acrylique.

De manière analogue à l'exemple 2(h), à partir de 640 mg (1.47 mmole) de 3-[4-hydroxy-3-(5,5,8,8-tetramethyl-4-propoxy-5,6,7,8-tetrahydro-naphthalen-2-yl) phenyl]acrylate d'éthyle obtenu précédemment, on obtient 270 mg (45%) de produit attendu sous forme d'une poudre blanche de point de fusion 209-211°C.

### EXEMPLE 42

### Acide 3-{4-methoxy-3-[3-benzyloxy-5,5,8,8-tetramethyl-5,6,7,8-tetrahydronaphthalen-2-yl]phenyl}acrylique.

### (a) 3-{4-methoxy-3-[3-benzyloxy-5,5,8,8-tetramethyl-5,6,7,8-tetrahydro-naphthalen-2-yl]phenyl}acrylate d'éthyle.

De manière analogue à l'exemple 1(a) par réaction de 1.40 g (3.4 mmoles) de 3-[3-(3-hydroxy-5,5,8,8-tetramethyl-5,6,7,8-tetrahydro-naphthalen-2-yl)-4-methoxyphenyl]acrylate d'éthyle obtenu à l'exemple 13(f) avec 450 µl (3.77 mmoles) de bromure de benzyle, on obtient 1.64 g (97%) de produit attendu sous forme d'une huile jaune.
RMN ¹H (CDCl₃): 1.28 à 1.35 (m,15H), 1.69 (s,4H), 3.76 (s,3H), 4.25 (q,J=7.0Hz,2H), 5.02 (s,2H), 6.30 (d,J=15.75Hz,1H), 6.89 (s,1H), 6.93 (d,J=8.25Hz,1H), 7.19 (s,1H), 7.20 à 7.35 (m,5H), 7.43 à 7.52 (m,2H), 7.67 (d,J=16.0Hz,1H).

### (b) acide 3-{4-methoxy-3-[3-benzyloxy-5,5,8,8-tetramethyl-5,6,7,8-tetrahydronaphthalen-2-yl]phenyl}acrylique

De manière analogue à l'exemple 2(h) à partir de 1.64 g (3.3 mmoles) de 3-{4-methoxy-3-[3-benzyloxy-5,5,8,8-tetramethyl-5,6,7,8-tetrahydro-naphthalen-2-yl]phenyl}acrylate d'éthyle obtenu précédemment, on obtient 1.27 g (82%) de produit attendu sous forme d'une poudre blanche de point de fusion 200-202°C.

### EXEMPLE 43

### Acide 3-methyl-5-[3-(3,5,5,8,8-pentamethyl-5,6,7,8-tetrahydro-naphthalen-2-yl)-phenyl]-penta-2,4-dienoique.

### (a) 3-(3,5,5,8,8-pentamethyl-5,6,7,8-tetrahydro-naphthalen-2-yl)benzaldehyde.

De manière analogue à l'exemple 1(c) par réaction de 2.0 g (8.12 mmoles) d'acide 3,5,5,8,8-pentaméthyl-5,6,7,8-tétrahydro-2-naphtylboronique (préparé à l'exemple 5(a) du brevet WO 97/33881) et de 1.89 g (10.15 mmoles) de 3-bromo-benzaldéhyde, on obtient 3.10 g (100%) du produit attendu sous forme d'une poudre blanche de point de fusion 99-101°C.

### (b) 3-methyl-5-[3-(3,5,5,8,8-pentamethyl-5,6,7,8-tetrahydronaphthalen-2-yl)phenyl] penta-2,4-dienoate d'éthyle.

De manière analogue à l'exemple 2(c) par réaction de 2.0 g (6.53 mmoles) de 3-(3,5,5,8,8-pentamethyl-5,6,7,8-tetrahydronaphthalen-2-yl)benzaldehyde obtenu à l'exemple précédent et de 1.90 g (7.20 mmoles) de triéthyl-3-méthyl-4-phosphonocrotonate, on obtient 1.80 g (66%) du produit attendu sous forme d'une poudre blanche de point de fusion 83-85°C.

### (c) acide 3-methyl-5-[3-(3,5,5,8,8-pentamethyl-5,6,7,8-tetrahydronaphthalen-2-yl) phenyl]penta-2,4-dienoique.

De manière analogue à l'exemple 2(h) à partir de 1.00 g (2.4 mmoles) de 3-methyl-5-[3-(3,5,5,8,8-pentamethyl-5,6,7,8-tetrahydro-naphthalen-2-yl)phenyl]penta-2,4-dienoate d'éthyle obtenu précédemment et après séparation des isomères sur colonne de silice fine (7-35 µm) éluée par un mélange heptane /acétate d'éthyle (95/5), on obtient : 420 m g (45%) de produit attendu sous forme d'une poudre blanche de point de fusion 218-220°C.

### B. EXEMPLES DE TESTS

Les tests biologiques effectués correspondent à ceux décrits dans la présente demande. Les résultats obtenus sont regroupés dans le tableau suivant :

| N° composé | Binding RXR | Transactivation RXRα | |
|---|---|---|---|
| | K_{D} (nM) | agoniste AC₅₀ (nM) | antagoniste IC₅₀ (nM) |
| 1 | 394 | 384 | ------ |
| 2 | 23 | inactif | 920 |
| 3 | 10 | inactif | 729 |
| 4 | 17 | 165 | ----- |
| 5 | 76 | inactif | 4000 |
| 6 | 14 | inactif | 505 |
| 7 | 235 | inactif | 1100 |
| 8 | 231 | inactif | 631 |

### C. EXEMPLES DE FORMULATION

### 1) VOIE ORALE

(a) On prépare la composition suivante sous la forme d'un comprimé de 0,8 g

| | |
|---|---|
| Composé de l'exemple 3 | 0,005 g |
| Amidon prégélatinisé | 0,265 g |
| Cellulose microcristalline | 0,300 g |
| Lactose | 0,200 g |
| Stéarate de magnésium | 0,030 g |

Pour le traitement de l'acné, on administrera à un individu adulte 1 à 3 comprimés par jour pendant 3 à 6 mois selon la gravité du cas traité.
(b) On prépare une suspension buvable, destinée à être conditionnée en ampoules de 5 ml

| | |
|---|---|
| Composé de l'exemple 2 | 0,050 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme | q.s. |
| Eau purifiée | q.s.p.5 ml |

Pour le traitement de l'acné, on administrera à un individu adulte 1 ampoule par jour pendant 3 mois selon la gravité du cas traité.
(c) On prépare la formulation suivante destinée à être conditionnée en gélules :

| | |
|---|---|
| Composé de l'exemple 5 | 0,025 g |
| Amidon de maïs | 0,060 g |
| Lactose q.s.p. | 0,300 g |

Les gélules utilisées sont constituées de gélatine, d'oxyde de titane et d'un conservateur.

Dans le traitement du psoriasis, on administrera à un individu adulte, 1 gélule par jour pendant 30 jours.

### 2) VOIE TOPIQUE

(a) On prépare la crème Eau-dans-Huile non ionique suivante :

| | |
|---|---|
| Composé de l'exemple 6 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p | 100,000 g |

Cette crème sera appliquée sur une peau psoriatique 1 à 2 fois par jour pendant 30 jours.
(b) On prépare un gel en réalisant la formulation suivante :

| | |
|---|---|
| Composé de l'exemple 17 | 0,050 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p | 100,000 g |

Ce gel sera appliqué sur une peau atteinte de dermatose ou une peau acnéique 1 à 3 fois par jour pendant 6 à 12 semaines selon la gravité du cas traité.
(c) On prépare une lotion antiséborrhéique en procédant au mélange des ingrédients suivants :

| | |
|---|---|
| Composé de l'exemple 4 | 0,030 g |
| Propylène glycol | 5,000 g |
| Butylhydroxytoluène | 0,100 g |
| Ethanol (à 95°) q.s.p | 100,000 g |

Cette lotion sera appliquée deux fois par jour sur un cuir chevelu séborrhéique et on constate une amélioration significative dans un délai compris entre 2 et 6 semaines.
(d) On prépare une composition cosmétique contre les effets néfastes du soleil en procédant au mélange des ingrédients suivants :

| | |
|---|---|
| Composé de l'exemple 24 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p | 100,000 g |

Cette composition sera appliquée quotidiennement, elle permet de lutter contre le vieillissement photo-induit.
(e) On prépare la crème Huile dans l'Eau non ionique suivante :

| | |
|---|---|
| Composé de l'exemple 37 | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p. | 100,000 g |

Cette crème sera appliquée sur une peau psoriatique 1 à 2 fois par jour pendant 30 Jours.
(f) On prépare un gel topique en procédant au mélange des ingrédients suivants :

| | |
|---|---|
| Composé de l'exemple 18 | 0,050 g |
| Ethanol | 43,000 g |
| α-tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau | 9,300 g |
| Propylène glycol qsp | 100,000 g |

Ce gel sera appliqué dans le traitement de l'acné 1 à 3 fois par jour pendant 6 à 12 semaines selon la gravité du cas traité.
(g) On prépare une lotion capillaire anti-chute et pour la repousse des cheveux en procédant au mélange des ingrédients suivants :

| | |
|---|---|
| Composé de l'exemple 40 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On appliquera cette lotion 2 fois par jour pendant 3 mois sur un cuir chevelu ayant subi une chute de cheveu importante.
(h) On prépare une crème anti-acnéique en procédant au mélange des ingrédients suivants :

| | |
|---|---|
| Composé de l'exemple 12 | 0,005 g |
| Acide rétinoïque | 0,010 g |
| Mélange de stéarates de glycérol et de polyéthylène glycol (75 moles) vendu sous le nom de "Gelot 64" par la société "GATTEFOSSE" | 15,000 g |
| Huile de noyau polyoxyéthylénée à 6 moles d'oxyde d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000g |

Cette crème sera appliquée sur une peau atteinte de dermatose ou une peau acnéique 1 à 3 fois par jour pendant 6 à 12 semaines.
(i) On prépare une crème huile dans l'eau en réalisant la formulation suivante :

| | |
|---|---|
| Composé de l'exemple 28 | 0,020 g |
| 17-valérate de bétamethasone | 0,050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p. | 100,000g |

Cette crème sera appliquée 2 fois par jour sur une peau atteinte de dermatose pendant 30 jours.
(j) On prépare la crème de type huile dans l'eau suivante :

| | |
|---|---|
| Acide lactique | 5,000 g |
| Composé de l'exemple 32 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | .6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Eau | q.s.p |
| | 100,000g |

Cette crème sera appliquée 1 fois par jour, elle aide à lutter contre le vieillissement qu'il soit photo-induit ou chronologique.

## Revendications

1. Composés antagonistes RXR, **caractérisés par le fait qu'**ils répondent à la formule générale (I) suivante : 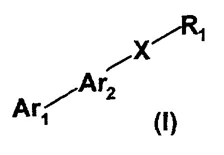 dans laquelle :
- R₁ représente
(i) le radical -CH₃
(ii) le radical -CH₂OR₂
(iii) le radical -CO-R₃
R₂ et R₃ ayant la signification donnée ci-après,
- Ar₁ représente un radical choisi parmi les radicaux de formules (a)-(c) suivante : 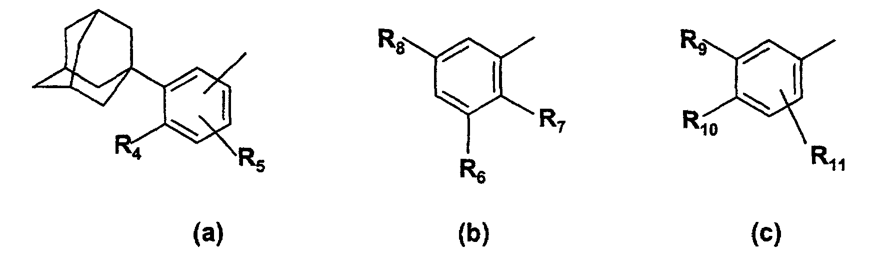 R₄ ,R₅ , R₆, R₇, R₈, R₉, R₁₀ et R₁₁ ayant les significations donnée ci-après,
- Ar₂ représente un radical choisi parmi les radicaux de formules (d)-(h) suivantes : 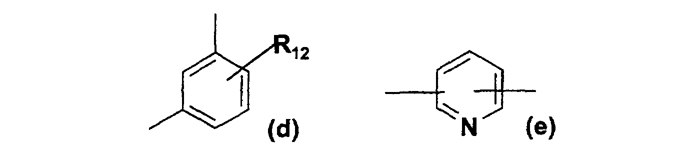 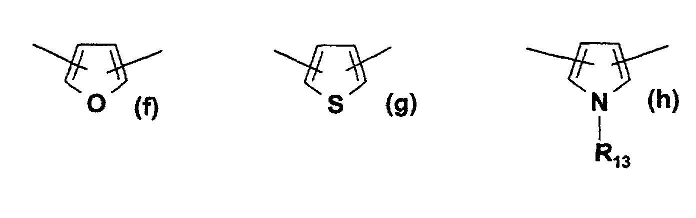 R₁₂ et R₁₃ ayant la signification donnée ci-après,
- X représente un radical choisi parmi les radicaux de formules (i)-(l), 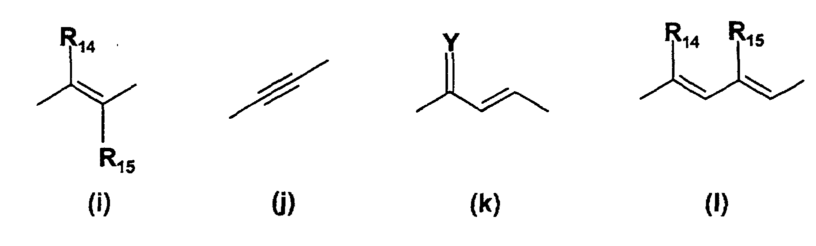 R₁₄, R₁₅ et Y ayant les significations données ci-après,
- R₂ représente un atome d'hydrogène, un radical alkyle ayant de 1 à 12 atomes de carbone ou un radical CO-R₁₆
R₁₆ ayant la signification donnée ci-après,
- R₃ représente:
(a) un atome d'hydrogène,
(b) un radical alkyle ayant de 1 à 12 atomes de carbone,
(c) un radical de formule : 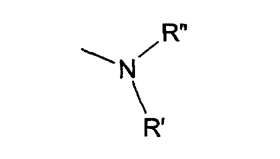
- R' et R" ayant la signification donnée ci-après,
(d) un radical -OR₁₇
R₁₇ ayant la signification donnée ci-après,
- R₄ représente un atome d'hydrogène, un radical polyéther, un radical alkyle ayant de 1 à 12 atomes de carbone ou un radical OR₁₈
R₁₈ ayant les significations ci-après ,
- R₅ représente un atome d'hydrogène, un radical alkyle ayant de 1 à 12 atomes de carbone, un radical polyéther, un radical OR₁₉
R₁₉ ayant les significations ci-après,
- R₆ représente un radical tertiobutyle,
- R₇ représente un radical alkyle ayant de 1 à 12 atomes de carbone, un radical polyéther ou un radical OR₂₀
R₂₀ ayant les significations ci-après,
- R₆ et R₇ pris ensemble peuvent former avec le cycle aromatique adjacent un cycle à 6 chaînons éventuellement substitué par des groupes méthyle et/ou éventuellement interrompu par un atome d'oxygène ou de soufre,
- R₈ représente un radical tertiobutyl, adamantyl, un radical phenyle événtuellement substitué par au moins un halogène, un alkyle ayant de 1 à 12 atomes de carbone, un hydroxyle, un alkoxy, une fonction nitro, un radical polyéther ou une fonction amino éventuellement protégé par un groupe acétyle ou éventuellement substitué par au moins un alkyle ayant de 1 à 12 atomes de carbone, ou un radical benzyle ou phénethyle événtuellement substitués par au moins un halogène, un alkyle ayant de 1 à 12 atomes de carbone, un hydroxyle, un alkoxy, une fonction nitro, un radical polyéther ou une fonction amino éventuellement protégé par un groupe acétyle ou éventuellement substitué par au moins un alkyle ayant de 1 à 12 atomes de carbone,,
- R₉ et R₁₀ pris ensemble forment avec le cycle aromatique adjacent un cycle à 5 ou 6 chaînons éventuellement substitué par des groupes méthyle et/ou éventuellement interrompu par un atome d'oxygène ou de soufre,
- R₁₁ représente un atome d'hydrogène, un radical alkyle ayant de1 à 9 atomes de carbone, un radical hydroxy, un radical alkoxy, un radical polyéther ou un radical -OR₂₁
R₂₁ ayant la signification donnée ci-après,
- R₁₂ représente, un atome d'hydrogène, un radical hydroxy, un radical alkoxy, un radical polyéther ou un radical -OR₂₂
R₂₂ ayant la signification donnée ci-après,
- R₁₃ représente un atome d'hydrogène, un radical afkyle ayant de 1 à 12 atomes de carbone ou un radical -COR₂₃
R₂₃ ayant la signification donnée ci-après,
- R₁₄ et R₁₅ identiques ou différents, représentent un atome d'hydrogène ou un radical alkyle ayant de 1 à 12 atomes de carbone,
- Y représente un atome d'oxygène ou un radical CH_{2,}
- R₁₆ représente un radical alkyle ayant de 1 à 12 atomes de carbone,
- R₁₇ représente un atome d'hydrogène, un radical alkyle linéaire ou ramifié, ayant de 1 à 20 atomes de carbone, un radical alkényle, un radical mono ou polyhydroxyalkyle, un radical phenyle événtuellement substitué par au moins un halogène, un alkyle ayant de 1 à 12 atomes de carbone, un hydroxyle, un alkoxy, une fonction nitro, un radical polyéther ou une fonction amino éventuellement protégé par un groupe acétyle ou éventuellement substitué par au moins un alkyle ayant de 1 à 12 atomes de carbone, ou un radical benzyle ou phénethyle événtuellement substitués par au moins un halogène, un alkyle ayant de 1 à 12 atomes de carbone, un hydroxyle, un alkoxy, une fonction nitro, un radical polyéther ou une fonction amino éventuellement protégé par un groupe acétyle ou éventuellement substitué par au moins un alkyle ayant de 1 à 12 atomes de carbone,ou un reste de sucre,
- R' et R" identiques ou différents, représentent un atome d'hydrogène, un radical alkyle ayant de 1 à 12 atomes de carbone, un radical mono ou polyhydroxyalkyle, un radical phenyle événtuellement substitué par au moins un halogène, un alkyle ayant de 1 à 12 atomes de carbone, un hydroxyle, un alkoxy, une fonction nitro, un radical polyéther ou une fonction amino éventuellement protégé par un groupe acétyle ou éventuellement substitué par au moins un alkyle ayant de 1 à 12 atomes de carbone,ou un reste d'amino acide ou encore pris ensemble forment un hétérocycle,
- R₁₈, R₁₉, R₂₀ identiques ou différents, représentent un atome d'hydrogène, un radical alkyle ayant de 1 à 12 atomes de carbone, un radical alkényle, un radical mono ou polyhydroxyalkyle, un radical phenyle événtuellement substitué par au moins un halogène, un alkyle ayant de 1 à 12 atomes de carbone, un hydroxyle, un alkoxy, une fonction nitro, un radical polyéther ou une fonction amino éventuellement protégé par un groupe acétyle ou éventuellement substitué par au moins un alkyle ayant de 1 à 12 atomes de carbone, ou un radical benzyle ou phénethyle événtuellement substitués par au moins un halogène, un alkyle ayant de 1 à 12 atomes de carbone, un hydroxyle, un alkoxy, une fonction nitro, un radical polyéther ou une fonction amino éventuellement protégé par un groupe acétyle ou éventuellement substitué par au moins un alkyle ayant de 1 à 12 atomes de carbone, ou un radical -(CH₂)ₙ-R₂₄,
n et R₂₄ ayant les significations ci-après,
- R₂₁ et R₂₂ identiques ou différents, représentent un radical alkényle, un radical mono ou polyhydroxyalkyle, un radical phenyle événtuellement substitué par au moins un halogène, un alkyle ayant de 1 à 12 atomes de carbone, un hydroxyle, un alkoxy, une fonction nitro, un radical polyéther ou une fonction amino éventuellement protégé par un groupe acétyle ou éventuellement substitué par au moins un alkyle ayant de 1 à 12 atomes de carbone, ou un radical benzyle ou phénethyle événtuellement substitués par au moins un halogène, un alkyle ayant de 1 à 12 atomes de carbone, un hydroxyle, un alkoxy, une fonction nitro, un radical polyéther ou une fonction amino éventuellement protégé par un groupe acétyle ou éventuellement substitué par au moins un alkyle ayant de 1 à 12 atomes de carbone, ou un radical -(CH₂)ₙ-R₂₄,
n et R₂₄ ayant les significations ci-après,
- R₂₃ représente un radical alkyle ayant de 1 à 12 atomes de carbone,
- R₂₄ représente un hétérocycle, un radical monohydroxyalkyle, un radical thiol éventuellement substitué par un alkyle ayant de 1 à 12 atomes de carbone, un radical amino éventuellement substitué par au moins un alkyl ayant de 1 à 12 atomes de carbone, un radical -COOR₂₅, ou un radical -CON(R₂₆)R₂₇,
R₂₅, R₂₆, et R₂₇ ayant les significations ci-après,
- R₂₅ représente un atome d'hydrogène ou un radical alkyle ayant de 1 à 12 atomes de carbone,
- R₂₆ et R₂₇ identiques ou différents représentent un atome d'hydrogène, un radical alkyle ayant de 1 à 12 atomes de carbone, un radical phenyle événtuellement substitué par au moins un halogène, un alkyle ayant de 1 à 12 atomes de carbone, un hydroxyle, un alkoxy, une fonction nitro, un radical polyéther ou une fonction amino éventuellement protégé par un groupe acétyle ou éventuellement substitué par au moins un alkyle ayant de 1 à 12 atomes de carbone, ou encore pris ensemble forment un hétérocycle,
- n est un nombre entier tel que 2 ≤ n ≤ 9,
avec les conditions que :
- lorsque Ar₁ représente le radical de formule (c) et X représente les radicaux de formule (i) ou (j) alors,
. R₁₁ représente le radical -OR₂₁ ou un radical polyéther, ou
. Ar₂ représente le radical de formule (d) avec R₁₂ représente le radical -OR₂₂ ou un radical polyéther,
- les composés de formule (I) sont différents de :
R₁₁ représente le radical méthoxyméthoxy en position ortho par rapport au substituant Ar₂ lorsque Ar₁ représente le radical de formule (c), X représente le radical de formule (i), R₁₂ représente l'hydrogène et R₁ représente le radical COR₃ avec R₃ représente le radical -OR₁₇ et R₁₇ représente l'hydrogène,
R₁₂ représente le radical méthoxyméthoxy en position ortho ou para par rapport au substituant Ar₁ lorsque Ar₁ représente le radical de formule (c), X représente le radical de formule (i), R₁₁ représente le radical méthyle en position ortho par rapport à Ar₂, R₁ représente le radical COR₃ avec R₃ représente le radical -OR₁₇ et R₁₇ représente l'hydrogène,
et les isomères optiques et géométriques desdits composés de formule (I) ainsi que leurs sels.

2. Composés selon la revendication 1, **caractérisés par le fait qu'**ils se présentent sous forme de sels d'un métal alcalin ou alcalino-terreux, de zinc, d'une amine organique ou d'un acide minéral ou organique.

3. Composés selon l'une des revendications 1 ou 2, **caractérisés par le fait que** les radicaux alkyles ayant de 1 à 12 atomes de carbones sont choisis parmi les radicaux méthyle, éthyle, isopropyle, butyle, tertiobutyle ou hexyle.

4. Composés selon l'une des revendications précédentes, **caractérisés en ce que** les radicaux alkyles linéaires ou ramifiés ayant de 1 à 20 atomes de carbone sont choisis parmi les radicaux méthyle, éthyle, tertiobutyle, propyle, octyle ou 2-éthylhexyle.

5. Composés selon l'une des revendications précédentes, **caractérisés en ce que** les radicaux alkoxy, sont choisis parmi les radicaux méthoxy, propyloxy, pentyloxy ou heptyloxy.

6. Composés selon l'une des revendications précédentes, **caractérisés en ce que** les radicaux monohydroxyalkyles correspondent à des radicaux présentant 2
ou 3 atomes de carbone, notamment un radical 2-hydroxyéthyle, 2-hydroxypropyle ou 3-hydroxypropyle.

7. Composés selon l'une des revendications précédentes, **caractérisés en ce que** les radicaux polyhydroxyalkyles sont choisis parmi les radicaux 2,3-dihydroxypropyle, 2,3,4-trihydroxybutyle, 2,3,4,5-tétrahydroxypentyle ou le reste du pentaérythritol.

8. Composés selon l'une des revendications précédentes, **caractérisés en ce que** les radicaux alkényles correspondent à des radicaux ayant de 2 à 5 atomes de carbone et présentent une ou plusieurs insaturations éthyléniques.

9. Composés selon l'une quelconque des revendications précédentes, **caractérisés en ce que** les radicaux polyéthers sont choisis parmi les radicaux méthoxyméthyl éther, méthoxyéthoxyméthyl éther ou méthylthiométhyl éther.

10. Composés selon l'une des revendications précédentes, **caractérisés en ce que** les restes de sucre sont choisis dans le groupe constitué par les restes de glucose, de galactose, de mannose ou d'acide glucuronique.

11. Composés selon l'une quelconque des revendications précédentes, **caractérisés en ce que** les restes d'aminoacide sont choisis dans le groupe constitué par les restes dérivant de la lysine, de la glycine ou de l'acide aspartique.

12. Composés selon l'une quelconque des revendications précédentes, **caractérisés en ce que** les radicaux hétérocycliques sont choisis dans le groupe constitué par les radicaux pipéridino, morpholino, pyrrolidino ou pipérazino, éventuellement substitués en position 4 par un radical alkyle en C₁-C₆ ou par un mono- ou polyhydroxyalkyle.

13. Composés selon la revendication 1, **caractérisés par le fait qu'**ils sont pris, seuls ou en mélanges, dans le groupe constitué par :
- acide 3-(2'-benzyloxy-3',5'-di-*tert*-butyl-6-hydroxybiphenyl-3-yl)acrylique,
- acide 3-(3',5'-di-*tert*-butyl-6-hydroxy-2'-pentyloxybiphenyl-3-yl)acrylique,
- acide 3-[3-(3-benzyloxy-5,5,8,8-tetramethyl-5,6,7,8-tetrahydronaphthalen-2-yl)-4-methoxymethoxyphenyl]acrylique,
- acide 3-[3-(3-benzyloxy-5,5,8,8-tetramethyl-5,6,7,8-tetrahydronaphthalen-2-yl)-4-hydroxyphenyl]acrylique,
- acide 3-[3-(3-propyloxy-5,5,8,8-tetramethyl-5,6,7,8-tetrahydronaphthalen-2-yl)-4-methoxymethoxyphenyl]acrylique,
- acide 3-[3-(3-pentyloxy-5,5,8,8-tetramethyl-5,6,7,8-tetrahydronaphthalen-2-yl)-4-methoxymethoxyphenyl]acrylique,
- acide 3-(5'-adamantan-1-yl-4'-methoxy-2'-methyl-biphenyl-3-yl)acrylique.
- acide 3-(5'-adamantan-1-yl-6-hydroxy-4'-methoxy-2'-methyl-biphenyl-3-yl)-acrylique,
- acide 3-(5'-adamantan-1-yl-4'-methoxy-6-methoxymethoxy-2'-methyl biphenyl-3-yl)acrylique,
- acide 3-{4-methoxy-3-[3-(3-methoxybenzyloxy)-5,5,8,8-tetramethyl-5,6,7,8-tetrahydronaphthalen-2-yl]phenyl}acrylique,
- acide 3-{4-methoxy-3-[3-(4-methoxybenzyloxy)-5,5,8,8-tetramethyl-5,6,7,8-tetrahydronaphthalen-2-yl]phenyl}acrylique,
- acide 3-{3-[3-(6-hydroxyhexyloxy)-5,5,8,8-tetramethyl-5,6,7,8-tetrahydronaphthalen-2-yl]-4-methoxyphenyl}acrylique,
- acide 3-{3-[3-(7-hydroxyheptyloxy)-5,5,8,8-tetramethyl-5,6,7,8-tetrahydro naphthalen-2-yl]-4-methoxyphenyl}acrylique,
- acide 3-{3-[3-(5-hydroxypentyloxy)-5,5,8,8-tetramethyl-5,6,7,8-tetrahydronaphthalen-2-yl]-4-methoxyphenyl}acrylique,
- acide 3-{3-[3-(3-hydroxypropyloxy)-5,5,8,8-tetramethyl-5,6,7,8-tetrahydronaphthalen-2-yl]-4-methoxyphenyl}acrylique,
- acide 3-[3-(1-benzyloxy-5,5,8,8-tetramethyl-5,6,7,8-tetrahydronaphthalen-2-yl)phenyl]acrylique,
- acide 3-(3'-adamantan-1-yl-4'-hydroxybiphenyl-3-yl)acrylique,
- acide 5-[4-methoxy-3-(5,5,8,8-tetramethyl-5,6,7,8-tetrahydronaphthalen-2-yl)-phenyl]-3-methylpenta-2,4-dienoique,
- acide 5-[4-methoxymethoxy-3-(5,5,8,8-tetramethyl-5,6,7,8-tetrahydronaphthalen-2-yl)-phenyl]-3-methylpenta-2,4-dienoique,
- acide5-[4-hydroxy-3-(5,5,8,8-tetramethyl-5,6,7,8-tetrahydro-naphthalen-2-yl)phenyl]-3-methylpenta-2,4-dienoique,
- acide 3-{3-[3-(5-tert-butoxycarbonylpentyloxy)-5,5,8,8-tetramethyl-5,6,7,8-tetrahydronaphthalen-2-yl]-4-methoxyphenyl}acrylique,
- acide 3-{3-[3-(7-tert-butoxycarbonylheptyloxy)-5,5,8,8-tetramethyl-5,6,7,8-tetrahydronaphthalen-2-yl]-4-methoxyphenyl}acrylique,
- acide 3-{3-[3-(7-carboxyheptyloxy)-5,5,8,8-tetramethyl-5,6,7,8-tetrahydronaphthalen-2-yl]-4-methoxyphenyl}acrylique,
- acide 3-{3-[3-(5-carboxypentyloxy)-5,5,8,8-tetramethyl-5,6,7,8-tetrahydronaphthalen-2-yl]-4-methoxyphenyl}acrylique,
- acide 3-{3-[3-(5-carbamoylpentyloxy)-5,5,8,8-tetramethyl-5,6,7,8-tetrahydro-naphthalen-2-yl]-4-methoxyphenyl}acrylique,
- acide 3-{3-[3-(7-carbamoylheptyloxy)-5,5,8,8-tetramethyl-5,6,7,8-tetrahydro-naphthalen-2-yl]-4-methoxyphenyl}acrylique,
- acide 3-{4-methoxy-3-[5,5,8,8-tetramethyl-3-(2-morpholin-4-yl-ethoxy)-5,6,7,8-tetrahydronaphthalen-2-yl]phenyl}acrylique,
- acide 3-{4-methoxy-3-[5,5,8,8-tetramethyl-3-(2-piperidin-1-yl-ethoxy)-5,6,7,8-tetrahydronaphthalen-2-yl]-phenyl}acrylique,
- acide 3-{4-methoxy-3-[3-(2-methoxymethoxybenzyloxy)-5,5,8,8-tetramethyl-5,6,7,8-tetrahydronaphthalen-2-yl]-phenyl}acrylique,
- acide 3-{4-methoxy-3-[3-(3-methoxymethoxybenzyloxy)-5,5,8,8-tetramethyl-5,6,7,8-tetrahydronaphthalen-2-yl]phenyl}acrylique,
- acide 3-{4-methoxy-3-[3-(4-methoxymethoxybenzyloxy)-5,5,8,8-tetramethyl-5,6,7,8-tetrahydronaphthalen-2-yl]phenyl}acrylique,
- acide 3-{4-methoxy-3-[3-(3-hydroxybenzyloxy)-5,5,8,8-tetramethyl-5,6,7,8-tetrahyd ronaphthalen-2-yl]phenyl}acrylique,
- acide 3-[4-fluoro-3-(3,5,5,8,8-pentamethyl-5,6,7,8-tetrahydronaphthalen-2-yl)phenyl]acrylique,
- acide 3-{4-hydroxy-3-[3-(3-methoxybenzyloxy)-5,5,8,8-tetramethyl-5,6,7,8-tetrahydronaphthalen-2-yl]phenyl}acrylique,
- acide 3-{4-hydroxy-3-[3-(4-methoxybenzyloxy)-5,5,8,8-tetramethyl-5,6,7,8-tetrahydronaphthalen-2-yl]-phenyl-acrylique,
- acide 3-{4-hydroxy-3-[3-(4-fluorobenzyloxy)-5,5,8,8-tetramethyl-5,6,7,8-tetrahydro-naphthalen-2-yl]phenyl}acrylique,
- acide 3-{4-hydroxy-3-[3-(4-chlorobenzyloxy)-5,5,8,8-tetramethyl-5,6,7,8-tetrahydro-naphthalen-2-yl]phenyl}acrylique,
- acide 3-[4-hydroxy-3-(3-methoxymethoxy-5,5,8,8-tetramethyl-5,6,7,8-tetrahydro-naphthalen-2-yl)-phenyl]acrylique,
- acide 3-(3',5'-di-*tert*-butyl-6-hydroxy-2'-propyloxybiphenyl-3-yl)acrylique,
- acide 3-(3',5'-di*-tert*-butyl-6-hydroxy-2'-butyloxybiphenyl-3-yl)acrylique,
- acide 3-(2'-butoxy-3',5'-di-tert-butyl-6-methoxy-biphenyl-3-yl)acrylique,
- acide 3-(3',5'-di-*tert*-butyl-6-methoxy-2'-propoxy-biphenyl-3-yl)acrylique,
- acide 3-[4-hydroxy-3-(5,5,8,8-tetramethyl-4-propoxy-5,6,7,8-tetrahydronaphthalen-2-yl)phenyl]acrylique,
- acide 3-[3-(3-benzyloxy-5,5,8,8-tetramethyl-5,6,7,8-tetrahydronaphthalen-2-yl)-4-methoxyphenyl]acrylique,
- 3-[3-(3-benzyloxy-5,5,8,8-tetramethyl-5,6,7,8-tetrahydronaphthalen-2-yl)-4-methoxyphenyl]acrylate d'ethyle,
- acide 3-methyl-5-[3-(3,5,5,8,8-pentamethyl-5,6,7,8-tetrahydro-naphthalen-2-yl)-phenyl]-penta-2,4-dienoique,
- 3-[3-(3-benzyloxy-5,5,8,8-tetramethyl-5,6,7,8-tetrahydro-naphthalen-2-yl)-4-methoxy-phenyl]prop-2-en-1-ol,
- 3-[3-(3-benzyloxy-5,5,8,8-tetramethyl-5,6,7,8-tetrahydro-naphthalen-2-yl)-4-methoxyphenyl]propenal,
- N-ethyl 3-[3-(3-benzyloxy-5,5,8,8-tetramethyl-5,6,7,8-tetrahydronaphthalen-2-yl)-4-methoxy-phenyl]acrylamide,
- 3-[3-(3-benzyloxy-5,5,8,8-tetramethyl-5,6,7,8-tetrahydro-naphthalen-2-yl)-4-methoxy-phenyl]-1-morpholin-4-yl-propenone,
- N-(4-hydroxyphenyl)-3-[3-(3-benzyloxy-5,5,8,8-tetramethyl-5,6,7,8-tetrahydro-naphthalen-2-yl)-4-methoxy-phenyl]acrylamide,
- acide 5-(5'-adamantan-1-yl-4'-methoxy-2'-methyl-biphenyl-3-yl)-3-methyl-penta-2,4-dienoïque,
- acide 5-[4-methoxymethoxy-3-(3,5,5,8,8-pentamethyl-5,6,7,8-tetrahydronaphthalen-2-yl)-phenyl]-3-methyl-penta-2,4-dienoïque,
- acide 5-[4-hydroxy-3-(3,5,5,8,8-pentamethyl-5,6,7,8-tetrahydronaphthalen-2-yl)-phenyl]-3-methyl-penta-2,4-dienoïque,
- acide 4-[3-(3,5,5,8,8-pentamethyl-5,6,7,8-tetrahydro-naphthalen-2-yl)-phenyl]-penta-2,4-dienoïque,
- acide 5-(3',5'-di-*tert*-butyl-2'-methoxy-biphenyl-3-yl)-3-methyl-penta-2,4-dienoïque,
- acide 5-(3',5'-di-*tert*-butyl-2'-propoxy-biphenyl-3-yl)-3-methyl-penta-2,4-dienoïque,
- acide 5-(2'-butoxy-3',5'-di-*tert*-butyl-biphenyl-3-yl)-3-methyl-penta-2,4-dienoïque,
- acide 5-(2'-butoxy-3',5'-di-*tert*-butyl-6-hydroxy-biphenyl-3-yl)-3-methyl-penta-2,4-dienoïque,
- acide 5-(3',5'-di-*tert*-butyl-6-hydroxy-2'-propoxy-biphenyl-3-yl)-3-methyl-penta-2,4-dienoïque,
- acide 5-(3',5'-di-*tert*-butyl-6-hydroxy-2'-methoxy-biphenyl-3-yl)-3-methyl-penta-2,4-dienoïque.

14. Composés selon la revendication 1, **caractérisés par le fait qu'**ils présentent l'une au moins l'une des, et de préférence toutes les, caractéristiques suivantes :
- R₁ représente le radical -CO-R₃
- Ar₂ représente les radicaux de formule (d) ou (e)
- R₁₁ représente le radical -O-R₂₁
- R₇ représente le radical -O-R₂₀

15. Composés selon l'une quelconque des revendications précédentes pour une utilisation comme médicament.

16. Utilisation d'un composé selon l'une quelconque des revendications 1 à 14 dans la préparation d'un médicament destiné au traitement :
- des affections dermatologiques liées à un désordre de la kératinisation portant sur la différenciation et sur la prolifération notamment les acnés vulgaires, comédoniennes, polymorphes, rosacées, les acnés nodulokystiques, conglobata, les acnés séniles, les acnés secondaires telles que l'acné solaire, médicamenteuse ou professionnelle;
- des ichtyoses, des états ichtyosiformes, de la maladie de Darier, des kératodermies palmoplantaires, des leucoplasies et des états leucoplasiformes, du lichen cutané ou muqueux (buccal);
- des affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale;
- des affections inflammatoires ne présentant pas de trouble de la kératinisation;
- des proliférations dermiques ou épidermiques qu'elles soient bénignes ou malignes, qu'elles soient ou non d'origine virale telles que les verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires;
- des dermatoses bulleuses et les maladies du collagène;
- des cornéopathies;
- du vieillissement de la peau, qu'il soit photoinduit ou chronologique ou pour réduire les pigmentations et les kératoses actiniques, ou toutes pathologies associées au vieillissement chronologique ou actinique;
- des stigmates de l'atrophie épidermique et/ou dermique induite par les corticostéroïdes locaux ou systémiques, ou tout autre forme d'atrophie cutanée;
- des troubles de la cicatrisation ou des vergetures; pour favoriser la cicatrisation;
- des troubles de la fonction sébacée tels que l'hyperséborrhée de l'acné ou la séborrhée simple;
- des états cancéreux ou précancéreux, plus particuliérement les leucémies promyélocytaires;
- d'affections inflammatoires telles que l'arthrite;
- d' affection d'origine virale au niveau cutané ou général;
- de l'alopécie;
- d'affections dermatologiques à composante immunitaire; d'affections du système cardiovasculaire telles que l'artériosclérose, l'hypertension, le diabète non-insulino dépendant ainsi que l'obésité;
- de désordres cutanés dus à une exposition aux rayonnements U.V..

17. Composition pharmaceutique, **caractérisée par le fait qu'**elle comprend, dans un support pharmaceutiquement acceptable, au moins l'un des composés tels que définis à l'une quelconque des revendications 1 à 14.

18. Composition selon la revendication 17, **caractérisée en ce que** la concentration en composé(s) selon l'une des revendications 1 à 14 est comprise entre 0,001 % et 5 % en poids par rapport à l'ensemble de la composition.

19. Composition cosmétique, **caractérisée par le fait qu'**elle comprend, dans un support cosmétiquement acceptable, au moins l'un des composés tels que définis à l'une quelconque des revendications 1 à 14.

20. Composition selon la revendication 19, **caractérisée en ce que** la concentration en composé(s) selon l'une des revendications 1 à 14 est comprise entre 0,001 % et 3 % en poids par rapport à l'ensemble de la composition.

21. Utilisation d'une composition cosmétique telle que définie à l'une des revendications 19 ou 20 pour l'hygiène corporelle ou capillaire.

## Claims

1. RXR antagonist compounds, **characterized in that** they correspond to the following general formula (I) : 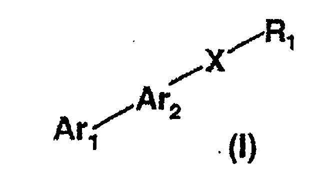 in which:
- R₁ represents
(i) the radical -CH₃
(ii) the radical -CH₂OR₂
(iii) the radical -CO-R₃
R₂ and R₃ having the meaning given below,
- Ar₁ represents a radical chosen from the radicals of the following formulae (a)-(c): 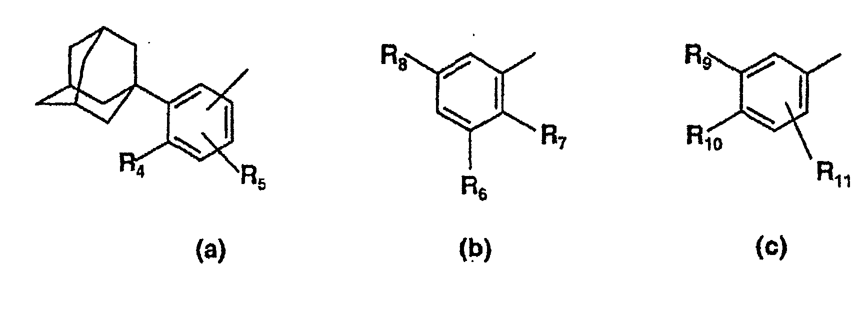 R₄, R₅, R₆, R₇, R₈, R₉, R₁₀ and R₁₁ having the meanings given below,
- Ar₂ represents a radical chosen from the radicals of the following formulae (d) - (h) : 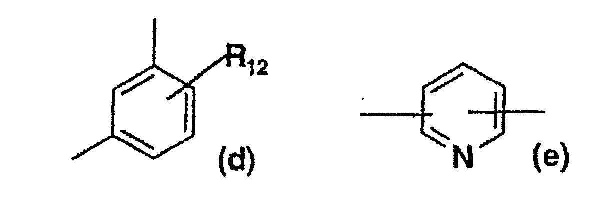 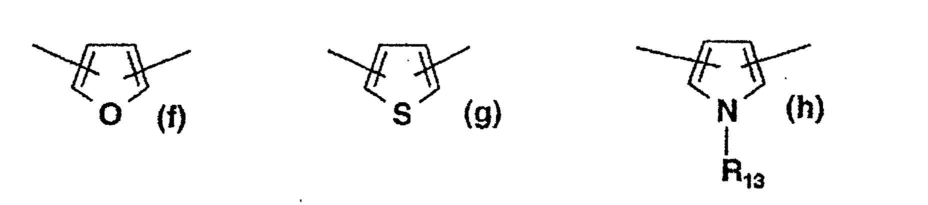 R₁₂ and R₁₃ having the meaning given below,
- X represents a radical chosen from the radicals of formulae (i)-(l), 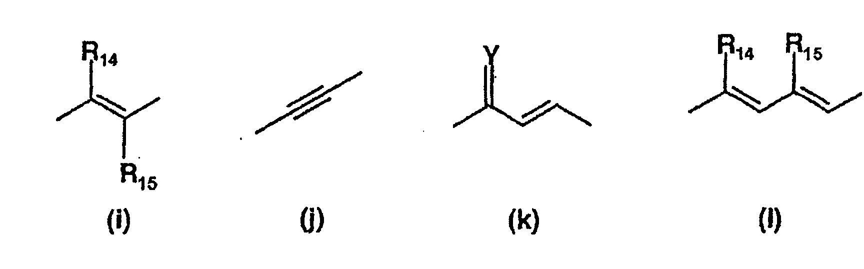 R₁₄, R₁₅ and Y having the meanings given below,
- R₂ represents a hydrogen atom, an alkyl radical having from 1 to 12 carbon atoms or a radical CO-R₁₆
R₁₆ having the meaning given below,
- R₃ represents:
(a) a hydrogen atom,
(b) an alkyl radical having from 1 to 12 carbon atoms,
(c) a radical of formula: 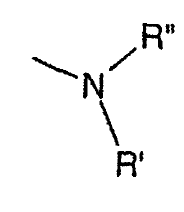
- R' and R" having the meaning given below,
(d) a radical -OR₁₇
R₁₇ having the meaning given below,
- R₄ represents a hydrogen atom, a polyether radical, an alkyl radical having from 1 to 12 carbon atoms or a radical OR₁₈
R₁₈ having the meanings below,
- R₅ represents a hydrogen atom, an alkyl radical having from 1 to 12 carbon atoms, a polyether radical or a radical OR₁₉
R₁₉ having the meanings below,
- R₆ represents a tert-butyl radical,
- R₇ represents an alkyl radical having from 1 to 12 carbon atoms, a polyether radical or a radical OR₂₀
R₂₀ having the meanings below,
- R₆ and R₇ taken together can form with the adjacent aromatic ring a 6-membered ring optionally substituted with methyl groups and/or optionally interrupted by an oxygen or sulphur atom,
- R₈ represents a tert-butyl radical, an adamantyl radical, a phenyl radical optionally substituted with at least one halogen, an alkyl having from 1 to 12 carbon atoms, a hydroxyl, an alkoxy, a nitro functional group, a polyether radical or an amino functional group optionally protected with an acetyl group or optionally substituted with at least one alkyl having from 1 to 12 carbon atoms, or a benzyl or phenethyl radical optionally substituted with at least one halogen, an alkyl having from 1 to 12 carbon atoms, a hydroxyl, an alkoxy, a nitro functional group, a polyether radical or an amino functional group optionally protected with an acetyl group or optionally substituted with at least one alkyl having from 1 to 12 carbon atoms,
- R₉ and R₁₀ taken together form with the adjacent aromatic ring a 5- or 6-membered ring optionally substituted with methyl groups and/or optionally interrupted by an oxygen or sulphur atom,
- R₁₁ represents a hydrogen atom, an alkyl radical having from 1 to 9 carbon atoms, a hydroxyl radical, an alkoxy radical, a polyether radical or a radical -OR₂₁
R₂₁ having the meaning given below,
- R₁₂ represents a hydrogen atom, a hydroxyl radical, an alkoxy radical, a polyether radical or a radical -OR₂₂
R₂₂ having the meaning given below,
- R₁₃ represents a hydrogen atom, an alkyl radical having from 1 to 12 carbon atoms or a radical -COR₂₃
R₂₃ having the meaning given below,
- R₁₄ and R₁₅, which are identical or different, represent a hydrogen atom or an alkyl radical having from 1 to 12 carbon atoms,
- Y represents an oxygen atom or a CH₂ radical,
- R₁₆ represents an alkyl radical having from 1 to 12 carbon atoms,
- R₁₇ represents a hydrogen atom, a linear or branched alkyl radical having from 1 to 20 carbon atoms, an alkenyl radical, a mono- or polyhydroxyalkyl radical, a phenyl radical optionally substituted with at least one halogen, an alkyl having from 1 to 12 carbon atoms, a hydroxyl, an alkoxy, a nitro functional group, a polyether radical or an amino functional group optionally protected with an acetyl group or optionally substituted with at least one alkyl having from 1 to 12 carbon atoms, or a benzyl or phenethyl radical optionally substituted with at least one halogen, an alkyl having from 1 to 12 carbon atoms, a hydroxyl, an alkoxy, a nitro functional group, a polyether radical or an amino functional group optionally protected with an acetyl group or optionally substituted with at least one alkyl having from 1 to 12 carbon atoms, or a sugar residue,
- R' and R", which are identical or different, represent a hydrogen atom, an alkyl radical having from 1 to 12 carbon atoms, a mono- or polyhydroxyalkyl radical, a phenyl radical optionally substituted with at least one halogen, an alkyl having from 1 to 12 carbon atoms, a hydroxyl, an alkoxy, a nitro functional group, a polyether radical or an amino functional group optionally protected with an acetyl group or optionally substituted with at least one alkyl having from 1 to 12 carbon atoms, or an amino acid residue, or alternatively taken together form a heterocycle,
- R₁₈, R₁₉ and R₂₀, which are identical or different, represent a hydrogen atom, an alkyl radical having from 1 to 12 carbon atoms, an alkenyl radical, a mono- or polyhydroxyalkyl radical, a phenyl radical optionally substituted with at least one halogen, an alkyl having from 1 to 12 carbon atoms, a hydroxyl, an alkoxy, a nitro functional group, a polyether radical or an amino functional group optionally protected with an acetyl group or optionally substituted with at least one alkyl having from 1 to 12 carbon atoms, or a benzyl or phenethyl radical optionally substituted with at least one halogen, an alkyl having from 1 to 12 carbon atoms, a hydroxyl, an alkoxy, a nitro functional group, a polyether radical or an amino functional group optionally protected with an acetyl group or optionally substituted with at least one alkyl having from 1 to 12 carbon atoms, or a radical -(CH₂)ₙ-R₂₄,
n and R₂₄ having the meanings below,
- R₂₁ and R₂₂, which are identical or different, represent an alkenyl radical, a mono- or polyhydroxyalkyl radical, a phenyl radical optionally substituted with at least one halogen, an alkyl having from 1 to 12 carbon atoms, a hydroxyl, an alkoxy, a nitro functional group, a polyether radical or an amino functional group optionally protected with an acetyl group or optionally substituted with at least one alkyl having from 1 to 12 carbon atoms, or a benzyl or phenethyl radical optionally substituted with at least one halogen, an alkyl having from 1 to 12 carbon atoms, a hydroxyl, an alkoxy, a nitro functional group, a polyether radical or an amino functional group optionally protected with an acetyl group or optionally substituted with at least one alkyl having from 1 to 12 carbon atoms, or a radical -(CH₂)ₙ-R₂₄,
n and R₂₄ having the meanings below,
- R₂₃ represents an alkyl radical having from 1 to 12 carbon atoms,
- R₂₄ represents a heterocycle, a monohydroxyalkyl radical, a thiol radical optionally substituted with an alkyl having from 1 to 12 carbon atoms, an amino radical optionally substituted with at least one alkyl having from 1 to 12 carbon atoms, a radical -COOR₂₅, or a radical -CON(R₂₆)R₂₇,
R₂₅, R₂₆ and R₂₇ having the meanings below,
- R₂₅ represents a hydrogen atom or an alkyl radical having from 1 to 12 carbon atoms,
- R₂₆ and R₂₇, which are identical or different, represent a hydrogen atom, an alkyl radical having from 1 to 12 carbon atoms, a phenyl radical optionally substituted with at least one halogen, an alkyl having from 1 to 12 carbon atoms, a hydroxyl, an alkoxy, a nitro functional group, a polyether radical or an amino functional group optionally protected with an acetyl group or optionally substituted with at least one alkyl having from 1 to 12 carbon atoms, or alternatively taken together form a heterocycle,
- n is an integer such that 2 ≤ n ≤ 9,
on the conditions that:
- when Ar₁ represents the radical of formula (c) and X represents the radicals of formula (i) or (j), then
. R₁₁ represents the radical -OR₂₁ or a polyether radical, or
. Ar₂ represents the radical of formula (d) with R₁₂ representing the radical -OR₂₂ or a polyether radical,
- the compounds of formula (I) are different from:
R₁₁ represents the methoxymethoxy radical at the ortho position with respect to the substituent Ar₂ when Ar₁ represents the radical of formula (c), X represents the radical of formula (i), R₁₁ represents hydrogen and R₁ represents the radical COR₃ with R₃ represents the radical -OR₁₇ and R₁₇ represents hydrogen,
R₁₂ represents the methoxymethoxy radical at the ortho or para position with respect to the substituent Ar₁ when Ar₁ represents the radical of formula (c), X represents the radical of formula (i), R₁₁ represents the methyl radical at the ortho position with respect to Ar₂, R₁ represents the radical COR₃ with R₃ represents the radical -OR₁₇ and R₁₇ represents hydrogen,
and the optical and geometric isomers of the said compounds of formula (I) as well as their salts.

2. Compounds according to Claim 1, **characterized in that** they exist in the form of salts of an alkali or alkaline-earth metal, of zinc, of an organic amine or of an inorganic or organic acid.

3. Compounds according to either of Claims 1 and 2, **characterized in that** the alkyl radicals having from 1 to 12 carbon atoms are chosen from methyl, ethyl, isopropyl, butyl, tert-butyl or hexyl radicals.

4. Compounds according to one of the preceding claims, **characterized in that** the linear or branched alkyl radicals having from 1 to 20 carbon atoms are chosen from the methyl, ethyl, tert-butyl, propyl, octyl or 2-ethylhexyl radicals.

5. Compounds according to one of the preceding claims, **characterized in that** the alkoxy radicals are chosen from the methoxy, propyloxy, pentyloxy or heptyloxy radicals.

6. Compounds according to one of the preceding claims, **characterized in that** the monohydroxyalkyl radicals correspond to radicals having 2 or 3 carbon atoms, especially a 2-hydroxyethyl, 2-hydroxypropy] or 3-hydroxypropyl radical.

7. Compounds according to one of the preceding claims, **characterized in that** the polyhydroxyalkyl radicals are chosen from the 2,3-dihydroxypropyl, 2,3,4-trihydroxybutyl or 2,3,4,5-tetrahydroxypentyl radicals or from the pentaerythritol residue.

8. Compounds according to one of the preceding claims, **characterized in that** the alkenyl radicals correspond to radicals having from 2 to 5 carbon atoms and have one or more ethylenic unsaturations.

9. Compounds according to any one of the preceding claims, **characterized in that** the polyether radicals are chosen from the methoxymethyl ether, methoxyethoxymethyl ether or methylthiomethyl ether radicals.

10. Compounds according to one of the preceding claims, **characterized in that** the sugar residues are chosen from the group consisting of glucose, galactose, mannose or glucuronic acid residues.

11. Compounds according to any one of the preceding claims, **characterized in that** the amino acid residues are chosen from the group consisting of the residues derived from lysine, glycine or aspartic acid.

12. Compounds according to any one of the preceding claims, **characterized in that** the heterocyclic radicals are chosen from the group consisting of piperidino, morpholino, pyrrolidino or piperazino radicals, optionally substituted at the 4-position with a C₁-C₆ alkyl radical or with a mono- or polyhydroxyalkyl.

13. Compounds according to Claim 1, **characterized in that** they are taken, alone or in the form of mixtures, from the group consisting of:
- 3-(2'-benzyloxy-3',5'-di-tert-butyl-6-hydroxybiphenyl-3-yl)acrylic acid,
- 3-(3',5'-di-*tert*-butyl-6-hydroxy-2'-pentyloxybiphenyl-3-yl)acrylic acid,
- 3-[3-(3-benzyloxy-5,5,8,8-tetramethyl-5,6,7,8-tetrahydronaphthalen-2-yl)-4-methoxymethoxyphenyl]-acrylic acid,
- 3-[3-(3-benzyloxy-5,5,8,8-tetramethyl-5,6,7,8-tetrahydronaphthalen-2-yl)-4-hydroxyphenyl]acrylic acid,
- 3-[3-(3-propyloxy-5,5,8,8-tetramethyl-5,6,7,8-tetrahydronaphthalen-2-yl)-4-methoxymethoxyphenyl]-acrylic acid,
- 3-[3-(3-pentyloxy-5,5,8,8-tetramethyl-5,6,7,8-tetrahydronaphthalen-2-yl)-4-methoxymethoxyphenyl]acrylic acid,
- 3-(5'-adamantan-1-yl-4'-methoxy-2'-methylbiphenyl-3-yl)acrylic acid,
- 3-(5'-adamantan-1-yl-6-hydroxy-4'-methoxy-2'-methylbiphenyl-3-yl)acrylic acid,
- 3-(5'-adamantan-1-yl-4'-methoxy-6'-methoxymethoxy-2'-methylbiphenyl-3-yl)acrylic acid,
- 3-{4-methoxy-3-[3-(3-methoxybenzyloxy)-5,5,8,8-tetramethyl-5,6,7,8-tetrahydronaphthalen-2-yl]phenyl}acrylic acid,
- 3-{4-methoxy-3-[3-(4-methoxybenzyloxy)-5,5,8,8-tetramethyl-5,6,7,8-tetrahydronaphthalen-2-yl]phenyl}acrylic acid,
- 3-{3-[3-(6-hydroxyhexyloxy)-5,5,8,8-tetramethyl-5,6,7,8-tetrahydronaphthalen-2-yl]-4-methoxyphenyl}acrylic acid,
- 3-{3-[3-(7-hydroxyheptyloxy)-5,5,8,8-tetramethyl-5,6,7,8-tetrahydronaphthalen-2-yl]-4-methoxyphenyl}acrylic acid,
- 3-{3-[3-(5-hydroxypentyloxy)-5,5,8,8-tetramethyl-5,6,7,8-tetrahydronaphthalen-2-yl]-4-methoxyphenyl}acrylic acid,
- 3-{3-[3-(3-hydroxypropyloxy)-5,5,8,8-tetramethyl-5,6,7,8-tetrahydronaphthalen-2-yl]-4-methoxyphenyl}acrylic acid,
- 3-[3-(1-benzyloxy-5,5,8,8-tetramethyl-5,6,7,8-tetrahydronaphthalen-2-yl)phenyl]acrylic acid,
- 3-(3'-adamantan-1-yl-4'-hydroxybiphenyl-3-yl)-acrylic acid,
- 5-[4-methoxy-3-(5,5,8,8-tetramethyl-5,6,7,8-tetrahydronaphthalen-2-yl)phenyl]-3-methylpenta-2,4-dienoic acid,
- 5-[4-methoxymethoxy-3-(5,5,8,8-tetramethyl-5,6,7,8-tetrahydronaphthalen-2-yl)phenyl]-3-methylpenta-2,4-dienoic acid,
- 5-[4-hydroxy-3-(5,5,8,8-tetramethyl-5,6,7,8-tetrahydronaphthalen-2-yl)phenyl]-3-methylpenta-2,4-dienoic acid,
- 3-{3-[3-(5-tert-butoxycarbonylpentyloxy)-5,5,8,8-tetramethyl-5,6,7,8-tetrahydronaphthalen-2-yl]-4-methoxyphenyl}acrylic acid,
- 3-{3-[3-(7-tert-butoxycarbonylheptyloxy)-5,5,8,8-tetramethyl-5,6,7,8-tetrahydronaphthalen-2-yl]-4-methoxyphenyl}acrylic acid,
- 3-{3-[3-(7-carboxyheptyloxy)-5,5,8,8-tetramethyl-5,6,7,8-tetrahydronaphthalen-2-yl]-4-methoxyphenyl}acrylic acid,
- 3-(3-[3-(5-carboxypentyloxy)-5,5,8,8-tetramethyl-5,6,7,8-tetrahydronaphthalen-2-yl]-4-methoxyphenyl}acrylic acid,
- 3-{3-[3-(5-carbamoylpentyloxy)-5,5,8,8-tetramethyl-5,6,7,8-tetrahydronaphthalen-2-yl]-4-methoxyphenyl}acrylic acid,
- 3-{3-[3-(7-carbamoylheptyloxy)-5,5,8,8-tetramethyl-5,6,7,8-tetrahydronaphthalen-2-yl]-4-methoxyphenyl}acrylic acid,
- 3-{4-methoxy-3-[5,5,8,8-tetramethyl-3-(2-morpholin-4-yl-ethoxy)-5,6,7,8-tetrahydronaphthalen-2-yl]phenyl}acrylic acid,
- 3-{4-methoxy-3-[5,5,8,8-tetramethyl-3-(2-piperidin-1-yl-ethoxy)-5,6,7,8-tetrahydronaphthalen-2-yl]phenyl}acrylic acid,
- 3-{4-methoxy-3-[3-(2-methoxymethoxybenzyloxy)-5,5,8,8-tetramethyl-5,6,7,8-tetrahydronaphthalen-2-yl]phenyl}acrylic acid,
- 3-{4-methoxy-3-[3-(3-methoxymethoxybenzyloxy)-5,5,8,8-tetramethyl-5,6,7,8-tetrahydronaphthalen-2-yl]phenyl}acrylic acid,
- 3-{4-methoxy-3-[3-(4-methoxymethoxybenzyloxy)-5,5,8,8-tetramethyl-5,6,7,8-tetrahydronaphthalen-2-yl]phenyl}acrylic acid,
- 3-{4-methoxy-3-[3-(3-hydroxybenzyloxy)-5,5,8,8-tetramethyl-5,6,7,8-tetrahydronaphthalen-2-yl]phenyl}acrylic acid,
- 3-[4-fluoro-3-(3,5,5,8,8-pentamethyl-5,6,7,8-tetrahydronaphthalen-2-yl)phenyl]acrylic acid,
- 3-{4-hydroxy-3-[3-(3-methoxybenzyloxy)-5,5,8,8-tetramethyl-5,6,7,8-tetrahydronaphthalen-2-yl]phenyl}acrylic acid,
- 3-{4-hydroxy-3-[3-(4-methoxybenzyloxy)-5,5,8,8-tetramethyl-5,6,7,8-tetrahydronaphthalen-2-yl]phenyl}acrylic acid,
- 3-{4-hydroxy-3-[3-(4-fluorobenzyloxy)-5,5,8,8-tetramethyl-5,6,7,8-tetrahydronaphthalen-2-yl]phenyl}acrylic acid,
- 3-{4-hydroxy-3-[3-(4-chlorobenzyloxy)-5,5,8,8-tetramethyl-5,6,7,8-tetrahydronaphthalen-2-yl]phenyl}acrylic acid,
- 3-[4-hydroxy-3-(3-methoxymethoxy-5,5,8,8-tetramethyl-5,6,7,8-tetrahydronaphthalen-2-yl)phenyl]-acrylic acid,
- 3- (3',5'-di-*tert*-butyl-6-hydroxy-2'-propyloxybiphenyl-3-yl)acrylic acid,
- 3-(3', 5'-di-*tert*-butyl-6-hydroxy-2'-butyloxybiphenyl-3-yl)acrylic acid,
- 3- (2'-butoxy-3',5'-di-tert-butyl-6-methoxybiphenyl-3-yl)acrylic acid,
- 3- (3', 5'-di-*tert*-butyl-6-methoxy-2'-propoxybiphenyl-3-yl)acrylic acid,
- 3-[4-hydroxy-3-(5,5,8,8-tetramethyl-4-propoxy-5,6,7,8-tetrahydronaphthalen-2-yl)phenyl]acrylic acid,
- 3-[3-(3-benzyloxy-5,5,8,8-tetramethyl-5,6,7,8-tetrahydronaphthalen-2-yl)-4-methoxyphenyl]acrylic acid,
- Ethyl 3-[3-(3-benzyloxy-5,5,8,8-tetramethyl-5,6,7,8-tetrahydronaphthalen-2-yl)-4-methoxyphenyl]-acrylate,
- 3-methyl-5-[3-(3,5,5,8,8-pentamethyl-5,6,7,8-tetrahydronaphthalen-2-yl)phenyl]penta-2,4-dienoic acid,
- 3-[3-(3-benzyloxy-5,5,8,8-tetramethyl-5,6,7,8-tetrahydronaphthalen-2-yl)-4-methoxyphenyl]prop-2-en-1-ol,
- 3-[3-(3-benzyloxy-5,5,8,8-tetramethyl-5,6,7,8-tetrahydronaphthalen-2-yl)-4-methoxyphenyl]propenal,
- N-ethyl-3-[3-(3-benzyloxy-5,5,8,8-tetramethyl-5,6,7,8-tetrahydronaphthalen-2-yl)-4-methoxyphenyl]-acrylamide,
- 3-[3-(3-benzyloxy-5,5,8,8-tetramethyl-5,6,7,8-tetrahydronaphthalen-2-yl)-4-methoxyphenyl]-1-morpholin-4-yl-propenone,
- N-(4-hydroxyphenyl)-3-[3-(3-benzyloxy-5,5,8,8-tetramethyl-5,6,7,8-tetrahydronaphthalen-2-yl)-4-methoxyphenyl]acrylamide,
- 5-(5'-adamantan-1-yl-4'-methoxy-2'-methylbiphenyl-3-yl)-3-methylpenta-2,4-dienoic acid,
- 5-[4-methoxymethoxy-3-(3,5,5,8,8-pentamethyl-5,6,7,8-tetrahydronaphthalen-2-yl)phenyl]-3-methylpenta-2,4-dienoic acid,
- 5-[4-hydroxy-3-(3,5,5,8,8-pentamethyl-5,6,7,8-tetrahydronaphthalen-2-yl)phenyl]-3-methylpenta-2,4-dienoic acid,
- 4-[3-(3,5,5,8,8-pentamethyl-5,6,7,8-tetrahydronaphthalen-2-yl)phenyl]penta-2,4-dienoic acid,
- 5-(3',5'-di-*tert*-butyl-2'-methoxybiphenyl-3-yl)-3-methylpenta-2,4-dienoic acid,
- 5-(3',5'-di-*tert*-butyl-2'-propoxybiphenyl-3-yl)-3-methylpenta-2,4-dienoic acid,
- 5- (2'-butoxy-3',5'-di-*tert*-butylbiphenyl-3-yl)-3-methylpenta-2,4-dienoic acid,
- 5-(2'-butoxy-3',5'-di-*tert*-butyl-6-hydroxybiphenyl-3-yl)-3-methylpenta-2,4-dienoic acid,
- 5- (3', 5'-di-*tert*-butyl-6-hydroxy-2'-propoxybiphenyl-3-yl)-3-methylpenta-2,4-dienoic acid,
- 5-(3',5'-di-*tert*-butyl-6-hydroxy-2'-methoxybiphenyl-3-yl)-3-methylpenta-2,4-dienoic acid.

14. Compounds according to Claim 1, **characterized in that** they have one, at least one, and preferably all, of the following characteristics:
- R₁ represents the radical -CO-R₃
- Ar₂ represents the radicals of formula (d) or (e)
- R₁₁ represents the radical -O-R₂₁
- R₇ represents the radical -O-R₂₀.

15. Compounds according to any one of the preceding claims, for use as a medicament.

16. Use of a compound according to any one of Claims 1 to 14, in the preparation of a medicament intended for the treatment of:
- dermatological conditions linked to a keratinization disorder relating to differentiation and proliferation, especially acne vulgaris, comedo-type acne, polymorphic acne, rosacea, nodulocystic acne, acne conglobata, senile acne, secondary acne such as solar acne, acne medicamentosa or occupational acne;
- ichthyosis, ichthyosiform states, Darier's disease, keratosis palmaris et plantaris, leukoplasia and leukoplasiform states, cutaneous or mucosal (buccal) lichen;
- dermatological conditions linked to a keratinization disorder with an inflammatory and/or immunoallergic component, and especially all the forms of psoriasis, whether cutaneous, mucosal or ungual, and even psoriatic rheumatism, or cutaneous atopy, such as eczema or respiratory atopy or gingival hypertrophy;
- inflammatory conditions which do not exhibit keratinization disorder;
- dermal or epidermal proliferations whether benign or malignant, whether of viral origin or not, such as verruca vulgaris, verruca plana and epidermodysplasia verruciformis, oral or florid papillomatoses and proliferations which may be induced by ultraviolet radiation especially in the case of baso- and spinocellular epithelioma;
- bullous dermatoses and collagen diseases;
- corneopathies;
- skin ageing, whether photoinduced or chronologic, or for reducing pigmentations and actinic keratoses, or any pathologies associated with chronologic or actinic ageing;
- stigmas of epidermal and/or dermal atrophy induced by local or systemic corticosteroids, or any other form of cutaneous atrophy;
- cicatrization disorders or stretch marks; for promoting cicatrization;
- disorders of the sebaceous function, such as hyperseborrhoea of acne or simple seborrhoea;
- cancerous or precancerous states, more particularly promyelocytic leukaemias;
- inflammatory conditions such as arthritis;
- conditions of viral origin at the cutaneous level or in general;
- alopecia;
- dermatological conditions with an immunological component; conditions of the cardiovascular system, such as arteriosclerosis, hypertension, non-insulin-dependent diabetes as well as obesity;
- skin disorders caused by exposure to UV radiation.

17. Pharmaceutical composition, **characterized in that** it comprises, in a pharmaceutically acceptable carrier, at least one of the compounds as defined in any one of Claims 1 to 14.

18. Composition according to Claim 17, **characterized in that** the concentration of compound(s) according to one of Claims 1 to 14 is between 0.001% and 5% by weight relative to the whole composition.

19. Cosmetic composition, **characterized in that** it comprises, in a cosmetically acceptable carrier, at least one of the compounds as defined in any one of Claims 1 to 14.

20. Composition according to Claim 19, **characterized in that** the concentration of compound(s) according to one of Claims 1 to 14 is between 0.001% and 3% by weight relative to the whole composition.

21. Use of a cosmetic composition as defined in either of Claims 19 and 20 for body or hair care.

## Patentansprüche

1. RXR-Antagonisten, **dadurch gekennzeichnet, daß** sie der folgenden allgemeinen Formel (I) entsprechen: 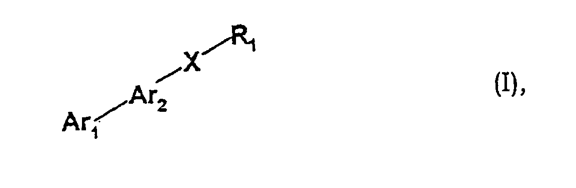 worin bedeuten:
- R₁
(i) die Gruppe -CH₃,
(ii) eine Gruppe -CH₂OR₂,
(iii) eine Gruppe -CO-R₃,
wobei R₂ und R₃ die nachfolgend angegebenen Bedeutungen aufweisen;
- Ar₁ eine Gruppe, die unter den folgenden Gruppen (a) bis (c) ausgewählt ist: 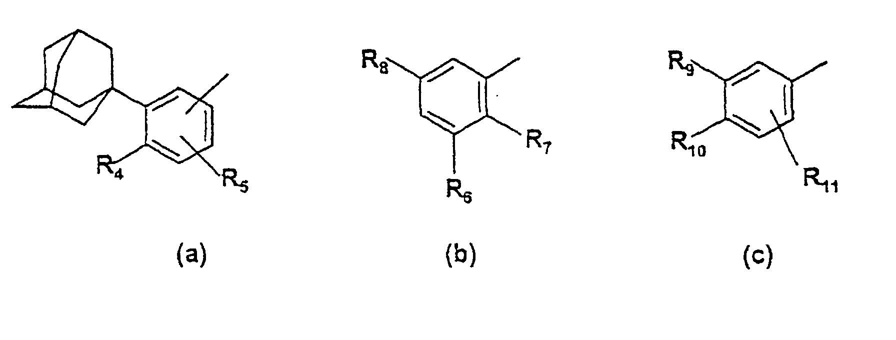
wobei die Gruppen R₄, R₅, R₆, R₇, R₈, R₉, R₁₀ und R₁₁ die nachstehend angegebenen Bedeutungen aufweisen;
- Ar₂ eine Gruppe, die unter den Gruppen der folgenden Formeln (d) bis (h) ausgewählt ist: 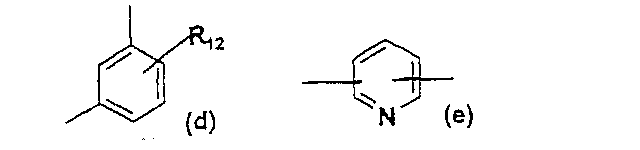 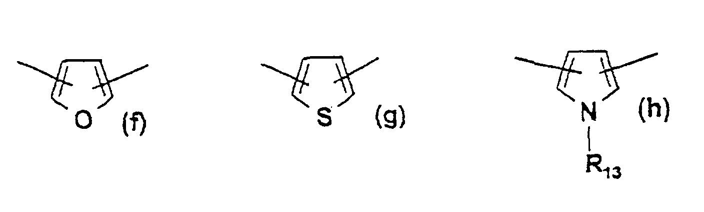 wobei die Gruppen R₁₂ und R₁₃ die nachstehend angegebenen Bedeutungen aufweisen;
- X eine Gruppe, die unter den Gruppen der folgenden Formeln (i) bis (1) ausgewählt ist: 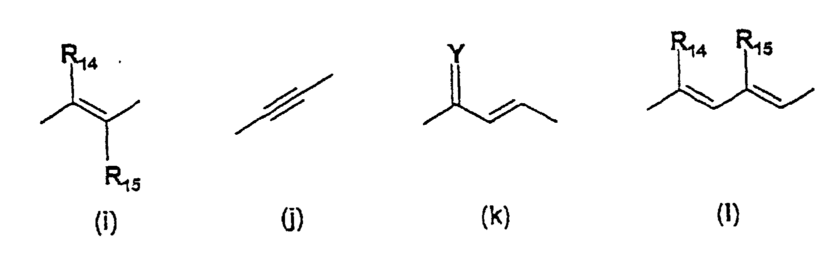 wobei die Gruppen R₁₄, R₁₅ und Y die nachfolgend angegebenen Bedeutungen aufweisen;
- R₂ ein Wasserstoffatom, eine Alkylgruppe mit 1 bis 12 Kohlenstoffatomen oder eine Gruppe -CO-R₁₆, wobei R₁₆ die nachfolgend angegebene Bedeutung hat;
- R₃:
(a) ein Wasserstoffatom,
(b) eine Alkylgruppe mit 1 bis 12 Kohlenstoffatomen,
(c) eine Gruppe der Formel: 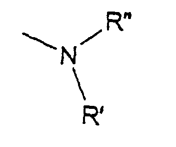 in der R' und R" die nachfolgend angegebenen Bedeutungen aufweisen,
(d) eine Gruppe -OR₁₇, wobei R₁₇ die nachfolgend angegebene Bedeutung hat;
- R₄ ein Wasserstoffatom, eine Polyethergruppe, eine Alkylgruppe mit 1 bis 12 Kohlenstoffatomen oder eine Gruppe -OR₁₈, wobei R₁₈ die nachfolgend angegebenen Bedeutungen aufweist;
- R₅ ein Wasserstoffatom, eine Alkylgruppe mit 1 bis 12 Kohlenstoffatomen, eine Polyethergruppe oder eine Gruppe -OR₁₉, wobei R₁₉ die nachfolgend angegebenen Bedeutungen aufweist;
- R₆ die *t*-Butylgruppe;
- R₇ eine Alkylgruppe mit 1 bis 12 Kohlenstoffatomen, eine Polyethergruppe oder eine Gruppe -OR₂₀, wobei R₂₀ die nachfolgend angegebenen Bedeutungen aufweist;
- R₆ und R₇ können mit dem angrenzenden aromatischen Ring auch einen 6-gliedrigen Ring bilden, der gegebenenfalls mit Methyl substituiert und/oder gegebenenfalls durch ein Sauerstoff- oder Schwefelatom unterbrochen ist;
- R₈ die t-Butylgruppe, Adamantyl, eine Phenylgruppe, die gegebenenfalls mit mindestens einer der folgenden Gruppen substituiert ist: Halogen, Alkyl mit 1 bis 12 Kohlenstoffatomen, Hydroxy, Alkoxy, Nitro, Polyether oder einer Aminogruppe, die gegebenenfalls in Form von Acetyl geschützt ist oder gegebenenfalls mit mindestens einer Alkylgruppe mit 1 bis 12 Kohlenstoffatomen substituiert ist, eine Benzyl- oder Phenethylgruppe, die gegebenenfalls mit mindestens einer der folgenden Gruppen substituiert ist: Halogen, Alkyl mit 1 bis 12 Kohlenstoffatomen, Hydroxy, Alkoxy, Nitro, Polyether oder einer Aminogruppe, die gegebenenfalls in Form von Acetyl geschützt ist oder gegebenenfalls mit mindestens einer Alkylgruppe mit 1 bis 12 Kohlenstoffatomen substituiert ist;
- R₉ und R₁₀ bilden mit dem angrenzenden aromatischen Ring einen 5- oder 6-gliedrigen Ring, der gegebenenfalls mit Methyl substituiert und/oder gegebenenfalls durch ein Sauerstoff- oder Schwefelatom unterbrochen ist;
- R₁₁ ein Wasserstoffatom, eine Alkylgruppe mit 1 bis 9 Kohlenstoffatomen, Hydroxy, Alkoxy, eine Polyethergruppe oder eine Gruppe -OR₂₁, wobei R₂₁ die nachfolgend angegebenen Bedeutungen aufweist;
- R₁₂ ein Wasserstoffatom, Hydroxy, Alkoxy, eine Polyethergruppe oder eine Gruppe -OR₂₂, wobei R₂₂ die nachfolgend angegebenen Bedeutungen aufweist;
- R₁₃ ein Wasserstoffatom, eine Alkylgruppe mit 1 bis 12 Kohlenstoffatomen oder eine Gruppe -COR₂₃, wobei R₂₃ die nachfolgend angegebenen Bedeutungen aufweist;
- R₁₄ und R₁₅, die identisch oder voneinander verschieden sind, ein Wasserstoffatom oder eine Alkylgruppe mit 1 bis 12 Kohlenstoffatomen;
- Y Sauerstoff oder die Gruppe CH₂;
- R₁₆ eine Alkylgruppe mit 1 bis 12 Kohlenstoffatomen;
- R₁₇ Wasserstoff, eine geradkettige oder verzweigte Alkylgruppe mit 1 bis 20 Kohlenstoffatomen, Alkenyl, Mono- oder Polyhydroxyalkyl, eine Phenylgruppe, die gegebenenfalls mit mindestens einer der folgenden Gruppen substituiert ist: Halogen, Alkyl mit 1 bis 12 Kohlenstoffatomen, Hydroxy, Alkoxy, Nitro, Polyether oder einer Aminogruppe, die gegebenenfalls in Form von Acetyl geschützt ist oder gegebenenfalls mit mindestens einer Alkylgruppe mit 1 bis 12 Kohlenstoffatomen substituiert ist, eine Benzyl- oder Phenethylgruppe, die gegebenenfalls mit mindestens einer der folgenden Gruppen substituiert ist: Halogen, Alkyl mit 1 bis 12 Kohlenstoffatomen, Hydroxy, Alkoxy, Nitro, Polyether oder einer Aminogruppe, die gegebenenfalls in Form von Acetyl geschützt ist oder gegebenenfalls mit mindestens einer Alkylgruppe mit 1 bis 12 Kohlenstoffatomen substituiert ist, oder einen Zukkerrest;
- R' und R", die identisch oder voneinander verschieden sind, ein Wasserstoffatom, eine Alkylgruppe mit 1 bis 12 Kohlenstoffatomen, eine Mono- oder Polyhydroxyalkylgruppe, eine Phenylgruppe, die gegebenenfalls mit mindestens einer der folgenden Gruppen substituiert ist: Halogen, Alkyl mit 1 bis 12 Kohlenstoffatomen, Hydroxy, Alkoxy, Nitro, Polyether oder einer Aminogruppe, die gegebenenfalls in Form von Acetyl geschützt ist oder gegebenenfalls mit mindestens einer Alkylgruppe mit 1 bis 12 Kohlenstoffatomen substituiert ist, einen Aminosäurerest oder die Gruppen R' und R" bilden gemeinsam einen Heterocyclus;
- R₁₈, R₁₉ und R₂₀, die identisch oder voneinander verschieden sind, ein Wasserstoffatom, eine Alkylgruppe mit 1 bis 12 Kohlenstoffatomen, eine Alkenylgruppe, eine Mono- oder Polyhydroxyalkylgruppe, eine Phenylgruppe, die gegebenenfalls mit mindestens einer der folgenden Gruppen substituiert ist: Halogen, Alkyl mit 1 bis 12 Kohlenstoffatomen, Hydroxy, Alkoxy, Nitro, Polyether oder einer Aminogruppe, die gegebenenfalls in Form von Acetyl geschützt ist oder gegebenenfalls mit mindestens einer Alkylgruppe mit 1 bis 12 Kohlenstoffatomen substituiert ist, eine Benzyl- oder Phenethylgruppe, die gegebenenfalls mit mindestens einer der folgenden Gruppen substituiert ist: Halogen, Alkyl mit 1 bis 12 Kohlenstoffatomen, Hydroxy, Alkoxy, Nitro, Polyether oder einer Aminogruppe, die gegebenenfalls in Form von Acetyl geschützt ist oder gegebenenfalls mit mindestens einer Alkylgruppe mit 1 bis 12 Kohlenstoffatomen substituiert ist, oder eine Gruppe -(CH₂)ₙ-R₂₄, wobei n und R₂₄ die nachfolgend angegebenen Bedeutungen aufweisen;
- R₂₁ und R₂₀, die identisch oder voneinander verschieden sind, eine Alkenylgruppe, eine Mono- oder Polyhydroxyalkylgruppe, eine Phenylgruppe, die gegebenenfalls mit mindestens einer der folgenden Gruppen substituiert ist: Halogen, Alkyl mit 1 bis 12 Kohlenstoffatomen, Hydroxy, Alkoxy, Nitro, Polyether oder einer Aminogruppe, die gegebenenfalls in Form von Acetyl geschützt ist oder gegebenenfalls mit mindestens einer Alkylgruppe mit 1 bis 12 Kohlenstoffatomen substituiert ist, eine Benzyl- oder Phenethylgruppe, die gegebenenfalls mit mindestens einer der folgenden Gruppen substituiert ist: Halogen, Alkyl mit 1 bis 12 Kohlenstoffatomen, Hydroxy, Alkoxy, Nitro, Polyether oder einer Aminogruppe, die gegebenenfalls in Form von Acetyl geschützt ist oder gegebenenfalls mit mindestens einer Alkylgruppe mit 1 bis 12 Kohlenstoffatomen substituiert ist, oder eine Gruppe -(CH₂)ₙ-R₂₄, wobei n und R₂₄ die nachfolgend angegebenen Bedeutungen aufweisen;
- R₂₃ eine Alkylgruppe mit 1 bis 12 Kohlenstoffatomen;
- R₂₄ einen Heterocyclus, eine Monohydroxyalkylgruppe, eine Thiolgruppe, die gegebenenfalls mit einer Alkylgruppe mit 1 bis 12 Kohlenstoffatomen substituiert ist, eine Aminogruppe, die gegebenenfalls mit mindestens einer Alkylgruppe mit 1 bis 12 Kohlenstoffatomen substituiert ist, eine Gruppe -COOR₂₅, oder eine Gruppe -CON(R₂₆)R₂₇, wobei R₂₅, R₂₆ und R₂₇ die nachfolgend angegebenen Bedeutungen aufweisen;
- R₂₅ Wasserstoff oder eine Alkylgruppe mit 1 bis 12 Kohlenstoffatomen;
- R₂₆ und R₂₇, die identisch oder voneinander verschieden sind, ein Wasserstoffatom, eine Alkylgruppe mit 1 bis 12 Kohlenstoffatomen, eine Phenylgruppe, die gegebenenfalls mit mindestens einer der folgenden Gruppen substituiert ist: Halogen, Alkyl mit 1 bis 12 Kohlenstoffatomen, Hydroxy, Alkoxy, Nitro, Polyether oder einer Aminogruppe, die gegebenenfalls in Form von Acetyl geschützt ist oder gegebenenfalls mit mindestens einer Alkylgruppe mit 1 bis 12 Kohlenstoffatomen substituiert ist, oder die Gruppen R₂₆ und R₂₇ bilden gemeinsam einen Heterocyclus;
- n eine ganze Zahl 2 ≤ n ≤ 9;
- mit der Maßgabe, daß
- wenn Ar₁ die Gruppe der Formel (c) ist und X die Gruppe der Formel (i) oder (j) bedeutet,
· R₁₁ eine Gruppe -OR₂₁ oder eine Polyethergruppe ist oder
· Ar₂ die Gruppe der Formel (d) ist, in der R₁₂ -OR₂₂ oder Polyether bedeutet;
- die Verbindungen der Formel (I) verschieden sind von Verbindungen mit:
R₁₁ bedeutet Methoxymethoxy in ortho-Stellung zu dem Substituenten Ar₂, wenn Ar₁ die Gruppe der Formel (c), X die Gruppe der Formel (i), R₁₂ Wasserstoff und R₁ die Gruppe COR₃ mit R₃ = -OR₁₇ ist, wobei R₁₇ Wasserstoff bedeutet,
R₁₂ bedeutet Methoxymethoxy in ortho- oder paraStellung zu dem Substituenten Ar₂, wenn Ar₁ die Gruppe der Formel (c), X die Gruppe der Formel (i), R₁₁ Methyl in ortho-Stellung zu Ar₂ und R₁ die Gruppe COR₃ mit R₃ = -OR₁₇ ist, wobei R₁₇ Wasserstoff bedeutet,
und die optischen und geometrischen Isomere der Verbindungen der Formel (I) sowie ihre Salze.

2. Verbindungen nach Anspruch 1, **dadurch gekennzeichnet, daß** sie in Form von Alkalimetallsalzen, Erdalkalimetallsalzen, Zinksalzen, Salzen von organischen Aminen oder Salzen von organischen oder anorganischen Säuren vorliegen.

3. Verbindungen nach einem der Ansprüche 1 oder 2, **dadurch gekennzeichnet, daß** die Alkylgruppen mit 1 bis 12 Kohlenstoffatomen unter Methyl, Ethyl, Isopropyl, Butyl, *t*-Butyl und Hexyl ausgewählt sind.

4. Verbindungen nach einem der vorhergehenden Ansprüche, **dadurch gekennzeichnet, daß** die geradkettigen oder verzweigten Alkylgruppen mit 1 bis 20 Kohlenstoffatomen unter Methyl, Ethyl, *t*-Butyl, Propyl, Octyl und 2-Ethylhexyl ausgewählt sind.

5. Verbindungen nach einem der vorhergehenden Ansprüche, **dadurch gekennzeichnet, daß** die Alkoxygruppen unter Methoxy, Propyloxy, Pentyloxy und Heptyloxy ausgewählt sind.

6. Verbindungen nach einem der vorhergehenden Ansprüche, **dadurch gekennzeichnet, daß** die Monohydroxyalkylgruppen Gruppen mit 2 oder 3 Kohlenstoffatomen sind, insbesondere 2-Hydroxyethyl, 2-Hydroxypropyl oder 3-Hydroxypropyl.

7. Verbindungen nach einem der vorhergehenden Ansprüche, **dadurch gekennzeichnet, daß** die Polyhydroxyalkylgruppen unter 2,3-Dihydroxypropyl, 2,3,4-Trihydroxybutyl, 2,3,4,5-Tetrahydroxypentyl oder Pentaerythrit ausgewählt sind.

8. Verbindungen nach einem der vorhergehenden Ansprüche, **dadurch gekennzeichnet, daß** die Alkenylgruppen Gruppen mit 2 bis 5 Kohlenstoffatomen sind, die eine oder mehrere ethylenische Doppelbindungen aufweisen.

9. Verbindungen nach einem der vorhergehenden Ansprüche, **dadurch gekennzeichnet, daß** die Polyethergruppen unter Methoxymethylether, Methoxyethoxymethylether oder Methylthiomethylether ausgewählt sind.

10. Verbindungen nach einem der vorhergehenden Ansprüche, **dadurch gekennzeichnet, daß** Zuckerreste unter den Resten von Glucose, Galactose, Mannose oder Glucuronsäure ausgewählt sind.

11. Verbindungen nach einem der vorhergehenden Ansprüche, **dadurch gekennzeichnet, daß** die Aminosäurereste unter den Resten von Lysin, Glycin oder Asparaginsäure ausgewählt sind.

12. Verbindungen nach einem der vorhergehenden Ansprüche, **dadurch gekennzeichnet, daß** die heterocyclischen Gruppen unter den Gruppen Piperidino, Morpholino, Pyrrolidino oer Piperazino ausgewählt sind, die gegebenenfalls in 4-Stellung mit einer C₁₋₆-Alkylgruppe oder einer Mono- oder Polyhydroxyalkylgruppe substituiert sind.

13. Verbindungen nach Anspruch 1, **dadurch gekennzeichnet, daß** sie unter den folgenden Verbindungen oder deren Gemischen ausgewählt sind:
- 3-(2'-Benzyloxy-3',5'-di-*t*-butyl-6-hydroxybiphenyl-3-yl)-acrylsäure,
- 3-(3',5'-di-*t*-Butyl-6-hydroxy-2'-pentyloxybiphenyl-3-yl)-acrylsäure,
- 3-[3-(3-Benzyloxy-5,5,8,8-tetramethyl-5,6,7,8-tetrahydronaphthalin-2-yl)-4-methoxymethoxyphenyl]-acrylsäure,
- 3-[3-(3-Benzyloxy-5,5,8,8-tetramethyl-5,6,7,8-tetrahydronaphthalin-2-yl)-4-hydroxyphenyl]-acrylsäure,
- 3-[3-(3-Propyloxy-5,5,8,8-tetramethyl-5,6,7,8-tetrahydronaphthalin-2-yl)-4-methoxymethoxyphenyl]-acrylsäure,
- 3-[3-(3-Pentyloxy-5,5,8,8-tetramethyl-5,6,7,8-tetrahydronaphthalin-2-yl)-4-methoxymethoxyphenyl]-acrylsäure,
- 3-(5'-Adamantan-1-yl-4'-methoxy-2'-methyl-biphenyl-3-yl)-acrylsäure,
- 3-(5'-Adamantan-1-yl-6-hydroxy-4'-methoxy-2'-methylbiphenyl-3-yl) - acrylsäure,
- 3-(5'-Adamantan-1-yl-4'-methoxy-6-methoxymethoxy-2'methyl-biphenyl-3-yl)-acrylsäure,
- 3-{4-Methoxy-3-[3-(3-methoxybenzyloxy)-5,5,8,8-tetramethyl-5,6,7,8-tetrahydro-naphthalin-2-yl]-phenyl}-acrylsäure,
- 3-{4-Methoxy-3-[3-(4-methoxybenzyloxy)-5,5,8,8-tetramethyl-5,6,7,8-tetrahydro-naphthalin-2-yl]-phenyl}-acrylsäure,
- 3-{3-[3-(6-Hydroxyhexyloxy)-5,5,8,8-tetramethyl-5,6,7,8-tetrahydro-naphthalin-2-yl]-4-methoxyphenyl}-acrylsäure,
- 3-{3-[3-(7-Hydroxyheptyloxy)-5,5,8,8-tetramethyl-5,6,7,8-tetrahydro-naphthalin-2-yl]-4-methoxyphenyl}-acrylsäure,
- 3-{3-[3-(5-Hydroxypentyloxy)-5,5,8,8-tetramethyl-5,6,7,8-tetrahydro-naphthalin-2-yl]-4-methoxyphenyl}-acrylsäure,
- 3-{3-[3-(3-Hydroxypropyloxy)-5,5,8,8-tetramethyl-5,6,7,8-tetrahydro-naphthalin-2-yl]-4-methoxyphenyl}-acrylsäure,
- 3-[3-(1-Benzyloxy-5,5,8,8-tetramethyl-5,6,7,8-tetrahydronaphthalin-2-yl)-phenyl]-acrylsäure,
- 3-(3'-Adamantan-1-yl-4'-hydroxybiphenyl-3-yl)-acrylsäure,
- 5-[4-Methoxy-3-(5,5,8,8-tetramethyl-5,6,7,8-tetrahydronaphthalin-2-yl)-phenyl]-3-methylpenta-2,4-diensäure,
- 5-[4-Methoxymethoxy-3-(5,5,8,8-tetramethyl-5,6,7,8-tetrahydro-naphthalin-2-yl)-phenyl]-3-methylpenta-2,4-diensäure,
- 5-[4-Hydroxy-3-(5,5,8,8-tetramethyl-5,6,7,8-tetrahydronaphthalin-2-yl)-phenyl]-3-methylpenta-2,4-diensäure,
- 3-{3-[3-(5-*t*-Butoxycarbonylpentyloxy)-5,5,8,8-tetramethyl-5,6,7,8-tetrahydronaphthalin-2-yl]-4-methoxyphenyl}acrylsäure,
- 3-{3-[3-(7-*t*-Butoxycarbonylheptyloxy)-5,5,8,8-tetramethyl-5,6,7,8-tetrahydronaphthalin-2-yl]-4-methoxyphenyl}acrylsäure,
- 3-{3-[3-(7-Carboxyheptyloxy)-5,5,8,8-tetramethyl-5,6,7,8-tetrahydronaphthalin-2-yl]-4-methoxyphenyl)-acrylsäure,
- 3-{3-[3-(5-Carboxypentyloxy)-5,5,8,8-tetramethyl-5,6,7,8-tetrahydronaphthalin-2-yl]-4-methoxyphenyl}-acrylsäure,
- 3-{3-[3-(5-Carbamoylpentyloxy)-5,5,8,8-tetramethyl-5,6,7,8-tetrahydronaphthalin-2 -yl] -4-methoxyphenyl}-acrylsäure,
- 3-{3-[3-(7-Carbamoylheptyloxy)-5,5,8,8-tetramethyl-5,6,7,8-tetrahydronaphthalin-2-yl]-4-methoxyphenyl}-acrylsäure,
- 3-{4-Methoxy-3-[5,5,8,8-tetramethyl-3-(2-morpholin-4-ylethoxy)-5,6,7,8-tetrahydronaphthalin-2-yl]phenyl}-acrylsäure,
- 3-{4-Methoxy-3-[5,5,8,8-tetramethyl-3-(2-piperidin-1-ylethoxy)-5,6,7,8-tetrahydronaphthalin-2-yl]phenyl}-acrylsäure,
- 3-{4-Methoxy-3-[3-(2-methoxymethoxybenzyloxy)-5,5,8,8-tetramethyl-5,6,7,8-tetrahydronaphthalin-2-yl]phenyl}acrylsäure,
- 3-{4-Methoxy-3-[3-(3-methoxymethoxybenzyloxy)-5,5,8,8-tetramethyl-5,6,7,8-tetrahydronaphthalin-2-yl]phenyl}acrylsäure,
- 3-{4-Methoxy-3-[3-(4-methoxymethoxybenzyloxy)-5,5,8,8-tetramethyl-5,6,7,8-tetrahydronaphthalin-2-yl]phenyl}acrylsäure,
- 3-{4-Methoxy-3-[3-(3-hydroxybenzyloxy)-5,5,8,8-tetramethyl-5,6,7,8-tetrahydronaphthalin-2-yl]phenyl}-acrylsäure,
- 3-[4-Fluor-3-(3,5,5,8,8-pentamethyl-5,6,7,8-tetrahydronaphthalin-2-yl)phenyl]-acrylsäure,
- 3-{4-Hydroxy-3-[3-(3-methoxybenzyloxy)-5,5,8,8-tetramethyl-5,6,7,8-tetrahydronaphthalin-2-yl]phenyl}-acrylsäure,
- 3-{4-Hydroxy-3-[3-(4-methoxybenzyloxy)-5,5,8,8-tetramethyl-5,6,7,8-tetrahydronaphthalin-2-yl]phenyl}-acrylsäure,
- 3-{4-Hydroxy-3-[3-(4-fluorbenzyloxy)-5,5,8,8-tetramethyl-5,6,7,8-tetrahydronaphthalin-2-yl]phenyl}-acrylsäure,
- 3-{4-Hydroxy-3-[3-(4-chlorbenzyloxy)-5,5,8,8-tetramethyl-5,6,7,8-tetrahydronaphthalin-2-yl]phenyl}-acrylsäure,
- 3-[4-Hydroxy-3-(3-methoxymethoxy-5,5,8,8-tetramethyl-5,6,7,8-tetrahydronaphthalin-2-yl]phenyl}-acrylsäure,
- 3-(3',5'-di-*t*-Butyl-6-hydroxy-2'-propyloxybiphenyl-3-yl)-acrylsäure,
- 3-(3',5'-di-*t*-Butyl-6-hydroxy-2'-butyloxybiphenyl-3-yl)-acrylsäure,
- 3-(2'-Butoxy-3',5'-di-*t*-butyl-6-methoxybiphenyl-3-yl)-acrylsäure,
- 3-(3',5'-di-*t*-Butyl-6-methoxy-2'-propoxybiphenyl-3-yl)-acrylsäure,
- 3-[4-Hydroxy-3-(5,5,8,8-tetramethyl-4-propoxy-5,6,7,8-tetrahydro-naphthalin-2-yl)-phenyl]-acrylsäure,
- 3-[3-(3-Benzyloxy-5,5,8,8-tetramethyl-5,6,7,8-tetrahydronaphthalin-2-yl)-4-methoxyphenyl]-acrylsäure,
- Ethyl-3-[3-(3-benzyloxy-5,5,8,8-tetramethyl-5,6,7,8-tetrahydronaphthalin-2-yl)-4-methoxyphenyl]-acrylat,
- 3-Methyl-5-[3-(3,5,5,8,8-pentamethyl-5,6,7,8-tetrahydronaphthalin-2-yl)-phenyl]-penta-2,4-diensäure,
- 3-[3-(3-Benzyloxy-5,5,8,8-tetramethyl-5,6,7,8-tetrahydronaphthalin-2-yl)-4-methoxyphenyl]-prop-2-en-1-ol,
- 3-[3-(3-Benzyloxy-5,5,8,8-tetramethyl-5,6,7,8-tetrahydronaphthalin-2-yl)-4-methoxyphenyl]-propenal,
- N-Ethyl-3-[3-(3-benzyloxy-5,5,8,8-tetramethyl-5,6,7,8-tetrahydronaphthalin-2-yl)-4-methoxyphenyl]-acrylamid,
- 3-[3-(3-Benzyloxy-5,5,8,8-tetramethyl-5,6,7,8-tetrahydronaphthalin-2-yl)-4-methoxyphenyl]-1-morpholin-4-ylpropenon,
- N-(4-Hydroxyphenyl)-3-[3-(3-benzyloxy-5,5,8,8-tetramethyl-5,6,7,8-tetrahydronaphthalin-2-yl)-4-methoxyphenyl]-acrylamid,
- 5-(5'-Adamantan-1-yl-4'-methoxy-2'-methyl-biphenyl-3-yl)-3-methyl-penta-2,4-diensäure,
- 5-[4-Methoxymethoxy-3-(3,5,5,8,8-pentamethyl-5,6,7,8-tetrahydro-naphthalin-2-yl)-phenyl]-3-methyl-penta-2,4-diensäure,
- 5-[4-Hydroxy-3-(3,5,5,8,8-pentamethyl-5,6,7,8-tetrahydronaphthalin-2-yl)-phenyl]-3-methyl-penta-2,4-diensäure,
- 4-[3-(3,5,5,8,8-Pentamethyl-5,6,7,8-tetrahydro-naphthalin-2-yl)-phenyl]-penta-2,4-diensäure,
- 5-(3',5'-di-*t*-Butyl-2'-methoxy-biphenyl-3-yl)-3-methyl-penta-2,4-diensäure,
- 5-(3',5'-di-*t*-Butyl-2'-propoxy-biphenyl-3-yl)-3-methyl-penta-2,4-diensäure,
- 5-(2'-Butoxy-3',5'-di-*t*-butyl-biphenyl-3-yl)-3-methyl-penta-2,4-diensäure,
- 5-(2'-Butoxy-3',5'-di-*t*-butyl-6-hydroxy-biphenyl-3-yl)-3-methyl-penta-2,4-diensäure,
- 5-(3',5'-di-*t*-Butyl-6-hydroxy-2'-propoxy-biphenyl-3-yl)-3-methyl-penta-2,4-diensäure, und
- 5-(3',5'-di-*t*-Butyl-6-hydroxy-2'-methoxy-biphenyl-3-yl)-3-methyl-penta-2,4-diensäure.

14. Verbindungen nach Anspruch 1, **dadurch gekennzeichnet, daß** sie mindestens eine und vorzugsweise alle folgenden Eigenschaften aufweisen:
- R₁ ist eine Gruppe -CO-R₃,
- Ar₂ bedeutet die Gruppe der Formel (d) oder (e),
- R₁₁ ist eine Gruppe -O-R₂₁,
- R₇ ist eine Gruppe -O-R₂₀.

15. Verbindungen nach einem der vorhergehenden Ansprüche zur Verwendung als Arzneimittel.

16. Verwendung einer Verbindung nach einem der Ansprüche 1 bis 14 zur Herstellung eines Arzneimittels, das zur Behandlung der folgenden Störungen und Erkrankungen vorgesehen ist:
- Zur Behandlung von dermatologischen Erkrankungen, die mit einer Verhornungsstörung verbunden sind, welche auf der Differenzierung und Proliferation beruht, insbesondere zur Behandlung von Acne vulgaris, Acne comedonica, polymorpher Acne, Acne rosaceae, nodulocystischer Acne, Acne conglobata, Acne senilis, sowie sekundären Akneformen, wie Acne solaris, Acne medikamentosa oder Acne professionalis;
- zur Behandlung von Ichtyosis, ichtyosisartigen Zuständen, der Darier Krankheit, Palmoplantarkeratosen, Leukoplakien und leukoplakieformen Zuständen und Lichen der Haut oder der Schleimhäute (buccalis);
- zur Behandlung von dermatologischen Erkrankungen, die mit einer Störung der Keratinisierung mit einer entzündlichen und/oder immunoallergischen Komponente verbunden sind, und insbesondere beliebigen Formen der Psoriasis der Haut, der Schleimhäute oder der Nägel oder auch zur Behandlung von Psoriasis arthropathica oder auch der Atopie der Haut wie Ekzemen oder der Atopie der Atemwege oder Hypertrophie des Zahnfleisches;
- zur Behandlung von entzündlichen Erkrankungen, die mit keiner Keratinisierungsstörung verbunden sind;
- zur Behandlung von Proliferationen der Dermis oder Epidermis, die gutartig oder bösartig und die gegebenenfalls viralen Ursprungs sein können, wie Verrucae vulgares, Verrucae planae und Epidermodysplasia verruciformis, Papillomatosis oralis oder florida, und Proliferationen, die durch UV-Strahlung hervorgerufen werden können, insbesondere im Falle von Epithelioma basocellulare und spinocellulare;
- zur Behandlung von bullösen Dermatosen und Erkrankungen des Kollagens;
- zur Behandlung von Corneopathien;
- zur Bekämpfung der Hautalterung, die lichtinduziert oder altersbedingt sein kann, oder zur Verminderung der Pigmentierung und der aktinischen Keratosen, oder beliebiger Erkrankungen, die mit der altersbedingten oder aktinischen Hautalterung zusammenhängen;
- zur Bekämpfung von Wundmalen der epidermalen und/oder dermalen Atrophie, die durch lokal oder systemisch verabreichte Corticosteroide hervorgerufen wird, oder beliebiger weiterer Formen der Atrophie der Haut;
- zur Behandlung von Störungen der Wundheilung, von Streifen oder zur Förderung der Wundheilung;
- zur Bekämpfung von Funktionsstörungen der Talgdrüsen, wie Hyperseborrhoe bei Acne oder Seborrhoe simplex;
- zur Behandlung von krebsartigen oder präcancerösen Zuständen, insbesondere promyelocytäre Leukämie;
- zur Behandlung von entzündlichen Erkrankungen, wie Arthritis;
- zur Behandlung von Hautkrankheiten viralen Ursprungs oder allgemeinen Erkrankungen viralen Ursprungs;
- zur Behandlung von Alopezie;
- zur Behandlung von dermatologischen oder allgemeinen Erkrankungen mit Immunkomponente; und Erkrankungen des cardiovasculären Systems, wie Arteriosklerose oder Bluthochdruck sowie nicht insulinpflichtiger Diabetes und Adipositas;
- zur Behandlung von Hautstörungen, die von einer Exposition gegenüber UV-Strahlung herrühren.

17. Pharmazeutische Zusammensetzung, **dadurch gekennzeichnet, daß** sie in einem pharmazeutisch akzeptablen Träger mindestens eine Verbindung nach einem der Ansprüche 1 bis 14 enthält.

18. Zusammensetzung nach Anspruch 17, **dadurch gekennzeichnet, daß** die Konzentration der Verbindung(en) nach einem der Ansprüche 1 bis 14 im Bereich von 0,001 bis 5 Gew.-%, bezogen auf die gesamte Zusammensetzung, liegt.

19. Kosmetische Zusammensetzung, **dadurch gekennzeichnet, daß** sie in einem kosmetisch akzeptablen Träger mindestens eine Verbindung nach einem der Ansprüche 1 bis 14 enthält.

20. Zusammensetzung nach Anspruch 19, **dadurch gekennzeichnet, daß** die Konzentration der Verbindung(en) nach einem der Ansprüche 1 bis 14 im Bereich von 0,001 bis 3 Gew.-%, bezogen auf die gesamte Zusammensetzung, liegt.

21. Verwendung einer kosmetischen Zusammensetzung nach einem der Ansprüche 19 oder 20 zur Körper- oder Haarpflege.
